# EUROPEAN PATENT APPLICATION

(11) **EP 4 541 257 A2**
(43) Date of publication of application: **23.04.2025**
(21) Application number: 24216184.2
(22) Date of filing: 31.08.2016
(51) Int. Cl.: A61B 1/00

(54) **ENDOSCOPE WITH PANNABLE CAMERA AND RELATED METHOD**

(30) Priority: 01.09.2015 US 201562212871 P; 10.03.2016 US 201662306288 P
(62) Divisional of application: 16763683.6
(71) Applicant: DEKA Products Limited Partnership, Manchester, NH 03101 (US)
(72) Inventor: HAGGERTY, Andrew, M., Lake Mary, 43746 (US); GRANT, Kevin, L., Litchfield, 03052 (US); VONDRAS, Peter, K, Somerville, 02144 (US); MOREAU, Timothy, D., Manchester, 03104 (US); DEMERS, Jason, A., Manchester, 03104 (US); DAVIS, Daniel, B., Nashua, 03062 (US)
(74) Representative: Elkington and Fife LLP

(57) **Abstract**

An endoscope and related method comprise a proximal handle and a distal shaft having an insertion end. A housing comprising a camera assembly may be mounted on an insertion end of the shaft and include at least one lens and an image sensor. The camera assembly housing is rotatable about an axis perpendicular to the long axis of the shaft, giving the camera assembly a variable field of view. The rotatable camera assembly housing may be mounted to the insertion end of the shaft so that the rotatable housing of the camera assembly comprises the distal-most element of the endoscope shaft or insertion end. The endoscope may include a circuit board having a first portion disposed within the proximal handle and one or more extension portions that extend within the shaft to the camera assembly and/or to a light source near the distal end of the shaft. At least one light emitter may be mounted on the insertion end of the shaft and configured to project light in a direction either toward or away from the field of view of the camera assembly. The light emitter may also be mounted on the camera assembly housing to direct light toward the field of view of the camera assembly. Power and communication lines can be co-located within a lumen of the shaft of the endoscope used for fluid irrigation or suction.

## Description

### Cross-References to Related Applications

This is a Non-Provisional Application claiming the benefit of U.S. Provisional Patent Application Serial No. 62/306,288, filed March 10, 2016 entitled **Endoscope with Pannable Camera and Related Method** (Attorney Docket No. R44), and U.S. Provisional Patent Application Serial No. 62/212,871, filed September 1, 2015 entitled **Endoscope with Pannable Camera and Related Method** (Attorney Docket No. Q57), which are hereby incorporated herein by reference in their entireties.

### BACKGROUND

### Field of Disclosure

The present disclosure relates to endoscopic instruments for viewing and working in relatively inaccessible spaces; and in some aspects for operating in tight anatomical spaces within a body using an endoscope or arthroscope, or the like.

### Background Information

The use of endoscopic instruments in medicine, allowing for remote viewing and operating in difficult-to-access spaces has become well-established. These instruments have also been useful in automotive, aviation, plumbing, electronics, and many other industries. In the field of medicine or veterinary practice, endoscopy or arthroscopy is often used to view or treat an anatomical region when minimal or no incisions are desired, or to avoid disturbing nearby tissues. In orthopedics, for example, the condition of a joint such as a knee or shoulder may be accessed using one or more arthroscopic instruments introduced into the joint through one or more small skin incisions. These instruments may also be used to repair various intra-articular tissues. Standard techniques of open surgery to view and repair these anatomical areas can be comparatively more time consuming, associated with greater risk and trauma to a patient, and can be associated with longer recovery time. Furthermore, anesthesia associated with open surgery may be more complicated, risky and costly. For improved field of view, an endoscope may be equipped with an actively flexible distal segment, controllable by the user at the handle end of the instrument. This may not be an effective option when the tip of the instrument is positioned in a confined space that may not accommodate the range of motion required for flexing the distal segment of an endoscope. In medical applications, one such example would include intra-articular surgery. Generally, using an instrument with a rigid insertion shaft may be preferred if the use of an instrument with an actively flexible distal segment is impractical. A non-flexible shaft may provide improved optics or image reproduction, increased space within the instrument for additional functionality, and greater durability. However, rigid endoscopes or arthroscopes have a limited field of view and may need to be repositioned or rotated frequently to increase the field of view. Some endoscopes or arthroscopes must be physically removed from the patient to have parts swapped out in order to change the field of view. Cannula systems may facilitate this approach, but may also increase the complexity of the procedure and the size of an incision. These limitations may reduce operator efficiency, increase surgery time, and may increase the risk of iatrogenic injury. In medical and other applications, it would be advantageous for an endoscope to have an increased or variable field of view without the use of an actively flexible distal segment. It may also be advantageous to combine functions within a single conduit in order to decrease the overall diameter of the shaft of an endoscope. Additionally, current instruments are prone to degradation in function and optical quality over repeated use, cleaning and/or sterilization. An endoscope design whose manufacturing and assembly cost is low enough to economically justify its non-re-use would also be advantageous. The costs of repeated cleaning or sterilization and re-packaging would be eliminated, and it may also be easier to standardize the sterility, quality and reliability of a single-use device.

### SUMMARY

An endoscope may comprise a proximal handle and a shaft having a distal insertion end at which a camera assembly is mounted in a rotatable housing. The rotatable housing is configured to rotate about an axis generally perpendicular to a long axis of the insertion end, with the rotatable housing being the distal-most element of the endoscope at the insertion end. The camera assembly may include a lens adjacent to an image sensor, which can be a CMOS or CCD device. A pull wire may extend from the handle to the insertion end of the shaft, the pull wire wrapping around a portion of the rotatable housing, and configured to rotate the housing upon for and aft movement of the pull wire in the endoscope shaft. The housing may have a range of motion that provides the camera assembly a field of view that includes a region in line with the long axis of the insertion end and a region at least perpendicular to the long axis of the insertion end. In some cases this may comprise a range of about 0 degrees to about 120 degrees with respect to the long axis of the insertion end, or between about 35 degrees and about 115 degrees. The housing may be a spheroid shell constructed from two half-shells in which a cutout of one or both shells is configured to accommodate an image sensor or camera assembly (e.g. lens plus image sensor). The light source may be mounted on the rotatable housing so that it can illuminate the field of view to which the camera assembly is pointing.

In another aspect, an endoscope may comprise a proximal handle and a shaft having a distal insertion end, the camera assembly configured to rotate about an axis generally perpendicular to a long axis of the insertion end. A light source may be located on the insertion end of the shaft, and oriented to project light in a direction generally perpendicular to the long axis of the insertion end. The rotation range of the field of view of the camera assembly may include the area illuminated by the light source, or alternatively it may exclude the area illuminated by the light source. In this case, the illumination by the light source provided to the image sensor or camera is indirect, reflected or ambient light. The light source may comprise one or more LED's.

In another aspect, an endoscope may include a printed circuit board (PCB) that comprises a base portion residing within a handle of the endoscope, and one or more elongate extension portions of the PCB configured to extend from the base portion of the PCB in the handle through a shaft of the endoscope, and terminating at a distal insertion end of the shaft. The base portion of the PCB may be a composite of a flexible board mated to or sandwiched with a rigid board, at least one of the extension portions comprising a flexible board extension, or at least one of the extension portions comprising a rigid board extension, or at least two of the extension portions comprising a flexible board extension and a rigid board extension. A proximal leg of the flexible board extension may be angled at about 90 degrees to a proximal end of the rigid board extension, and a distal leg of the flexible board extension may curve back to be parallel to the rigid board extension. The proximal leg of the flexible board extension can then be folded so as to bring the distal leg of the flexible board extension into alignment adjacent to the rigid board extension. Both the rigid board extension and the flexible board extension may extend through a lumen of the shaft of the endoscope. The PCB and its extensions may be coated with a water resistant coating or membrane, so that the extensions can run through a fluid carrying lumen of the endoscope shaft. The flexible board extension can be connected to a rotatable image sensor (such as a CMOS or a CCD) in the distal insertion end of the endoscope shaft, while the rigid board extension can be connected to one or more stationary light sources at the insertion end of the shaft. The flexible board extension is configured to have sufficient slack to allow free rotation of the image sensor within a pre-determined rotational range.

In another aspect an endoscope may comprise a proximal handle housing configured to house an electronic processing board for processing signals from an image sensor located at a distal end of a shaft of the endoscope. A distal handle housing is configured to hold the electronic processing board in a position fixed with respect to the distal handle section. One or more magnets are attached to an internal wall of the proximal handle housing, said magnets being located next to a Hall effect sensor on the electronic processing board. Thus the proximal handle housing is rotatable with respect to the distal handle housing, and the Hall effect sensor is configured to provide a signal to an electronic processor representing the relative rotation of the proximal handle housing with respect to the distal handle housing. The electronic processor may be connected to a user interface displaying an image generated by the image sensor, and a rotational orientation of the image can thus altered by a change in the relative rotation of the proximal handle housing with respect to the distal handle housing.

In another aspect, an endoscope comprises a handle enclosing an electronic processing board for processing signals from an image sensor located at a distal end of a shaft of the endoscope. A button on the handle includes a member that encloses a magnet positioned above or adjacent to a portion of the electronic processing board on which a Hall effect sensor is located. Thus depression, release or movement of the button alters a magnetic field near the Hall effect sensor sufficiently to alter a signal produced by the Hall effect sensor. The button can be configured to cause an electronic controller connected to the electronic processing board to start a recording of an image generated by the image sensor, to stop a recording of an image generated by the image sensor, or to take a photograph of an image generated by the image sensor, based on a movement or release of the button by a user. The button can also be configured to cause an electronic controller connected to the electronic processing board to turn on, turn off, or adjust a light source located at a distal insertion end of a shaft of the endoscope, based on a movement or release of the button by a user. The movement or release of the button may comprise a short or longer duration depression of the button, a pre-determined series of two or more depressions and releases of the button, or a release of the button between two depressions having two or more variable durations.

### BRIEF DESCRIPTION OF THE DRAWINGS

These and other aspects will become more apparent from the following detailed description of the various embodiments of the present disclosure with reference to the drawings wherein:
FIG. 1 is a representational illustration of a two-component handle design for an endoscope;
FIG. 2 shows additional features of the illustration of FIG. 1;
FIG. 3A shows an exemplary side view of an endoscope;
FIG. 3B shows an exemplary perspective view of another endoscope;
FIG. 4 shows a disassembled view of an example of a handle proximal section of an endoscope;
FIG. 5 shows a disassembled view of an alternate example of a handle proximal section of an endoscope;
FIG. 6 shows a disassembled view of an alternate example of a handle proximal section of an endoscope;
FIG. 7A shows a top perspective view of an example of a handle distal section of an endoscope;
FIG. 7B shows a side view of an example endoscope with a portion of the handle removed;
FIG. 7C shows a detailed view of a portion of an example handle distal section of an endoscope;
FIG. 8 shows an exploded view of a handle distal section and an example of a rotation sensing assembly of an endoscope;
FIG. 9A shows a partially assembled view of an exemplary endoscope handle;
FIG. 9B shows a partial cutaway view of a handle of an endoscope including an example rotation sensing arrangement;
FIG. 10A is a representational illustration of a pass-through fluid barrier allowing utility components to pass from the handle to a conduit of an endoscope;
FIG. 10B shows a representational illustration of a pass-through barrier with a flexible component;
FIG. 11A shows an exploded view of an example of an inner sheath mount serving as a pass-through fluid barrier;
FIG. 11B shows an example embodiment of a bulkhead or pass-through fluid barrier;
FIG. 11C shows another example embodiment of a bulkhead or pass-through fluid barrier;
FIG. 11D shows an embodiment of a pass-through barrier through which a number of utility components extend;
FIG. 12 shows an exploded view of an example of a pivot control assembly;
FIG. 13 shows a perspective view of an example of a sealing member ;
FIG. 14 shows a partially assembled view of an exemplary endoscope with an inner sheath mount, a pivot control structure or assembly, and sealing member in their assembled locations;
FIG. 15 shows another partially assembled view of an example endoscope with a pass-through barrier, pivot control structure, and printed circuit board encased in a protective material or housing;
FIG. 16 shows a perspective view of an outer sheath mount;
FIG. 16A shows a perspective view of an outer sheath and mount of an endoscope;
FIG. 16B is a rear perspective view of the outer sheath and mount of FIG. 16A;
FIG. 17 shows a close up partial view of an endoscope in which an inner sheath mount, inner sheath, and outer sheath are in their assembled locations;
FIG. 17A shows an exemplary trocar or obturator for insertion into an outer sheath of an endoscope;
FIG. 18 shows an example of a camera assembly mount separated from an inner sheath;
FIG. 19 shows an alternate example of a camera assembly mount as part of an inner sheath;
FIG. 20 depicts a cross-sectional view of example camera assembly mount and inner sheath of FIG. 19 taken at line 20-20 of FIG. 19;
FIG. 21 shows an example of a camera assembly, part of an outer sheath, and part of a camera assembly mount;
FIG. 22 shows an alternate example of a camera assembly, part of an outer sheath, and part of a camera assembly mount;
FIG. 23 shows an alternate example of a camera assembly, part of an outer sheath, and part of a camera assembly mount;
FIG. 23.1 shows a perspective view of the distal end of an endoscope shaft in which the camera assembly is mounted at the tip of the shaft without a protective guard, shield or tip structure;
FIG. 23.2 shows a rotatable camera housing with pull wire, and a bank of LED's at an exposed end of an endoscope shaft (surrounding sheath removed);
FIGs. 23.3 and 23.4 show each half of a spheroid rotatable housing for a camera assembly;
FIGs. 23.5 and 23.6 show the pivoting and bearing elements allowing for rotation of a housing for a camera assembly;
FIG. 24 shows a perspective view of a camera assembly;
FIG. 25 shows a side view of a camera assembly and a camera assembly mount with a wall of the camera assembly mount removed for clarity;
FIG. 26 shows a side view of an alternate exemplary camera assembly and camera assembly mount with a wall of the camera assembly mount removed for clarity;
FIG. 27 shows a side view of an alternate exemplary camera assembly and camera assembly mount with a wall of the camera assembly mount removed for clarity;
FIGS. 28-32 depict some of the possible rotational positions of an alternate camera assembly;
FIG. 33 shows an example camera assembly;
FIG. 33.1 shows a relationship between a camera assembly and LED's and their respective power and communications PCB extensions;
FIG. 33.2 shows a form factor for a PCB for an endoscope with extension components for the endoscope shaft;
FIG. 33.3 shows the PCB of FIG. 33.2 with one flexible extension folded over another extension of the PCB;
FIG. 33.4 shows how the PCB extensions are positioned in an endoscope shaft (sheath removed);
FIG. 33.5 shows a partially assembled endoscope showing a pass-through fluid barrier or bulkhead and PCB;
FIG. 33.6 shows a relationship between an endoscope PCB and other internal components of the endoscope handle;
FIG. 33.7 shows a fluid carrying lumen and PCB extensions located within an inner sheath of an endoscope shaft;
FIG. 33.8 shows an internal fluid path within the handle of an endoscope;
FIG. 34 shows an example camera assembly with attached optical fiber bundle and electronic flex cable;
FIG. 35 shows a top view of an exemplary camera assembly and camera assembly mount;
FIG. 36 shows a perspective view of a camera assembly and flexible optical fiber bundle or ribbon;
FIG. 37 shows a perspective view of a camera assembly having a monolithic camera housing and light emitting feature;
FIG. 38 shows a side view of the camera assembly of FIG. 37;
FIG. 39 shows an example of a flexible optical fiber bundle or ribbon;
FIG. 40 shows a side view of the flexible optical fiber ribbon of FIG. 39;
FIG. 41 shows a perspective view of an example of a light projection element;
FIG. 42 shows a perspective view of another example of a light projection element;
FIG. 43 shows a perspective view of another example of a light projection element;
FIG. 44 shows a bottom perspective view of the light projection element shown in FIG. 43;
FIG. 45 shows a cross sectional view of the light projection element shown in FIGS. 43 & 44 taken at line 43-43 of FIG. 43;
FIG. 46 shows a cross sectional view of the light projection element shown in FIGS. 43 & 44 taken at line 44-44 of FIG. 43;
FIG. 47 shows a cross sectional view of the light projection element shown in FIGS. 43 & 44 taken at line 45-45 of FIG. 43;
FIG. 48 shows a top perspective view of a camera assembly on which the light projection element of FIG. 43 is mounted;
FIG. 63 shows a cross sectional view of an example camera assembly taken at line 61-61 of FIG. 24;
FIG. 64 shows a cross sectional view of an example camera assembly taken at line 62-62 of FIG. 34;
FIG. 65 shows a cross-section view of an example camera assembly taken at line 62-62 of FIG. 34;
FIG. 66 depicts a representational illustration of a camera assembly at the distal tip of an endoscope in addition to an example sensor and number of example illumination sources;
FIG. 67 depicts a top down view of an example printed circuit board which includes an extension portion for projection into an endoscope shaft;
FIG. 68 depicts a side view of an example printed circuit board including a projecting portion;
FIG. 69 depicts an example flowchart detailing a number of example steps which may be used to control, with a processor, at least one variable light source of an endoscope based on received sensor data;
FIG. 70 shows a side view of a projecting portion of a printed circuit board having an example camera assembly, example sensor, and number of example light sources mounted thereto;
FIG. 71 shows a top down view of a distal tip of an example endoscope including the example projecting portion of a printed circuit board shown in FIG. 70;
FIG. 72 shows a cross sectional view of the distal tip of an endoscope taken at line 72-72 of FIG. 71;
FIG. 72.1 shows a perspective view of the distal end of an endoscope shaft in which the light source is located on the shaft to project light in a direction generally away from the field of view of the camera assembly;
FIG. 72.2 is a cross-sectional view of the distal end of the endoscope shaft of FIG. 72.1;
FIG. 73 depicts an example endoscope and example calibration fixture;
FIG. 74 depict flowchart including a number of example steps which may be used to calibrate lighting parameters values for variable illumination sources of an endoscope;
FIG. 96 shows a partially assembled view of an endoscope with a handle printed circuit board, power/HDMI cable, illumination fibers, and irrigation line in their assembled locations;
FIG. 97 shows a block diagram of an example image processing system; and
FIG. 98 depicts an example diagram illustrating how an image may be righted using input from a rotation sensing assembly.

### DETAILED DESCRIPTION

The terms 'endoscope' and 'arthroscope' as used herein are meant to be used interchangeably and are to be given their broadest interpretation, each term denoting an instrument having an elongate section for insertion into a space that is otherwise difficult to access, for the purpose of visual inspection, diagnosis and/or treatment or repair. In the field of medicine or veterinary practice, such a space may include a body or organ cavity, joint space, tissue plane or other body structure. The instrument may also be used in a number of non-medical (e.g., industrial) applications, in which the diameter of the insertion portion of an endoscope needs to be minimized, or in which the space within which an endoscope must operate is too confined to permit the use of an actively flexible distal segment.

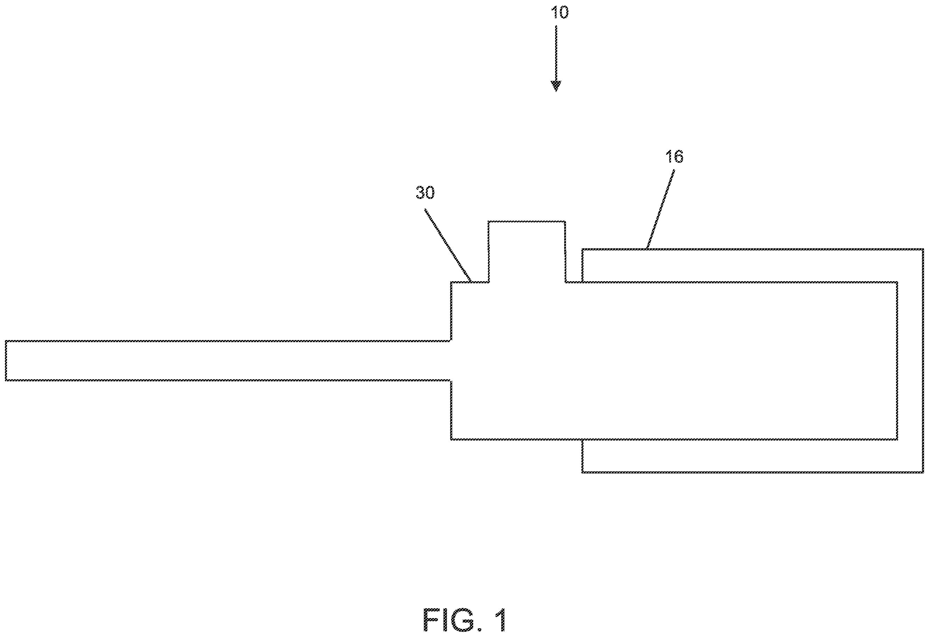

A two-component handle design of an endoscope **10** is shown in **FIG. 1****.** The example endoscope **10** includes a handle proximal section **16** and a handle distal section **30.** The handle proximal section **16** may be a housing. As shown, the handle distal section **30** may extend at least partially into the handle proximal section **16.** The handle distal section **30** and the handle proximal section **16** may be rotatable relative to each other. In some embodiments, a user may hold the handle proximal section **16** immobile while rotating the handle distal section **30** with a thumb or finger. The endoscope **10** may have a number of features such as, but not limited to, a rotation sensing assembly, fluid conduits, lighting, an imager or camera assembly, pivot control for the imager etc.

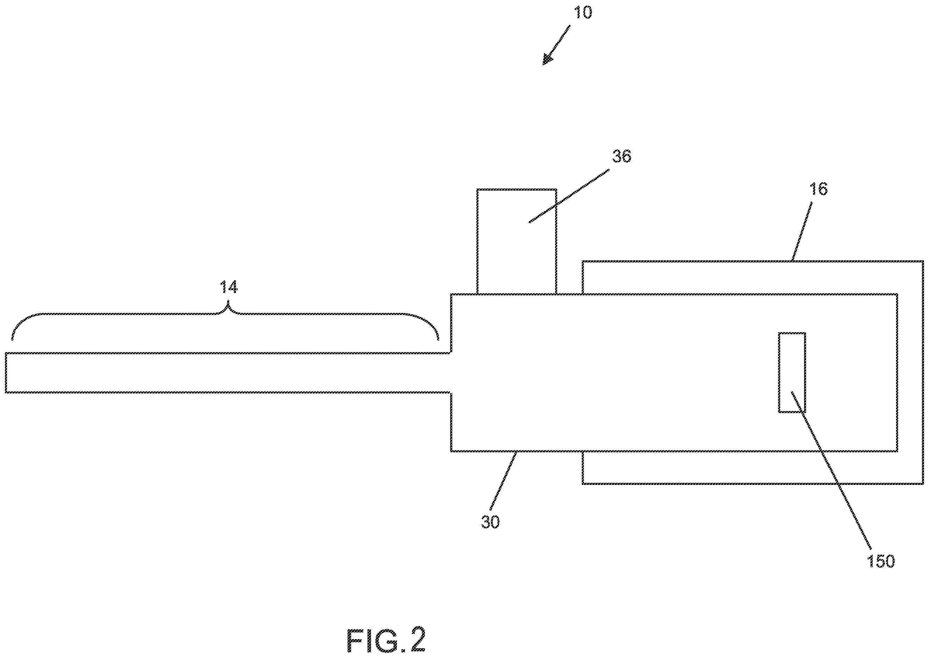

Additional features of the endoscope **10** are represented in **FIG. 2****.** The endoscope **10** includes a handle proximal section **16** and a handle distal section **30.** In this example, at least a part of an insertion shaft or section **14** is fixed to the handle distal section **30** and moves with the handle distal section **30.** The handle distal section **30** includes a handle protuberance or fin **36** which provides a surface for a user to press against to facilitate rotating the handle distal section **30** relative to the handle proximal section **16.** In some embodiments, a user's hand may hold the handle proximal section **16** immobile while the handle distal section **30** is rotated using one of the user's fingers or thumb.

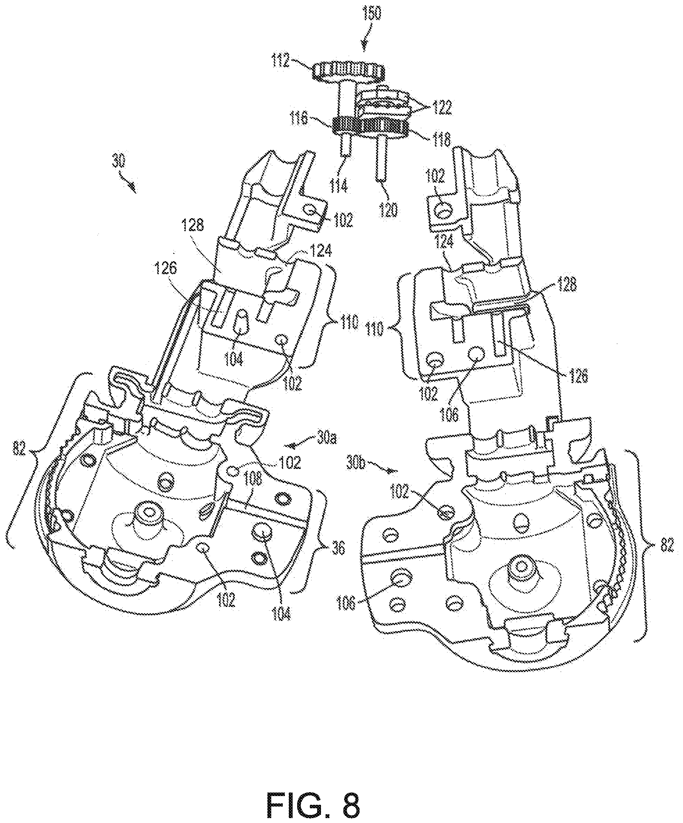

In some embodiments, one or both the handle proximal section **16** and the handle distal section **30** may function as a housing or provide a support structure for other components of the endoscope **10.** The endoscope **10** shown in **FIG. 2** may include a rotation sensing assembly **150.** The rotation sensing assembly **150** may track the rotation of handle distal section **30** relative to the handle proximal section **16.** In some embodiments, the rotation sensing assembly **150** may include a component which is stationary with respect to the handle proximal section **16** and a component that is stationary with respect to the handle distal section **30.** For example, the rotation sensing assembly **150** may include a potentiometer and a keyed shaft. The potentiometer may be mounted, for example to a support member comprising the internal housing of the handle proximal section **16.** Alternatively, the handle distal section **30** may also comprise a support member for mounting one or more components of the rotation sensing assembly **150** (see for example the rotation sensor holder in **FIG. 8**). In either case, a rotational or translational component of the rotation sensing assembly is arranged to move in proportion to the degree of rotation of the handle distal section **30** relative to the handle proximal section **16.**

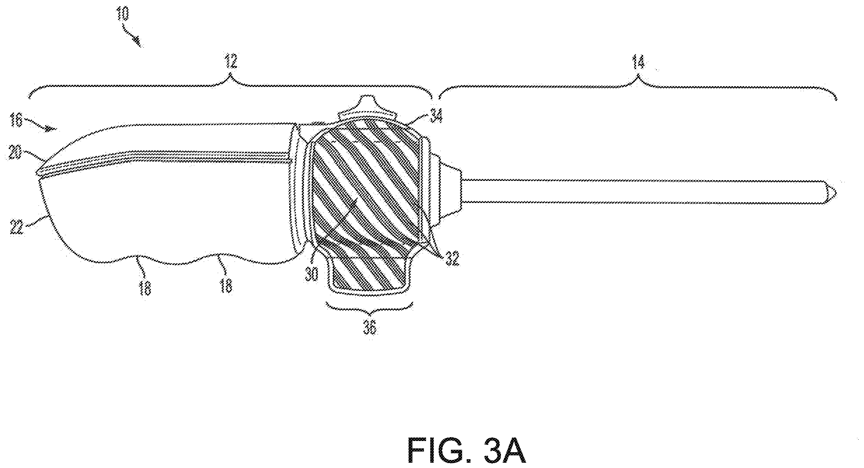

An exemplary embodiment of an endoscope (or, e.g., arthroscope) **10** is shown in **FIG. 3A****.** The endoscope **10** may be used in various endoscopic procedures, including arthroscopy, among others. As shown, the endoscope **10** includes a handle **12** and an insertion section or shaft **14,** which may comprise an elongate hollow shaft within which one or more actuation members, electrical/communications wires, lighting or light-transmitting cables and/or fluid channels may be located. As shown, in an embodiment the handle **12** may be roughly cylindrical and rounded in shape. The insertion section **14** may also be roughly cylindrical in shape and extend along a longitudinal axis. In an embodiment, the insertion section **14** may be rigid and relatively straight. In other embodiments, the insertion section **14** may be curved or angled along at least a portion of its length. In yet other embodiments, the insertion section **14** may comprise semi-rigid, malleable material permitting it to be bent and held to a desired shape. The diameter of the insertion section **14** is significantly smaller than that of the handle **12.** In some embodiments, the diameter of the insertion section **14** may be approximately 5.5 mm or smaller. The insertion section **14** of the endoscope **10** may be roughly the same length as that of the handle **12.** In alternative embodiments, the lengths and shapes of the handle **12** and insertion section **14** may differ substantially.

At least a portion of the insertion section **14** may be detachable from the handle **12.** In such embodiments, the insertion section **14** or detachable portion of the insertion section **14** may be coupled to the handle **12** by any of a variety of means including, but not limited to friction fit, snap fit, threaded coupling, bayonet mount, etc. In some embodiments, the insertion section **14** may be a disposable component and the handle **12** may be a reusable component. In embodiments in which the insertion section **14** is disposable, the insertion section **14** may be discarded after use. In other embodiments, the insertion section **14** may be sterilized after use via an autoclave, solution soaking, or other suitable sterilization procedure. In a preferred embodiment, both the handle **12** and the insertion section **14** are disposable and may be discarded after use, obviating the need for and cost of sterilization procedures and equipment (aside from a pre-usage sterilization with ethylene oxide, radiation, or the like, during, for example, manufacture, assembly or packaging of the device). Additionally, by making both the handle **12** and insertion section **14** of the endoscope **10** disposable, there is no degradation in function or reliability resulting from repeated use and repeated cleaning. Making the entire endoscope **10** disposable has other benefits, some which will be discussed below.

Preferably, a disposable endoscope **10** may be equipped with a means to prevent its reuse, particularly if sterilization of a used instrument is likely to degrade its function. For example, the endoscope **10** may include a memory chip storing an identification code that can be recognized by an electronic processor in a base unit to which the endoscope **10** must be connected for operability and display of images. The connection may include wired communications between a controller in the base unit and a memory chip in the endoscope **10,** or, for example wireless communications using an RFID device mounted in the endoscope **10.** (Other types of wireless transmission, such as, e.g. Bluetooth or Wi-Fi, may also be used). In an embodiment, the base unit may be programmed to encode a memory device on the endoscope **10** upon first use, and may be programmed to read and identify a code signifying that the endoscope **10** has been previously used whenever the endoscope **10** is subsequently re-connected to any base unit. Upon identification of a 'used' endoscope **10,** the controller may be programmed to prevent electronic and imaging communications between the endoscope **10** and the base unit. The code and its communication may be encrypted to enhance system security. Alternatively, the endoscope **10** may include a disablement feature in its software which renders the endoscope **10** inoperable after usage.

As shown in **FIG. 3A****,** the handle **12** of the endoscope **10** may include a number of different features. The handle **12** may include a handle proximal section **16.** The handle proximal section **16** may be relatively smooth as shown in **FIG. 3A****.** The handle proximal section **16** may comprise one or more hollow sections. The handle proximal section **16** may also be contoured such that it includes a number of ergonomic attributes. In some embodiments, at least a portion of the handle proximal section **16** may not have a smooth surface and may include a knurled, ribbed, roughened, honeycombed, etc. type texture, and/or a rubberized or elastomeric surface layer to facilitate gripping the endoscope **10** during its operation. In the example embodiment, the handle proximal section **16** is formed with a number of finger grooves **18.** In some embodiments, the handle proximal section **16** may be made of a material (e.g. rubber or other elastomer) that has a soft feel or is otherwise comfortable to hold. In some embodiments, a pistol grip-like feature (not shown) may be included as part of the handle proximal section **16.**

As shown in **FIG. 3A****,** the handle proximal section **16** may be divided into two separate parts. The handle proximal section **16** in **FIG. 3A** includes a handle top section **20** and a handle bottom section **22.** The handle top section **20** and handle bottom section **22** of the handle proximal section **16** may be manufactured as two separate parts and coupled together by any suitable means, such as, e.g., adhesive, screws, snap-fit, etc. As shown, the handle top section **20** is smooth and contoured differently from the handle bottom section **22.** This may help a user quickly and easily determine orientation of the endoscope **10** by feel. In some embodiments the handle top section **20** and handle bottom section **22** may comprise surface materials that have a different feel (e.g., metallic vs. plastic, metallic vs. elastomeric, smooth vs. textured, etc.).

The handle **12** of the endoscope **10** may also include a handle distal section **30.** As shown in **FIG. 3A****,** the handle distal section **30** extends from the handle proximal section **16** toward the insertion section **14.** The handle distal section **30** may be smaller in diameter than the handle proximal section **16.** As shown, the handle distal section **30** may be longer in length than the handle proximal section **16,** but in alternate embodiments, the relative dimensions of the handle distal section **30** and handle proximal section **16** may differ.

On at least a portion of the handle distal section **30** there may be a gripping texture as shown in **FIG. 3A****.** In the example embodiment shown in **FIG. 3A****,** the grip texture is a series of spiraling ribs **32.** In other embodiments, other gripping textures, such as non-spiraling ribs, nubs, bumps, grooves, honeycomb patterning or other form of knurling or checkering, etc. may also be used. As shown, the spiraling ribs **32** in the example embodiment encircle most of the outer diameter of the handle distal section **30.** In some embodiments including a gripping texture on the handle distal section **30,** the gripping texture may not be formed as a continuous part of the handle distal section **30.** In such embodiments, the gripping texture may be a 'skin' or sleeve applied onto the handle distal section **30.** The gripping texture skin may be coupled to the handle distal section **30** by any suitable means such as, but not limited to, adhesive, snap fit, various fasteners, over-mold, etc. In some embodiments, the gripping texture skin may be made of a material different from the handle distal section **30.** The gripping texture skin, for example, may be a softer, elastomeric or rubbery, material which is more comfortable to grip/less slippery than the handle distal section **30** material.

In the example embodiment of Fig. 3A, the handle distal section **30** includes a handle raised portion **34** projecting from the top of the handle distal section **30.** In this example, the handle raised portion **34** does not project sharply up from the rest of the handle distal section **30.** Instead, the handle raised portion **34** may be constructed to gently curve up from the rest of the handle distal section **30.** In this example, the spiraling ribs **32** do not extend over and onto the top of the handle raised portion **34.** Additional features of the handle raised portion **34** will be further described below.

In one aspect, projecting from the bottom of the handle distal section **30** may be a handle fin or paddle **36.** In this example, the handle fin **36** may be constructed to gently curve away from the rest of the handle distal section **30** toward an inferior or dependent position of the endoscope **10.** The spiraling ribs **32** may or may not extend over and onto the bottom of the handle fin **36.** In other embodiments, a handle fin **36** may be configured to project from the top of the handle distal section **30,** while the handle raised portion **34** may be configured to project from another aspect of the handle distal section **30.** The handle fin **36** may be disposed so as to correspond to the location of an entry point for various cables, irrigation, etc. in endoscopes which may already be familiar to a physician. This may be desirable since such an entry point is often used as a surface to press against to facilitate rotation and as an orientation marker. Additional features of the handle fin **36** will be further described below.

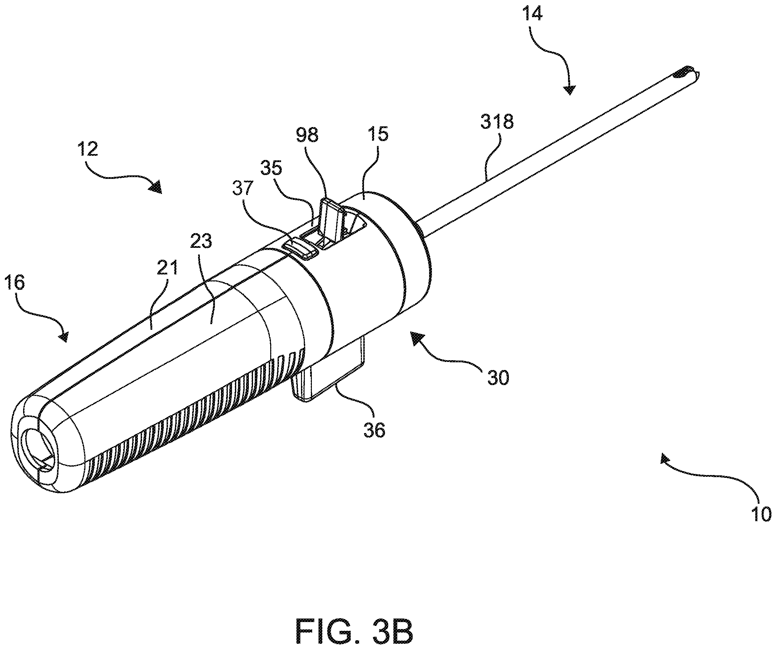

An alternative embodiment of an endoscope (or, e.g., arthroscope) **10** is shown in **FIG. 3B****.** As shown, the endoscope **10** includes a handle **12** and an insertion section or shaft **14,** which may comprise an elongate hollow shaft within which one or more actuation members, electrical/communications wires, lighting or light-transmitting cables and/or fluid channels may be located. At least a portion of the shaft **14** may be detachable from the handle **12.** In the example embodiment, the shaft 14 of the endoscope comprises an outer sheath or cannula **318** attached to a mounting structure **15** which may facilitate attachment of the cannula to and detachment of the cannula from the handle **12** by any of a variety of means including, but not limited to friction fit, snap fit, threaded coupling, bayonet mount, etc.

As shown in **FIG. 3B****,** the handle **12** of the endoscope **10** may include a handle proximal section **16** which encloses (among other components) a printed circuit board (PCB) for controlling or processing image data detected by a sensor at the distal end of the shaft, and/or for providing power to light sources (e.g. LED's) at the end of the shaft. It may also house a fluid conduit for connecting to a fluid carrying lumen within the shaft 14. A handle proximal section **16** may be divided into two separate parts. The handle proximal section **16** in **FIG. 3B** includes a handle first half-shell **21** and a handle second half-shell **23.** The handle first half-shell **21** and handle second half-shell **23** of the handle proximal section **16** may be manufactured as two separate parts and coupled together during assembly by any suitable means, such as, e.g., adhesive, screws, snap-fit, etc and may be symmetrical. For example, the handle first half-shell **21** may be ultrasonically welded to the handle second half-shell **23** using any ultrasonic welding techniques. Additionally, alternatively, or optionally, the hand half-shells **21, 23** may be manufactured using injection molding techniques know in the art.

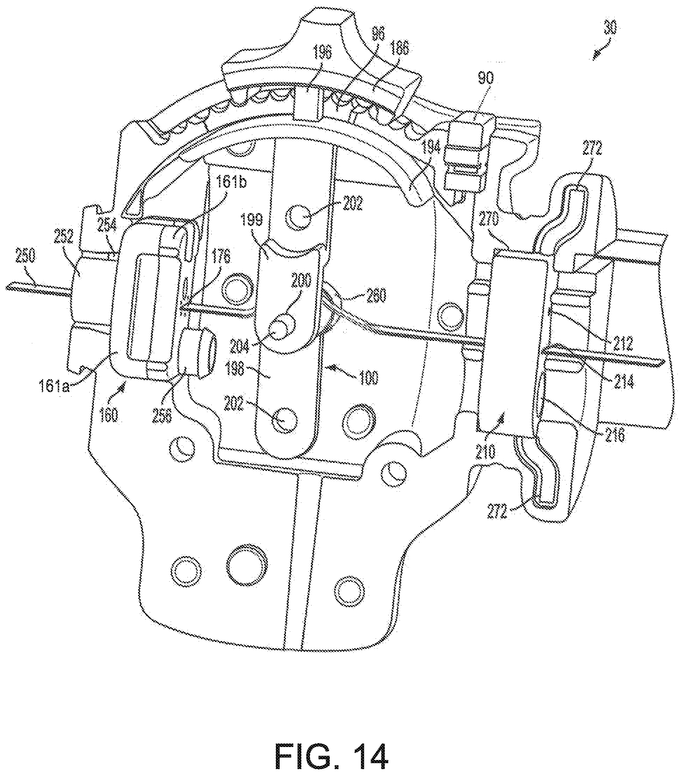

The handle **12** of the endoscope **10** may also include a handle distal section **30.** As shown in **FIG. 3B****,** the handle distal section **30** extends from the handle proximal section **16** toward the shaft **14.** Projecting from the bottom of the handle distal section **30** may be a handle fin or paddle **36.** The handle distal section **30** also includes a recess **35** which is sized to accommodate a finger contact **98** of a pivot control structure **100** (see, e.g. **FIG. 14**). Examples of pivot control structures **100** are also described below, and are used to rotate a pivotable sensor housing at the distal end of the shaft 14.

Also shown in FIG. 3B is a display or camera control button 37. It may be used to capture images produced by the image sensor at the distal end of the shaft 14. In some embodiments, a user can depress the button 37 using a pre-determined pattern or sequence to, for example, turn on or off a video recording of images shown on a display of the field of view of the image sensor at the distal end of the shaft 14, record a snapshot of the image shown on a display of the field of view of the image sensor at the distal end of the shaft 14, alter the brightness of the light elements (e.g. LED's) at the end of the endoscope shaft 14, or adjust other characteristics of the sensor or displayed image (such as, e.g., white balance, color saturation, digital magnification, etc..). It may be preferable to have the sensor characteristics and LED illumination controlled by a processor associated with a graphical user interface connected to the endoscope, rather than by the endoscope PCB itself in order to reduce the amount and cost of on-board processing power of the endoscope PCB.

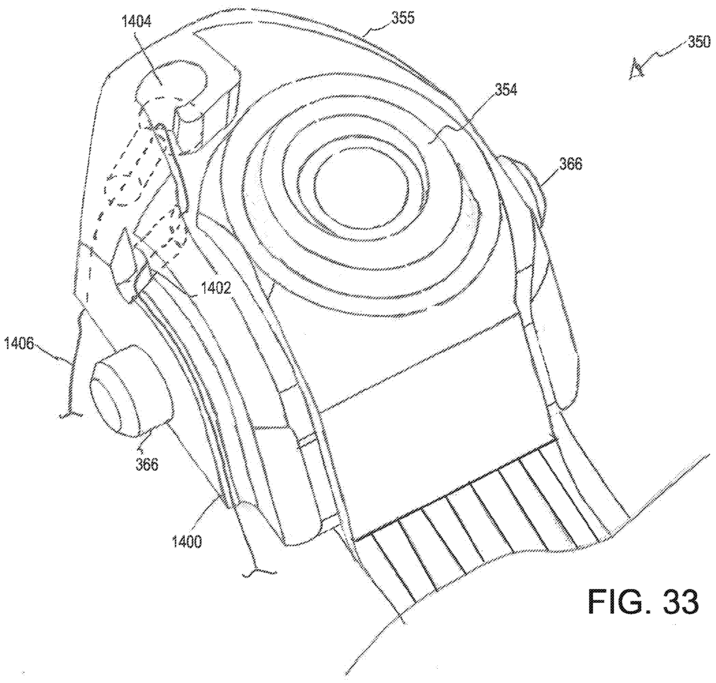

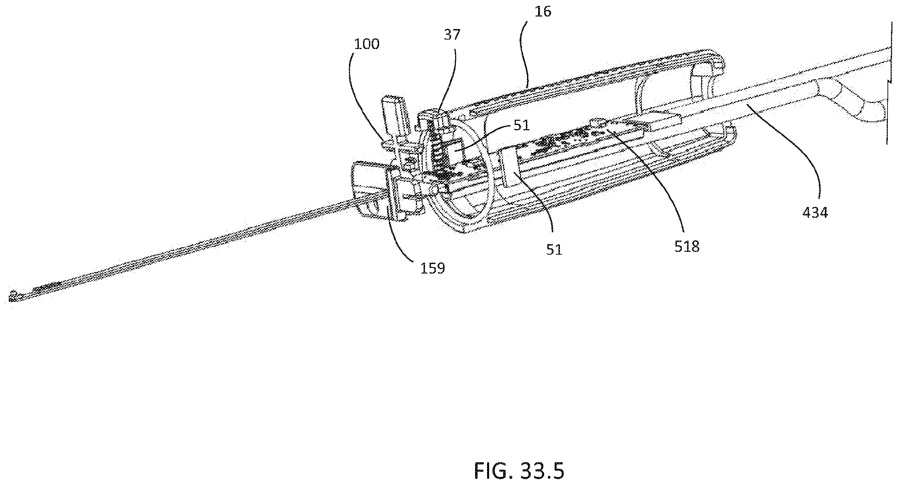

The button 37 may operate an electromechanical switch located on the main PCB within the endoscope handle distal section 30. To ensure maximum moisture resistance of the PCB electronic components, it may be preferable to employ a magnetic or optical sensor assembly to detect the movements of button 37. As shown in FIG. 33.5 (showing relative positions of some of the components within handle 12), in one embodiment a Hall effect sensor on the endoscope PCB 518 may be positioned below and near the location of a shaft 38 connected to button 37. The button 37 may be spring loaded, and the end of the shaft 38 closest to the PCB 518 can be made to include an embedded magnet. As the button shaft 38 is moved closer to or away from the PCB-based Hall effect sensor, the sensor can generate the appropriate signal corresponding to the position of the button shaft 38 and its duration in that position.

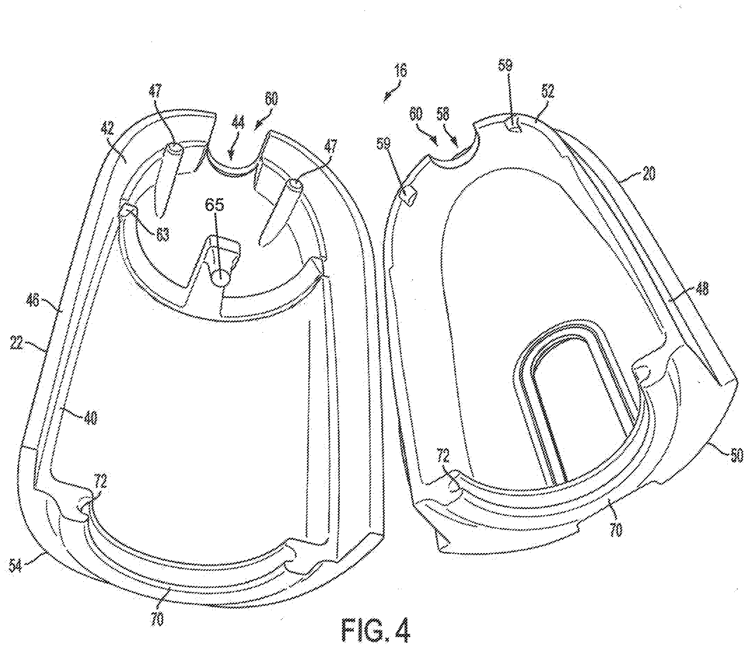

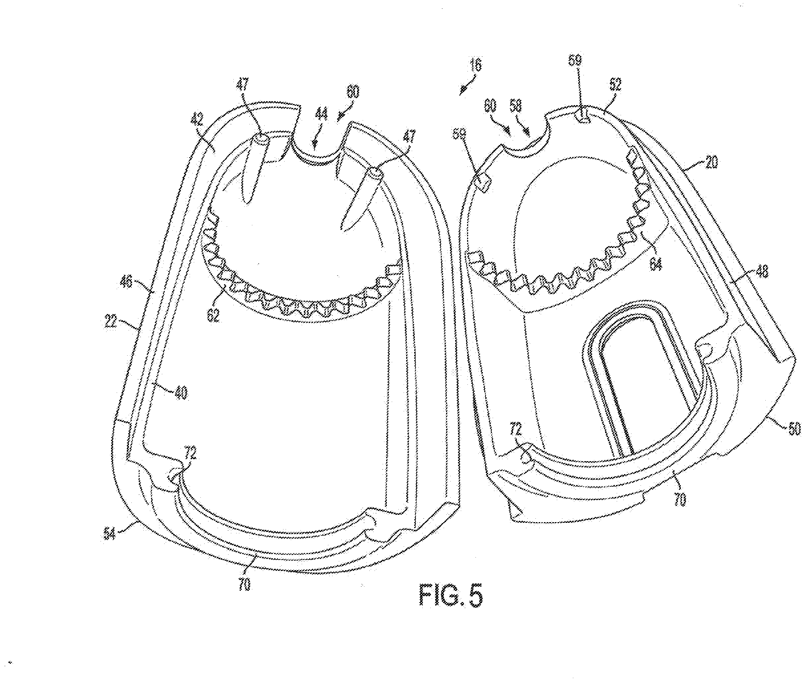

**FIG. 4** and **FIG. 5** show example embodiments of a handle top section **20** and handle bottom section **22** of the handle proximal section **16** shown in **FIG. 3A****.** The handle top section **20** and handle bottom section **22** are shown in an uncoupled or disassembled view. The handle proximal section **16** forms a shell-like structure when assembled. The handle bottom section **22** may include a ledge **40** that wraps around a bottom section inner wall **42** at a distance from the top face **46** of the handle bottom section **22.** As shown, there is a curved or U-shaped cutout **44** in the handle bottom section **22** disposed at an angle substantially perpendicular to the top face **46** of the handle bottom section **22.** Two peg projections **47** may be included near the rear of the handle bottom section **22.** The peg projections **47** may extend slightly above the ledge **40** and be angled approximately perpendicular to the top face of the ledge **40.**

As shown in **FIG. 4** and **5****,** a portion of the handle top section **20** may be dimensioned so that it may be overlapped by the handle bottom section **22** when the handle proximal section **16** is assembled. The overlapped section **48** may be stepped in from the handle top section outer surface **50** as shown in **FIG. 4** and **5****.** The height of the overlapped section **48** may be selected so that it is approximately equal to or slightly greater than the distance between the top of the ledge **40** of the handle bottom section **22** and the top face **46** of the handle bottom section **22.** In such embodiments, when fully assembled, the bottom face **52** (refers to orientation when assembled) of the handle top section **20** abuts the top of the ledge **40** of the handle bottom section **22.** Additionally in such embodiments, the handle top section outer surface **50** and handle bottom section outer surface **54** may be flush with each other and form a nearly continuous surface with little gap between the two. In some embodiments, there may be a small gap between the handle top section outer surface **50** and handle bottom section outer surface **54** (small gap shown in **FIG. 3**).

As shown, the handle top section **20** may include peg cutouts **59** which are shaped and disposed such that they may accept the peg projections **47** in the handle bottom section **22.** The handle top section **20** may include a curved cutout **58** at the butt or proximal portion of the handle top section **20.** As shown the curved cutout **58** may be recessed into the handle top section **20** at an angle substantially perpendicular to the bottom face **52** (refers to orientation when assembled) of the handle top section **20.** When the handle proximal section **16** is assembled, the curved or U-shaped cutout **44** of the handle bottom section **22** and the curved cutout **58** of the handle top section **20** together may form a substantially circular or ovoid handle void or opening **60** which will be further described below. It should be appreciated that the use of the terms "cutout", "cut", etc. herein should not be construed to imply material must be physically removed by a cutting or material removal process. In some embodiments, the curved or U-shaped cutout **44** and the curved cutout **58** may be formed during manufacture without physically removing material.

As shown in **FIG. 4** the handle bottom section **22** may include a shaft support member **63.** The shaft support member **63** in **FIG. 4** has a curved or semi-circular portion which roughly corresponds to the location of the toothed projection **62** in **FIG. 5****.** The shaft support member **63** also includes a post. The post projects perpendicularly from a mid-point of the semi-circular portion, leaving approximately 90° of the semi-circular portion on each side of the post. Projecting perpendicularly from the top of the post of the shaft support member **63** toward the distal end of handle proximal section **16** is a shaft supporting section **65.** The shaft supporting section **65** may include a depression in which a portion of a sensor gear shaft **120** (see **FIG. 8**) may be seated. The post of the shaft support member **63** may be approximately the length of the radius of the semi-circular portion when the handle proximal section **16** is fully assembled. The shaft support member **63,** toothed projection **62,** and toothed projection **64** will be further described below.

As shown in **FIG. 5****,** the handle bottom section **22** may instead or optionally include a curved toothed projection **62.** The curved toothed projection **62** is complemented by a similar toothed projection **64** included on the handle top section **20.** The toothed projection **62** and toothed projection **64** may be disposed so that they are in line with one another and form an annulus or internal ring gear when the handle proximal section **16** is fully assembled.

As shown in **FIG. 4** and **5****,** the face of the handle bottom section **22** opposite the curved or U-shaped cutout **44** and face of the handle distal section **20** opposite the curved cutout **58** may include semi-circular openings or voids **70.** A curved or U-shaped track **72** may be recessed into the edges of the semi-circular voids **70** along the entire arc of each semi-circular void **70** as shown in **FIG. 4** and **5****.**

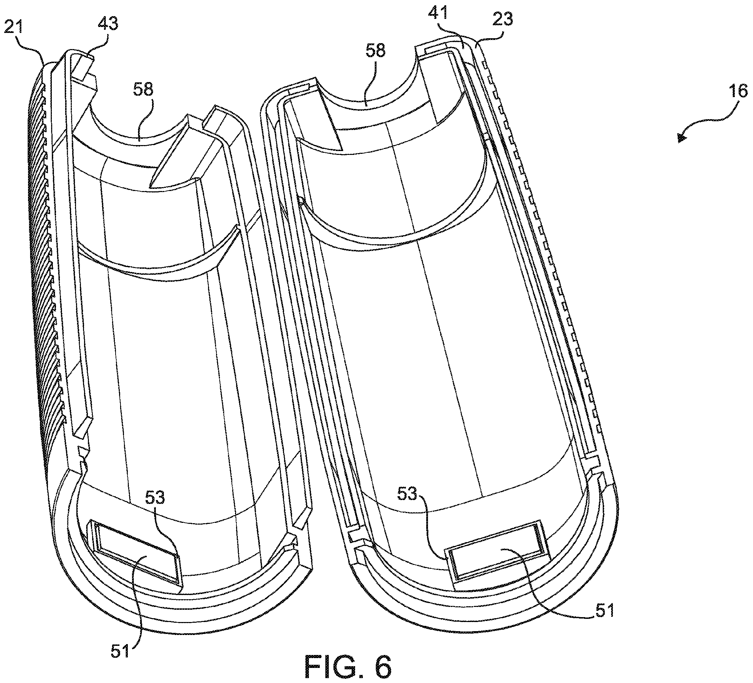

**FIG. 6** shows an example embodiment of a handle first half-shell **21** and handle second half-shell **23** of the handle proximal section **16** shown in **FIG. 3B****.** The handle first half-shell **21** and handle second half-shell **23** are shown in an uncoupled or disassembled view. The handle proximal section **16** forms a shell-like structure when assembled. One of the first or second handle half-shells **21, 23** may include a slot **41** which is sized to fit a cooperating wall extension **43** in the other of the first and second handle half-shells **21, 23** to facilitate assembly. Similar to **FIG. 4** and **5****,** the example embodiment in **FIG. 6** includes curved cutouts **58.** These cutouts **58** allow access into the interior volume of the proximal handle section **16** when the proximal handle section **16** is assembled.

It may be useful to be able to track the rotational orientation of the handle proximal section 16 in relation to the handle distal section 30, shaft 14, and the sensor or camera at the distal end of the shaft. In an embodiment, this can be accomplished through the interaction between a Hall effect sensor and an associated magnet. The Hall sensor may be positioned on the handle proximal section 16, with a magnet located on an internal component within the handle proximal section 16, or one or more magnets may be positioned in the handle proximal section 16 with one or more associated Hall effect sensors mounted on a PCB within the handle proximal section 16. In some embodiments, one or more magnets **51** may be embedded in or attached to a part of the handle **12.** In the example embodiment shown in **FIG. 6** the proximal handle section **16** includes two magnets **51** situated generally opposite each other. A different number of magnets **51** may be used in other embodiments. As shown, in this case, each of the first and second handle half-shells **21, 23** includes a magnet **51** inserted in an interior wall of each half-section. In the example embodiment, the magnets **51** are coupled to a retaining structure **53** which holds the magnet **51** in place in the proximal handle section **16.** The magnets optionally may be constructed of any appropriate rare earth or transition metal, or alloy thereof.

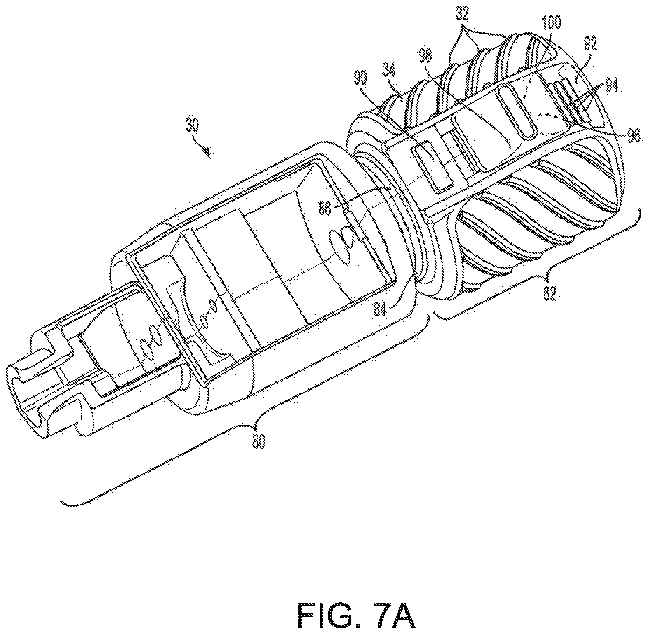

The example handle distal section **30** of **FIG. 3A** is shown in **FIG. 7A** isolated from the rest of the handle **12.** **FIG. 7A** shows the handle distal section **30** from a substantially top perspective view. As shown, the spiraling ribs **32** and front handle raised section **34** detailed above are visible on handle distal section **30.** As indicated by the seam running down the vertical center plane of the handle distal section **30,** the handle distal section **30** may be constructed as two or more separate parts (**30a** and **30b** in the example embodiment) which are coupled together by any suitable means or combination of suitable means, such as, e.g., snap fit, adhesive and/or screws.

The handle distal section **30** in **FIG. 7A** additionally includes a section not shown in **FIG. 3A****.** When the endoscope **10** is assembled, as it is in **FIG. 3A****,** part of the handle distal section **30** may be housed inside the handle proximal section **16.** For example, a housed handle electronics section **80** projects proximally from the external handle distal section **82** (which is visible in both **FIG. 3A** and **FIG. 7A**). The housed handle electronics section **80** will be further described below.

Between the housed handle electronics section **80** and the external handle distal section **82** is a small diameter span **84.** As shown, the small diameter span **84** may include a rounded groove **86** which is recessed into the outer surface of the small diameter span **84.** In some embodiments, when fully assembled, the small diameter span **84** of the handle distal section **30** may be disposed within the semi-circular openings **70** of the handle proximal section **16.** The rounded groove **86** in the small diameter span **84** and the curved or U-shaped track **72** in the semi-circular openings **70** may be in line with one another. This may allow the handle distal section **30** and handle proximal section **16** to be rotated relative to one another as the endoscope **10** is used. Optionally, ball bearings (not shown) or other types of bearings may track along the rounded groove **86** in the small diameter span **84** of the handle distal section **30** and the U-shaped track **72** in the semi-circular openings **70** of the handle proximal section **16.** In a preferred embodiment, an o-ring (not shown) may be placed in the rounded groove **86** of the small diameter span **84** of the handle distal section **30.** The o-ring (not shown) may function as a dynamic seal between the handle proximal section **16** and handle distal section **30.** In such embodiments, the handle proximal section **16** and handle distal section **30** may be rotated relative to one another while sealing the interior of the handle proximal section **16** from liquid.

A handle fin or paddle **36** or other protuberance may serve as an orientation marker for the user as the handle proximal portion **16** and handle distal section **30** are rotated relative to one another. Orientation may be checked either visually or by feel. In some embodiments, the gripping texture on the handle fin/paddle **36** may be different than spiraling ribs **32** on the rest of the handle distal section **30** to facilitate orientation-checking by feel.

As shown in **FIG. 7A****,** the handle raised section **34** may include a button **90.** In some embodiments, the handle raised section **34** may include more than one button **90,** or no button at all. The button **90** may be located elsewhere on the handle distal section **30** or elsewhere on the handle **12.** In some embodiments, the handle raised section **34** may include a button **90** and one or more additional buttons **90** may be located elsewhere on the handle **12.** In some embodiments, a button **90** may be a mechanically actuated switch including a depressible member which when depressed completes or breaks a circuit. The button **90** may comprise a magnetic or Hall effect based switche by embedding a magnet into a portion of the button near a Hall effect sensor within the handle distal section 30. Other types of buttons or switches may be used. The button **90** may be assigned multiple functions that may be activated by various user manipulations. In some embodiments one or more of the buttons **90** may be sealed with respect to the external handle section **82** to inhibit liquid infiltration.

The button **90** may be an image capture button. In such embodiments, depressing the button **90** may cause a photograph to be recorded of a display imaged generated by the endoscope **10.** In some embodiments, a user may double tap the button **90,** long-press the button 90, or hold down the button **90** to cause the display equipment connected to the endoscope **10** to start recording video. To stop recording video, a user may double tap the button **90,** long-press the button **90,** or release the button 90. In some embodiments, a user may only be required to depress and release the button **90** to stop recording video. In some embodiments, a single depression of the button **90** by a user while the endoscope **10** is recording video may cause a still image to be recorded without the need to pause video recording. In other arrangements, a quick press-and-release of the button 90 may trigger the recording of a still image, while a more prolonged press-and-release, or a press-and-hold may trigger the recording of a video segment.

The handle raised section **34** may additionally include a slide button recess **92.** As shown in **FIG. 7A****,** the slide button recess **92** is arranged to permit fore and aft movement of a slide button or finger contact **98** (see **FIG. 14**) while constraining lateral movement. The slide button may be part of a pivot control or pivot control structure **100** (see, for example, **FIG. 14**) in some embodiments. In some embodiments, including the example embodiment shown in **FIG. 7A****,** the slide button recess **92** may be slightly curved to conform to the shape of the portion of the handle within which it resides.

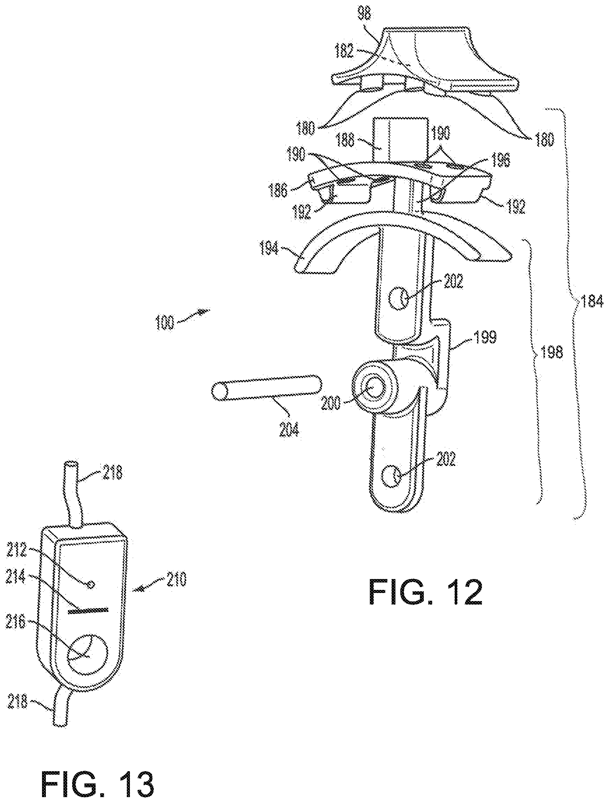

As shown in **FIG. 7A****,** the slide button recess **92** may include a number of ridges or detents **94** that can engage with a corresponding element on the slide button to provide a series of discrete, positive stops when a user moves the slide button fore and aft. Some embodiments may not include the ridges **94.** In some embodiments, the portion of a pivot control structure **100** (see **FIG. 12**) with which a user may interface may project through a pivot control structure notch **96** (see **FIG. 14**) located in the slide button recess **92** of the handle raised section **34.** In the example embodiment in **FIG. 7A****,** such a portion of the pivot control structure **100** includes a finger contact **98.** As shown, the finger contact **98** may have sloped contours for ergonomic reasons. The pivot control structure **100** will be further described below.

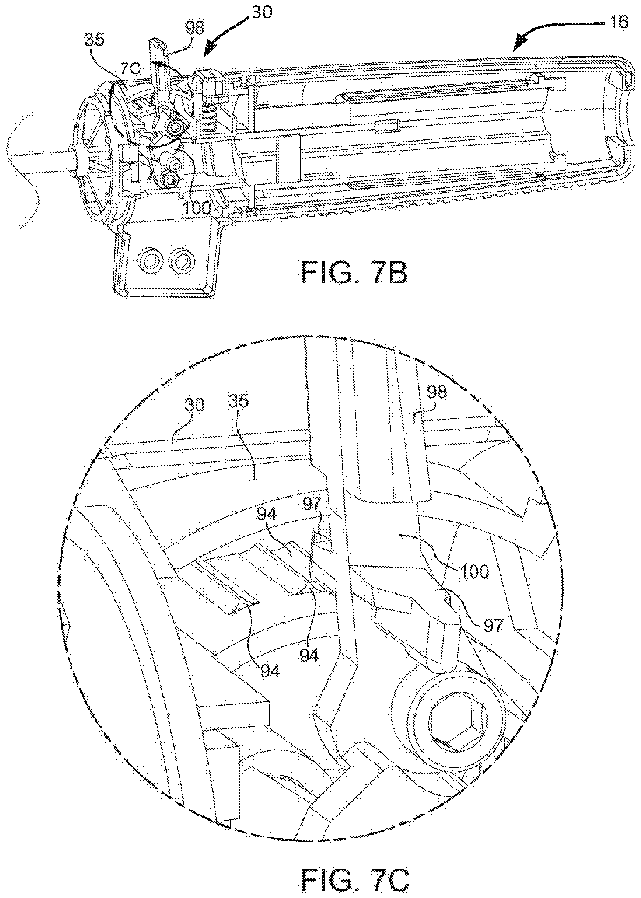

**FIG. 7B** and **FIG. 7C** depict an alternate embodiment of a recess **35** which may be used to accommodate a finger contact **98** of a pivot control structure **100.** A portion of the handle proximal section **16** and handle distal section **30** has been removed for clarity. **FIG. 7C** depicts a detail view of region **7C** of **FIG. 7B****.** As best shown in **FIG. 7C****,** in some embodiments, the recess **35** includes ridges **94** for stepwise movement of the pivot control structure similar to those of the slide button recess **96** shown in **FIG. 7A****.** Alternatively, the recess **35** may be generally smooth and may be curved to accommodate the travel path of a pivot control structure **100.**

The interior of the distal handle section **30** may include a shelf **95** which is located beneath the recess **35.** The shelf **95** may have a surface whose contour mimics the contour of the recess **35.** The shelf **95** may include one or more ridge(s) or detent(s) **94** to provide a series of discrete, positive stops when a user moves the pivot control structure **100** fore and aft. These ridges **94** may interact with one or more arms **97** extending from the pivot control structure **100.** The arms **97** may move freely over portions of the shelf **95** which are smooth and devoid of ridges or ribs **94.** When one of the arms **97** encounters a ridge **94,** the ridge may abut the respective arm of the arms **97** and impede further displacement of the pivot control structure **100** until a force sufficient to overcome the mechanical interference presented by the ridge **94** is applied. That is, the ridges **94** may form force barriers that impede the actuation of the finger contact **98** out of a dwell position. Depending on the embodiment, the ridges **94** may be placed in pairs along a face of the shelf **95.** The each rib or ridge **94** of the pair of ridges **94** may be placed apart by about the width of the arm **97** of the pivot control structure **100.**

**FIG. 8** shows a more detailed illustration of an exemplary handle distal section **30** without an attached insertion section **14.** An example rotation sensing assembly **150** is also shown in **FIG. 8****.** As shown, the handle distal section **30** is manufactured as two separate parts **30a** and **30b.** In the example embodiment, the two separate parts **30a** and **30b** of the handle distal section **30** include a number of screw holes **102,** which may be threaded. Screws (not shown) or other suitable fasteners may be used to couple the two separate parts **30a** and **30b** of the handle distal section **30** together. In some embodiments, the two separate parts **30a** and **30b** may be coupled together via a snap fit, ultrasonic weld, adhesive, etc.

In some embodiments one of the two separate parts **30a** and **30b** of the handle distal section **30** may include peg-like projections **104** which fit into complimentary peg accepting cavities **106** on the other of the two separate parts **30a** and **30b.** This may help to align and/or couple the two separate parts **30a** and **30b** together. In some embodiments, including the embodiment shown in **FIG. 8****,** the external handle distal section **82** may be substantially hollow. In some embodiments, the hollow of the external handle distal section **82** may not be sealed against fluid. In the example embodiment shown in **FIG. 8****,** a drain channel **108** may be included, for example, in the handle fin **36.** The drain channel **108** may allow any fluid which enters the hollow of the external handle distal section **82** to easily drain out. Alternate embodiments may include additional and/or different drain arrangements.

The handle distal section **30** may also include a rotation sensor holder **110** as shown in **FIG. 8****.** The rotation sensor holder **110** may retain the rotation sensing assembly **150** when the endoscope **10** is fully assembled. As shown, the rotation sensing assembly **150** may include a forward gear **112.** The forward gear **112** is disposed about a forward gear shaft **114.** As shown in **FIG. 8****,** a transfer gear **116** is also placed on the forward gear shaft **114,** such that rotation of the forward gear **112** causes the transfer gear **116** to rotate as well. The transfer gear **116** may mesh with a sensor shaft gear **118,** disposed on a sensor gear shaft **120.** As the forward gear **112** rotates, so will the sensor shaft gear **118** and the sensor gear shaft **120.** Use of a gear assembly may allow for placement of an attached potentiometer **122** in a location that is off-center from the central rotational axis of the handle distal section **30,** which may advantageously allow for a central placement of other internal structures (e.g., irrigation conduit, optical fiber bundle, electronic flex cable, or other electronic components).

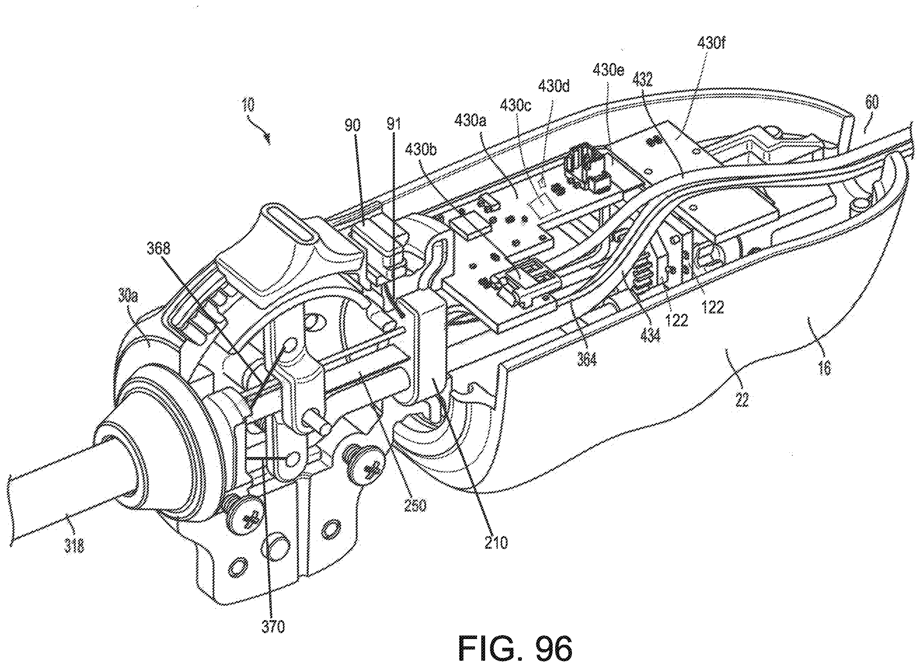

As in the example embodiment in **FIG.8****,** the sensor gear shaft **120** may include a splined, or keyed (e.g., a D-shaped) portion. The keyed portion may operatively engage with one or more rotational potentiometers **122.** In the example embodiment in **FIG. 8****,** there are two rotational potentiometers **122.** The potentiometers **122** may be mounted on or otherwise attached to a mounting element, or a part of a printed circuit board in the handle as described in reference to **FIG. 96****.** The potentiometers **122** each include a keyed (e.g. D-shaped) void with which the corresponding keyed portion of the sensor gear shaft **120** mates. As the sensor gear shaft **120** rotates, the electrical resistance of the potentiometer(s) **122** will vary proportionately. Since the resistance will predictably change with the amount of rotation of the sensor gear shaft **120** the measured resistance of the potentiometer(s) **122** may be used to determine the amount of rotation that has taken place between the handle proximal section **16** and the handle distal section **30** (and by extension, the insertion section **14**).

In some embodiments, the housing of each potentiometer **122** may be mounted to elements of the housed handle electronics section **80** (or other elements attached to the handle distal section **30**), and thus immobilized relative to the handle distal section **30** (and by extension the insertion section **14**), while the shaft or rotating hub of the potentiometer **122** is connected to the handle proximal section **16.** In other embodiments, the housing of the potentiometer **122** may be immobilized relative to the handle proximal section **16,** while its shaft or rotating hub may be connected to elements of the handle distal section **30** or the handle electronics section **80.**

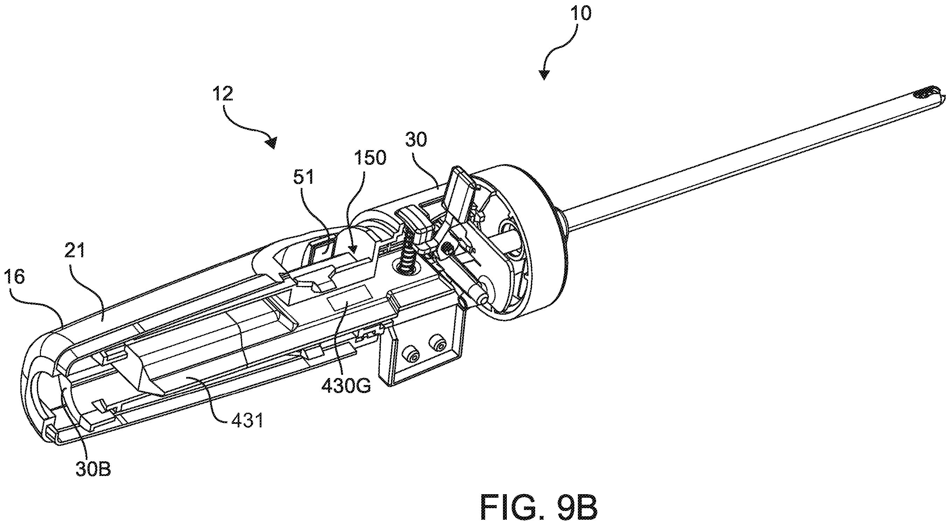

The example embodiment in **FIG. 8** includes two rotational potentiometers **122** stacked together, and offset rotationally from one another. In an alternate embodiment, the potentiometers **122** may be spaced apart from each other, but share a common rotational axis (e.g., the wipers of both potentiometers **122** are caused to move by a common shaft). This arrangement permits a controller receiving electrical resistance values from both potentiometers **122** to compute the degree of rotation of a sensor shaft (and ultimately of components at the distal end of the endoscope) with a desired accuracy through 360 degrees of rotation, thus helping to eliminate computational "blind spots" in measuring the rotation of the components at the distal shaft (e.g., a camera) of the endoscope. Any blind spot created by the position of a wiper of one potentiometer **122** at the end of its range of motion may be compensated for by a wiper of a second potentiometer **122** whose position is not at the end of its range of motion. In alternative embodiments, more than two rotationally offset potentiometers **122** may be used. The rotational offset between the potentiometers **122** may be 180 degrees for computational simplicity, but other angular offsets may be used to achieve the same result, as long as the rotational offset allows any blind spot created by one potentiometer **122** to be overlapped by a functional range of another potentiometer **122.** In alternative embodiments, the gearing ratios between the forward, transfer, and sensor shaft gears **112, 116, 118** may vary, depending on the degree of precision desired in measuring rotation, the sensitivity of the potentiometers **122,** and other factors. In alternative embodiments, the rotation sensing assembly **150** may use belts rather than one or more of the gear assemblies. For example, the transfer gear **116** and sensor shaft gear **118** may be replaced by a belt. Other rotation to rotation arrangements known in the art may also be used. In some embodiments, the forward gear shaft **114** may include a keying feature (e.g., a D-shaped portion) which operatively engages the potentiometers **122** directly. Rotation sensors other than potentiometers **122** may also be used. Alternative embodiments may include rotation sensors such as, a rotary encoder, a rotary variable differential transformer, or other encoding devices. In embodiments using a rotary encoder, the encoder may be a gray encoder, magnetic encoder (see e.g., **FIG. 9B**), optical encoder, etc.

In an embodiment, the sensor gear shaft **120** may not extend to the shaft bearing section of a shaft support member **63.** Rather, the rotation sensing assembly **150** may be supported by the rotation sensor holder **110.** Among other benefits, this arrangement allows for an unlimited degree of rotation of the handle distal section **30** relative to the handle proximal section **16.** Additionally, as would be appreciated by one of skill in the art, it allows for components a of rotation sensing assembly **150** to be located in an off-center position. This may provide benefits during assembly. For example, it may simplify routing of an irrigation line **434** (see **FIG. 96**), power cable **432** (see **FIG. 96**), etc.

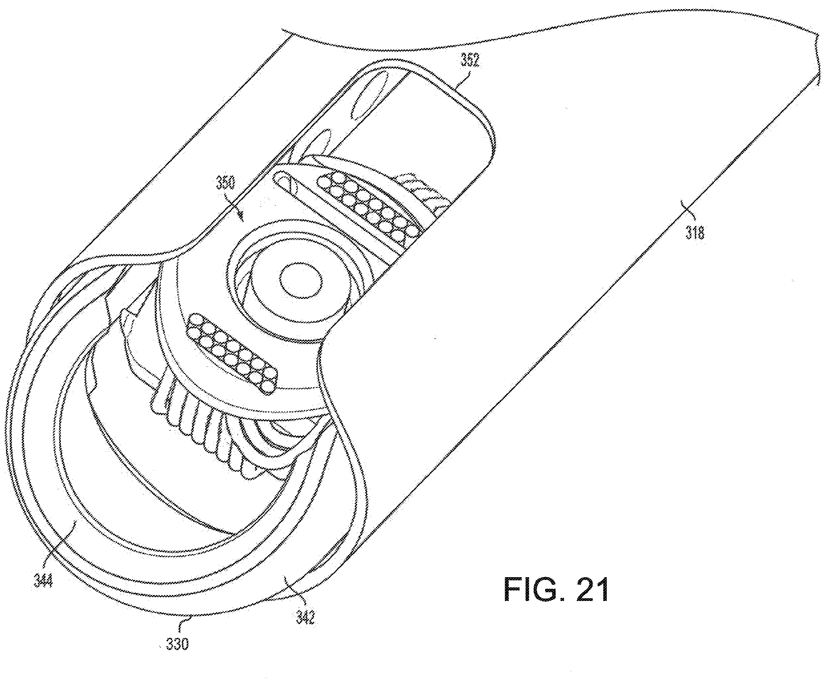

In other embodiments, the shaft support member **63** and potentiometers **122** may be directly connected by a shaft. A shaft splined or keyed on a distal end may extend from the shaft bearing section of the shaft support member **63** and extend through a corresponding splined or keyed (e.g., D-shaped) void in the potentiometers **122.** Since the shaft support member **63** may be fixed relative to the handle proximal section **16,** rotation of the distal handle section **30** relative to the handle proximal section **16** will vary the resistance measured by the potentiometers **122.** As mentioned above, since the resistance will predictably change with rotation of one handle section relative to the other, the resistance measurement may be used to determine the amount of rotation achieved by the handle distal section (and ultimately, the distal end of the endoscope or camera assembly **350** shown, for example, in **FIG. 21**).

In other embodiments, the rotation sensing assembly **150** may include a range finder which may be disposed on the housed handle electronics section **80** (see **FIG. 7A**). The interior walls of the handle proximal section **16** (see **FIG. 4**) may include a variable-thickness or variable-height raised surface that wraps around most or all of the 360 ° of the interior wall of the handle proximal section **16,** and varies in thickness or height in a pre-determined manner along its circumferential path. As the handle proximal section **16** and handle distal section **30** rotate relative to one another, the range finder may provide a controller with a signal that varies according to the distance read by the range finder to the varying surface (either its varying thickness or height). The signal may be correlated to the thickness/height or distance measured by the range finder relative to a pre-determined base position in which the surface has a specified thickness or height and is correlated to a specified angular rotation of the handle distal section **30** relative to the handle proximal section **16.** This distance may be compared to a previous distance to thereby determine the amount of rotation that has occurred. The range finder may be any type of range finder (e.g. a mechanical position sensor, a sonic range finder, laser or other optical range finder, etc.).

In yet another alternative embodiment, an optical mouse like sensor arrangement may be used. The sensor may be mounted on one of the housed handle electronics section **80** or handle proximal section **16** and may be configured to track movement of the other of the housed handle electronics section **80** or handle proximal section **16.** In such embodiments, the amount and direction of movement sensed by the sensor may be used to determine the amount and direction of rotational displacement that has occurred. In some embodiments, the surface tracked by the sensor may have a reference grid, number of unique indicators, pattern, markings, or other differentiating features, which allow sensor determination of rotational orientation upon start up. Other varieties of rotation sensing assemblies **150** known to those skilled in the art may also be used in various embodiments.

As shown in **FIG. 8****,** the rotation sensor holder **110** of the handle distal section **30** may be shaped such that when the two separate parts **30a** and **30b** of the handle distal section **30** are coupled together, the rotation sensing assembly **150** may be captured between the two separate parts **30a** and **30b.** Each side of the rotation sensor holder **110** may include a forward gear shaft trough **124** and a sensor gear shaft trough **126.** When assembled the forward gear shaft trough **124** and the sensor gear shaft trough **126** may act as bearing surfaces respectively for the forward gear shaft **114** and the sensor gear shaft **120.** Each side of the rotation sensor holder **110** may also include a holder void **128.** The holder void **128** may be sized and shaped such that the transfer gear **116,** sensor shaft gear **118,** and potentiometers **122** may fit within the rotation sensor holder **110** when the handle distal section **30** is fully assembled.

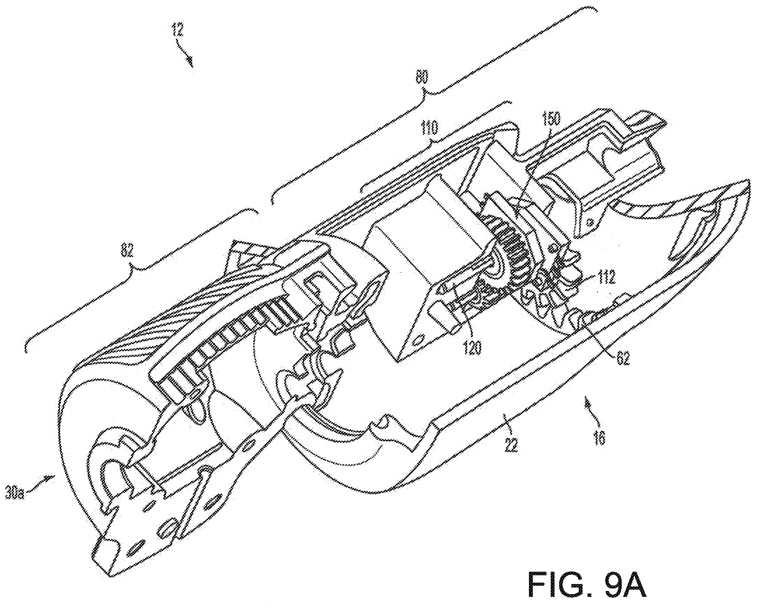

**FIG. 9A** shows a partially assembled view of the handle **12** of the endoscope **10.** Only the handle bottom section **22** of the handle proximal section **16** is shown in **FIG. 9A****.** As shown, a part of the handle bottom section **22** of the handle proximal section **16** has been cut away. Additionally, in the embodiment shown in **FIG. 9A****,** the handle distal section **30** is assembled from two separate parts **30a** and **30b** (see **FIG. 8**). One of the halves (**30b**) of the handle distal section **30** has been removed in **FIG. 9A** for clarity. (In the embodiment shown in **FIG. 9A****,** the handle distal section **30** is assembled from two separate parts **30a** and **30b** (see, e.g. **FIG. 8**). One of the halves (**30a**) of the handle distal section **30** has been removed in **FIG. 9A** for clarity). The housed handle electronics section **80** may be located inside the handle proximal section **16.** The external handle distal section **82** extends beyond the handle proximal section **16** and is exposed to the environment.

As described above, the rotation sensing assembly **150** is disposed within the rotation sensor holder **110.** As shown, the forward gear **112** of the rotation sensing assembly **150** may mesh with the annulus gear formed by the toothed projection **62** and toothed projection **64** (best shown in **FIG. 5**). In such embodiments, when the handle **12** is fully assembled, any rotation of the handle distal section **30** in relation to handle proximal section **16** causes the forward gear **112** to rotate since it meshes with the annulus gear formed by the toothed projection **62** and the toothed projection **64.** This rotation may then be translated through the rest of the rotation sensing assembly **150** allowing the rotation to be measured by the rotation sensing assembly **150.** In a preferred embodiment, the overall gear ratio may be approximately 1:1.

Alternatively, rather than gear elements, the handle proximal section **16,** similar to that shown in **FIG. 4****,** may comprise a keyed shaft or partially keyed shaft, affixed to the shaft support section **65** of the shaft support member **63.** The keyed portion of the shaft may be arranged to mate with the hub of one or more potentiometers **122,** which are held in rotation sensor holder **110.** Thus as the handle distal section **30** is rotated relative to the handle proximal section **16,** the wiper of the one or more potentiometers **122** is able to convert the relative positions of the handle distal section **30** and proximal section **16** into an electrical resistance value usable to determine rotational orientation.

Referring now to **FIG. 9B****,** an alternative embodiment of an example handle **12** of an endoscope **10** including a rotation sensing assembly **150** is depicted. Only the handle first half-shell **21** is shown in **FIG. 9B** to make the interior of the handle **12** visible. Additionally, a portion of the handle first half-shell **21** has been cut away.

Shown in Fig. 9B is an enclosure **431** for a printed circuit board (PCB) that comprises electronic components for processing image data from the image sensor at the distal end of the shaft, and optionally for providing power to light sources (e.g. LED's) at the distal end of the endoscope shaft. The enclosure 431 is an optional structure, because the PCB may also or additionally be encased in a water resistant material. The water resistant material may be any suitable potting material, such as, for example, Parylene, or other chemical vapor deposited polymer to coat and protect the individual electronic components mounted on the PCB. Also shown is a magnet **51** which is included in the handle first half-shell **21.** The printed circuit board within enclosure **431** may include one or more magnetic position sensors **430g** such as a Hall effect sensor or sensor array. As mentioned above in relation to **FIG. 6****,** each of the handle first half-shell **21** and handle second half-shell **23** may include a magnet **51** or multiple magnets **51** in some embodiments. In an embodiment, two magnets 51 are positioned opposite each other in each half of the handle 16. As handle proximal section **16** is rotated relative to the handle distal section **30** the magnet(s) **51** move relative to the enclosure **431** and the enclosed printed circuit board. The magnetic sensor(s) **430g** on the printed circuit board can detect the relative positions of the magnet(s) **51** through variations in magnetic field strength and location as the magnets move relative to the printed circuit board. In an embodiment, a single tri-axis position sensor is used for sensing the magnets 51. Data from the one or more sensors can be transmitted to a controller or processor for conversion of the sensor data into rotational position of the handle proximal portion 16 relative to the handle distal section 30 (and relative to the position of the optical sensor or camera at the distal end of the endoscope shaft). Thus, a displayed image of the field of view of the camera can be rotated to any desired orientation without actually moving the camera at the distal end of the endoscope shaft.

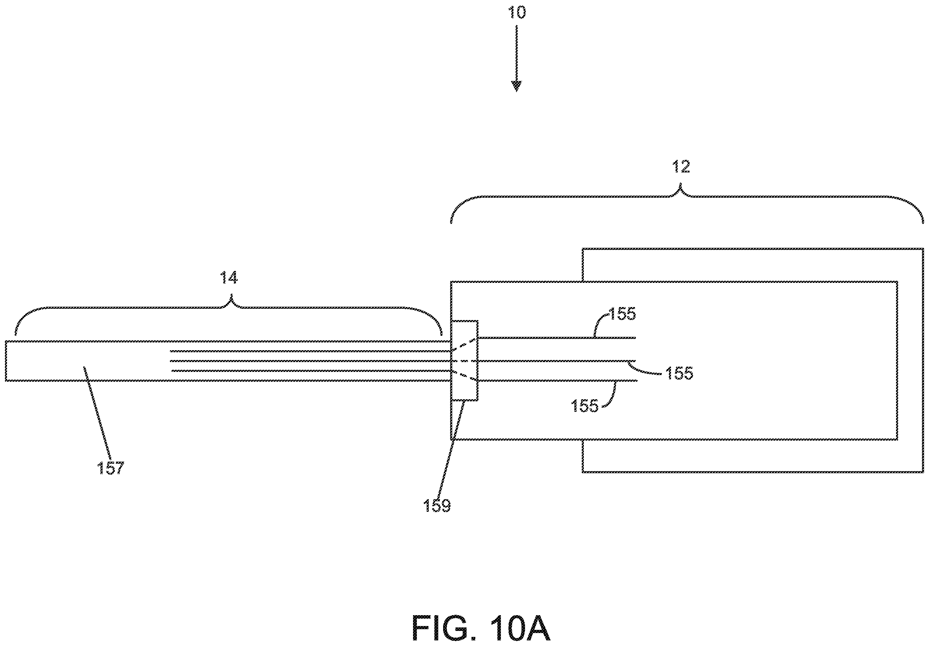

Referring now to **FIG. 10A****,** in an embodiment, the insertion section **14** of an endoscope **10** includes a conduit **157** through which operations or functions may be performed. In industrial or medical applications, this conduit **157** may be used to pass instruments to manipulate objects at the end of the insertion section **14** (instruments such as graspers, forceps, clamps, wire baskets, dilators, knives, scissors, magnetic pickups, etc.). Fluid (gas or liquid) may also be passed to/from an external source from/to the space within which the insertion section **14** is placed. In medical applications, such a conduit **157** may be used to insufflate a body cavity with a gas, evacuate gas from a body cavity, irrigate a space with liquid, or aspirate liquid and/or suspended particulates from a space. The conduit **157** optionally may carry utility components such as light transmission, information transmission, power transmission, and mechanical control components, saving space within the insertion section **14** and helping to reduce the overall diameter of the insertion section **14.** A light transmission component may include, for example, a fiberoptic bundle, ribbon, light pipe, light projection element, and/or the like. An information transmission component may include, for example, an electrical cable bundle or ribbon connecting an imager or image sensor at the end of the insertion section **14** to an image processing unit situated in the handle **12** or external to the endoscope **10.** Such a cable may also provide power to the image sensor. Mechanical control components may include, for example, pushrods, pull wires, etc. to control the movement of an element near the end of the insertion section **14.** This may include, for example, an actively flexible distal segment of the insertion section **14** that can be actively flexed by the use of the mechanical control component(s) extending from the handle **12.** It may also include, for example, a rotatable camera or camera mount at the end of insertion section **14** that can be actively moved by the use of the mechanical control component (s) extending from the handle **12.**

In an embodiment, a fluid carrying conduit **157** within the insertion shaft or section **14** is configured to enclose utility components of the endoscope **10,** such as, for example, fiberoptic bundles, communication cables and mechanical actuators. In a further embodiment, the conduit **157** may be in fluid communication with a camera assembly **350** (see, for example, **FIG. 21**) at the distal end of insertion shaft **14.** The camera assembly **350** may include a camera sensor or imager having connections to a communications cable. In this case, the camera sensor and communications cable connections, and the internal components of any associated lens assembly may be sealed against exposure to liquids present within the conduit **157.** Allowing a camera assembly **350,** lens assembly, communications cable, mechanical actuators (e.g. pull-wires) and fiberoptic cables or bundles to be exposed to a 'wet' conduit may be feasible if at least a portion of the endoscope **10** is configured to be a single use device, i.e., disposable after use in a medical procedure. Any technical challenges in adequately sterilizing intra-conduit components are thus obviated.

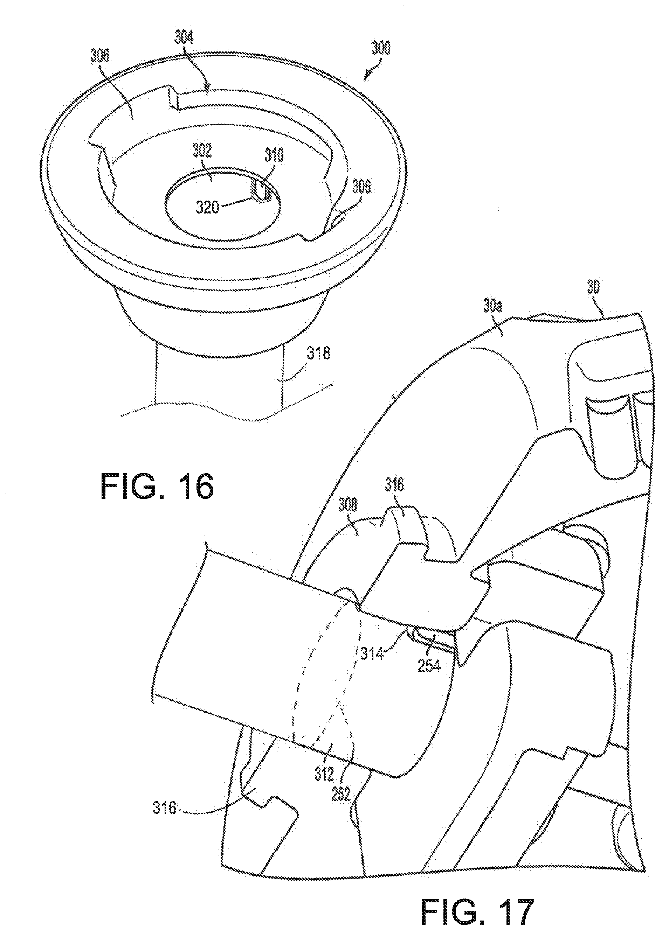

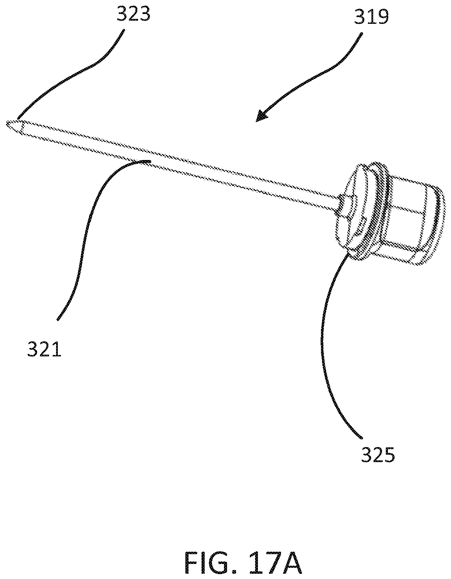

Some components of the endoscope **10,** particularly electronic components located within the handle section **12,** preferably should be kept dry. A bulkhead or barrier element **159** between the conduit **157** of the insertion section **14** and the interior of the handle **12** may allow passage of components from the handle **12** to the insertion section **14** conduit **157** (represented in **FIG. 10A** by line segments **155** and referred to as pass-through components), while also inhibiting infiltration of fluid from the conduit **157** into the interior space of the handle **12.** The barrier **159** may comprise passageways (holes, slits, etc.) through which pass-through components **155,** such as the utility components described supra, may pass from the handle **12** to the conduit **157** of the insertion section **14.** The passageways may be formed to provide a relatively tight fit around the outside surface of the pass-through components **155.** In some embodiments, elastomeric gaskets, O-rings, or other similar elements may further aid in inhibiting fluid infiltration from the conduit **157** of the insertion section **14** to the interior spaces of the handle **12.** The barrier **159** may comprise a wall separating a junction region between the handle **12** and a proximal end of the insertion section **14.** The junction region may be near an area where the conduit **157** connects to a conduit port providing an external fluid connection for the conduit **157.** The barrier **159** may alternatively comprise a block through which a routing channel connects a utility hole communicating with the conduit **157** on a first side of the block with one or more features (e.g. a conduit port) on a second side of the block opposite the first side of the block, or on a third side of the block (which in some embodiments, may be roughly perpendicular to the first side of the block). Passageways for cables, ribbons, wires, pushrods or other components from the handle **12** may be formed on the second side of the block, opposite the first side of the block and may be aligned with the utility hole of the block. The conduit **157** may be formed from a sheath (such as inner sheath **312** of **FIG. 17**) connected or attached to the handle **12** of the instrument. In some embodiments, the pass-through barrier **159** between the handle **12** and a sheath of the insertion section **14** may comprise a sheath mount, which serves to support the sheath of the insertion section **14** near its origin proximally at the handle **12,** and to attach or connect it to the handle **12.** In some embodiments, the insertion section **14** may comprise a cannula within which the sheath may be positioned. The cannula may be mounted to the handle **12** via a disconnect feature, allowing the cannula to remain in situ while the endoscope **10 -** including handle **12** and sheath - can be withdrawn from a site.

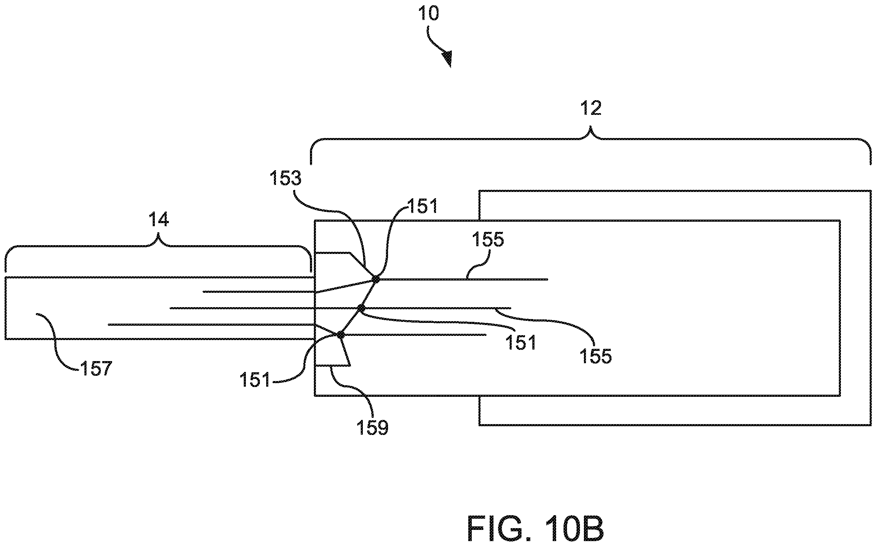

Referring now to **FIG. 10B****,** in some embodiments, the barrier **159** may include a flexible or elastomeric member **153.** One or more pass-through components **155** may extend through the flexible member **153** of the barrier **159** to the conduit **157** of the insertion section **14.** In some embodiments, one or both of the entry and exit points of the pass-through component **155** in the flexible member **153** may be sealed with a sealing member or agent **151.** The sealing member or agent **151** may prevent the flow of fluid between the conduit **157** and the handle **12.** The sealing member or agent **151** may also hold the pass-through component **155** such that it is prevented from moving relative to its entry and/or exit point in the flexible member **153.** Any suitable sealing member or agent **151** may be used, such as, for example, a glue, epoxy, or other adhesive. In other embodiments, the pass-through components may be solvent bonded, heat bonded, etc. to the flexible member **153.** In yet other embodiments, the flexible member **153** may be formed in place around the pass-through components **155** during manufacture such that a seal is created between the pass-through components **155** and the flexible member **153.**

As the pass-though components **155** move (e.g. actuating pull wires for rotation of a camera assembly) the flexible member **153** may stretch or flex since the pass-through components **155** are fixed and prevented from displacing relative to their entry and exit point in the flexible member **153** due to the sealing member or agent **151.** Thus, ingress or leakage of fluid into the handle from the conduit **157** may be substantially or totally inhibited while allowing the pass-through components **155** to move back and forth. Pass-though components **155** which remain in fixed position and do not displace need not necessarily pass though the flexible member **153.** Instead, these components may pass through a rigid portion of a barrier **159** which may or may not be coupled to the flexible member **153.** The flexible member **153** may be constructed, for example, as an elastomeric member, a flexible membrane, flaccid wall, a bellows-like arrangement, diaphragm, etc.

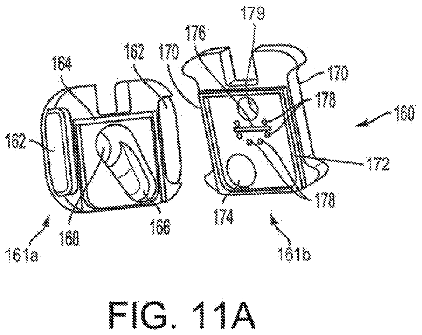

A barrier **159** described in relation to **FIG. 10A** is shown in **FIG. 11A** and can also be referred to as an inner sheath mount **160.** As shown, the inner sheath mount **160** includes a distal section **161a** and a proximal section **161b,** separated in **FIG. 11A** from one another to reveal the interior of the inner sheath mount **160.** As shown the distal section **161a** may include notches **162** on each side of the distal section **161a.** As shown in the example embodiment in **FIG. 11A****,** a portion of an interior face **164** (when assembled) of the distal section **161a** may be recessed. An irrigation or suction routing channel **166** may also be recessed into the distal section **161a** of the inner sheath mount **160.** As shown, the irrigation routing channel **166** is located within the recessed face **164.** The irrigation routing channel **166** may be in communication on a first end with a utility hole **168.** In the example embodiment, the utility hole **168** may be located substantially near the center of the distal section **161a,** within the recessed face **164** (although in other embodiments, the utility hole **168** need not be centered).

The proximal section **161b** of the inner sheath mount **160** may also include notches **170** in its right and left sides similar to the notches **162** recessed into distal section **161a.** The notches **170** may extend all the way through the proximal section **161b.** The notches **162** and **170** of the inner sheath mount **160** may be sized to accept a projection of the handle distal section **30,** which may help to hold the inner sheath mount **160** in place when the endoscope **10** is fully assembled.

The proximal section **161b** may also include a raised portion **172** of an interior face (when assembled). As shown, the raised portion **172** is of similar outer dimensions as the recessed face **164** in the distal section **161a.** When assembled, the raised portion **172** may be pressed into the recessed face **164** to couple the distal section **161a** and proximal section **161b** together. In some embodiments, glue or another suitable adhesive between the recessed face **164** and raised portion **172** may be used to bind the proximal section **161b** to the distal section **161a.** This may also serve to create a hydraulic seal between the two components.

The proximal section **161b** may include a number of other features. As shown, the proximal section **161b** includes an irrigation or suction passage **174.** The irrigation or suction passage **174** may be situated to align with a second end of the irrigation routing channel **166** when the proximal section **161b** is mated to the distal section **161a.** When the endoscope **10** is in use, irrigation or suctioned fluid may flow between the utility hole **168** and irrigation passage **174** via the irrigation routing channel **166.**

As shown in the example embodiment in **FIG. 11A****,** the proximal section **161b** of the inner sheath mount **160** may include a sheath mount slit **176.** As shown, the sheath mount slit **176** may be oriented horizontally (orientation refers to that shown in **FIG. 11A**) and located in the proximal section **161b** of the inner sheath mount **160,** roughly aligned with the utility hole **168.** The sheath mount slit **176** may be oriented differently in alternate embodiments. In the example embodiment in **FIG. 11A****,** the sheath mount slit **176** extends through the entire proximal section **161b** at an angle substantially perpendicular to the plane of the interior face (when assembled) of the proximal section **161b.**

The proximal section **161b** of the inner sheath mount **160** may also include a number of orifices **178.** In the example embodiment in **FIG. 11A****,** the orifices **178** are small diameter holes which extend through the entire proximal section **161b,** and can be used to allow passage of pull or push cables or wires from within the handle to the distal end of the endoscope **10.** The proximal section **161b** may also include a fiber optics passageway **179.** In the example embodiment, the orifices **178** and fiber optics passageway **179** are angled perpendicular to the interior face (when assembled) of the proximal section **161b.** In alternate embodiments, the orifices **178** and fiber optics passageway **179** may be angled differently or may have a different diameter. As shown, the orifices **178** are arranged around the sheath mount slit **176.** When the inner sheath mount **160** is fully assembled, the sheath mount slit **176** and orifices **178** are aligned with the utility hole **168** of the distal section 161a.

In alternative embodiments, the shape, location, dimensions, etc. of some features of a bulkhead, pass-through barrier or inner sheath mount **160** may differ. A pass-through barrier or inner sheath mount **160** may include additional features or may omit certain features. In some embodiments, there may be a larger or smaller number of orifices **178.** In some embodiments, the orifices **178** may not be arranged in the spatial arrangement shown in **FIG. 11A****.** There may be more than one irrigation passage **174.** In some embodiments, the inner sheath mount **160** may be associated with or include a gasket to further inhibit fluid infiltration into sensitive areas within the handle of the endoscope.

The handle electronics section **80** (see, e.g., Fig. 7A) is configured to enclose mechanical and electronic components that are preferably protected against excessive amounts of fluid infiltration. (Small amounts of fluid or moisture need not inhibit proper mechanical or electrical operation of the endoscope, particularly if the electronic components are coated with a moisture resistant film). The handle distal external section **82** (the pivot control housing), is configured to house the pivot control structures and actuation cables for controlling movement of a camera assembly in the distal end of the endoscope shaft or insertion shaft, and may be exposed to liquid with relatively minimal effect on the operation of the endoscope. Therefore, it is more important to maintain a liquid seal between the handle electronics section **80** and the handle distal external section **82.** A bulkhead or pass-through barrier such as sealing member **210,** shown in **FIG. 13** and **14** may be constructed to provide a tight seal (e.g. elastomeric seal) around an electronic flex cable, an optical fiber bundle, or other structures that must pass from the distal end of the endoscope to its proximal end before exiting. On the other hand, a pass-through barrier such as inner sheath mount **160,** shown in **FIG. 11A** and **14****,** may allow for a lesser seal, particularly as it may apply to any pull wires or cables that pass from the pivot control structure to the distal end of the endoscope shaft. Any fluid infiltration into the handle distal section **82** may be allowed to exit the housing through one or more drain holes or passages built into a dependent part of the housing, such as for example, passage **108** shown in **FIG. 8****.**

In an alternate embodiment, a pass-through barrier **159** (see **FIG. 10B**) between the handle distal section or pivot control housing **82** and the shaft of the endoscope may comprise a fully sealed structure that yet permits movement of the pull cables or actuation cables that extend from the pivot control housing to the distal end of the endoscope shaft. For example, the pass-through barrier may comprise a flexible (or floppy) diaphragm, a pleated elastomeric diaphragm, accordion-structured rubber boot, bellows structure, or otherwise displaceable diaphragm that is attached at its periphery to the housing, that forms a fluid-tight seal around any structures passing through it near its central region, and whose central region may freely move back and forth distally and proximally to permit free movement of any pivot control cables passing therethrough. With a more complete seal at this portion of the endoscope, the need for a secondary seal between the pivot control housing and the handle electronics section **80** may be reduced or eliminated.

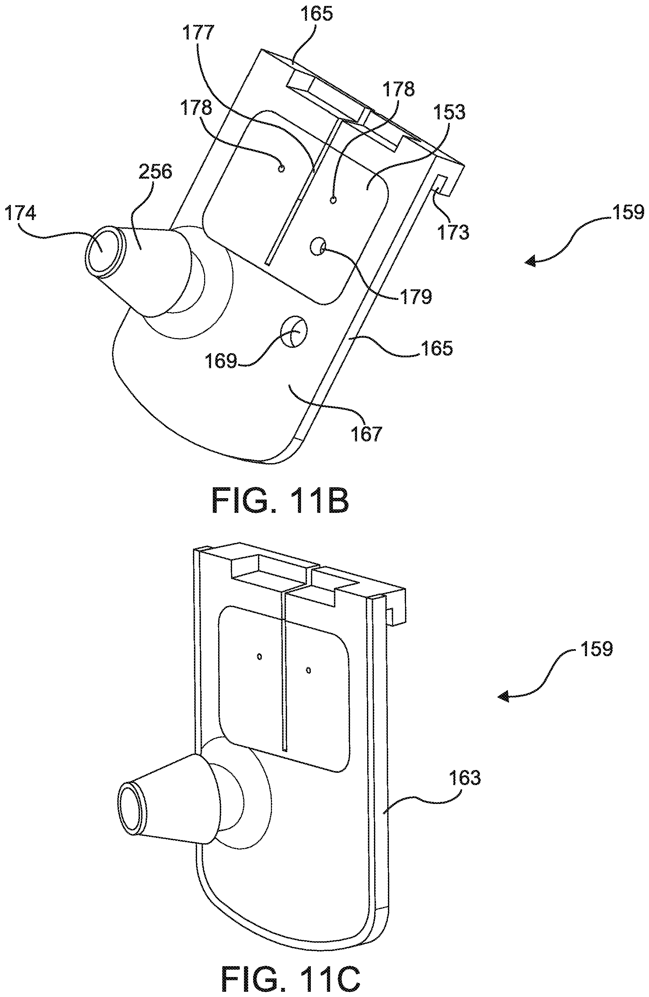

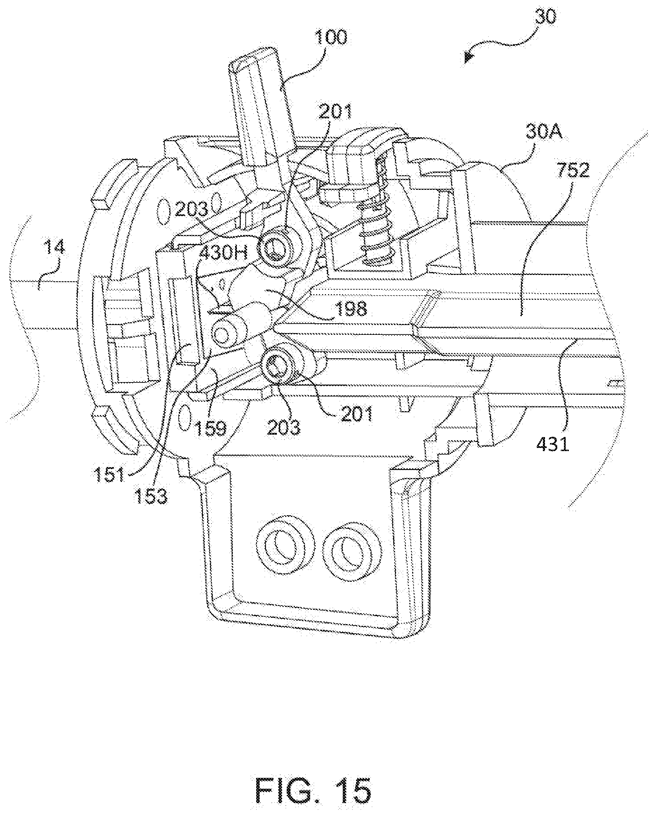

**FIG. 11B** depicts an alternative embodiment of a bulkhead or pass-through barrier **159** which comprises a flexible member **153.** As shown, the pass-through barrier **159** includes a rigid structure **167** and a flexible member **153.** A rigid portion or structure **167** may act as a frame to which the flexible member **153** is attached or fused. In some embodiments, a dual molding process may be used to couple the flexible member **153** and the rigid portion or structure **167** together during manufacture. The rigid structure **167** may include one or more mating features **173** which may be sized to mate with a cooperating mating feature of the handle distal section **30** (see, e.g. **FIG. 15**). In some embodiments, the interaction of the mating feature(s) **173** with the cooperating portion of the handle distal section **30** (see, e.g. **FIG. 15**) may create a seal between the pass-through barrier **159** and the handle distal section (see, e.g., **FIG. 15**).

Referring now also to **FIG. 11C****,** to facilitate the creation of such a seal, a gasket member **163** may be included around the periphery of the pass-through barrier **159.** Such a gasket member **163** may be placed along the outer edges **165** (**FIG. 11B**) of the pass-through barrier **159.** Alternatively, a dual molding process may be used to attach the gasket member **163** to the pass-through barrier **159** during manufacture. The gasket member **163** may completely encircle the rigid structure 167, and may be formed from a compressible or elastomeric material, such as, e.g., Metaprene^{®}.

Still referring to **FIG. 11B** and **FIG. 11C****,** the flexible member **153** includes a number of pass-through elements. A number of orifices **178** are included in the example flexible member **153.** Additionally, the flexible member **153** may include an optional illumination or fiber optic passage **179.** Such an illumination passage **179** may not be needed in embodiments in which illumination is provided by one or more LEDs at the distal end of the endoscope shaft, for example. Additionally, a slit or slot **177** may be included in the flexible member **153.** In the example embodiment, the slit **177** extends from the flexible member **153** through the rigid structure **167** to the edge of the pass-through barrier **159.**

Pass-through elements and passages in the pass-through barrier **159** may also be disposed in the rigid frame structure **167** of the pass-through barrier **159** as well. It may be desirable, for example, that pass-through elements or passages associated with fixed or non-displacing components be disposed in the rigid structure **167** of the pass-through barrier **159.** In the example embodiment a rigid structure passage **169** is shown providing a passageway through the rigid structure **167.**

A conduit attachment site or port **256** may also be included on a pass-through barrier **159.** As shown, the conduit attachment port **256** projects from the rigid structure **167** and optionally includes a barbed fitting over which a flexible tube or conduit may be secured. A conduit attachment site **256** may include an interior lumen which extends through the pass-through barrier **159.** In the example embodiment shown in **FIG. 11B** the interior lumen is an irrigation or suction passage way **174** through which fluid may be transferred from one side of the pass-through barrier **159** to the other.

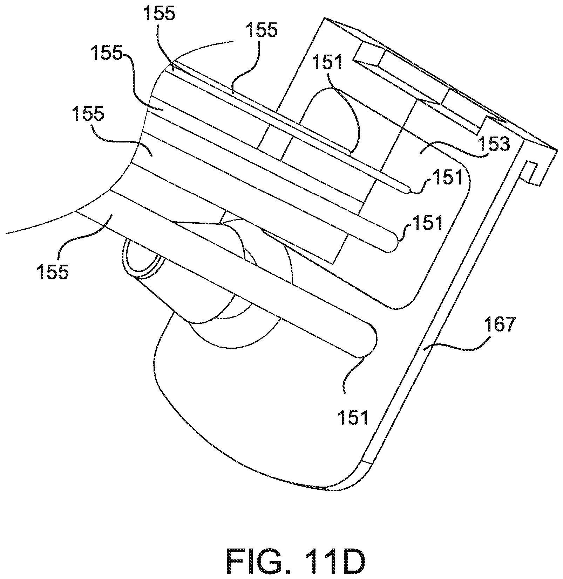

Referring now also to **FIG. 11D****,** when assembled, various pass through components **155** may be passed through the orifices **178,** passage **179** and slit **177** in the pass-through barrier **159.** Once these pass through components **155** have situated in the pass-through barrier **159,** a sealing member or agent **151** optionally may be applied to one or both of the entry and/or exit points of the pass through components **155** in the pass-through barrier **159.** In the example embodiment, the sealing member or agent **151** is fixative such as adhesive though in other embodiments any suitable sealing member or agent may be used. The sealing member or agent **151** may prevent fluid communication through the pass-through elements in the pass-through barrier **159.** Additionally, when applied on the flexible member **153** it may fix pass through components **155** such that may not displace relative to their entry and exit point in the flexible member **153.** As a result, in this case displacement of the pass-through components **155** will cause the flexible member **153** to move back and forth as well.

**FIG. 12** shows an example exploded view of an embodiment of a pivot control structure **100.** The pivot control structure **100** may control pivoting of a structure. The structure may for example be a camera assembly **350** (see **FIG. 21****)** at a distal end of the insertion section **14** (see **FIG. 3A**). In alternate embodiments, the pivot control structure **100** may be used to instead or additionally control the flexing of a flexible section of the insertion section **14.** Some embodiments of the pivot control structure **100** may include gearing, a motor, multi-bar linkage, dials, etc. that differ from the embodiment disclosed below.

The example pivot control structure **100** in **FIG. 12** is shown in an exploded view. The finger contact **98** detailed above is shown separated from the pivot control structure **100.** As shown, the bottom face of the finger contact **98** optionally may include a number of peg projections **180.** In the example embodiment shown in **FIG. 12****,** there are four peg projections **180** which are generally cylindrical in shape (number and shape of peg projections may differ). The finger contact **98** additionally includes a finger contact slot **182** situated in the under-surface of the finger contact **98.**

Below the finger contact **98,** an example embodiment of a pivoting portion **184** of the pivot control structure **100** is shown. The top of the pivoting member **184** of the pivot control structure **100** may include a slider **186.** Projecting from the center of the slider **186** is a finger contact post **188** arranged to mate with finger contact slot **182.** Optionally, finger contact peg holes **190** flank the finger contact post **188** on each side of the finger contact post **188.** When the finger contact **98** is attached to the pivot control structure **100** the finger contact slot **182** may be slid onto the finger contact post **188** on the slider **186.** Additionally, when assembled, the peg projections **180** of the finger contact **98,** if present, may be seated in the finger contact peg holes **190** of the slider **186.**

A pivot control structure **100** may interact with one or more feature of the endoscope allowing it to be locked or held in a desired orientation. As shown, the bottom face of the slider **186** of the pivoting member **184** optionally may include one or more catch bars or detent elements **192.** In other embodiments, multiple catch bars **192** may be disposed along the bottom of the slider **186,** arranged to engage with opposing raised features or ridges **94** on the handle **12.**

The catch bars or detent elements **192** may interact with the raised features or ridges **94** of the slide button recess **92** of the handle raised portion **32** described above (best shown in **FIG. 7A**). As the pivot control structure **100** is displaced by the user, the spaces between ridges **94** may act as detents in which the catch bars **192** of the slider **186** may be "parked". This helps to prevent drifting or movement of the pivot control structure **100** once a user moves it to a desired position and releases it. It may also help to ensure that the pivot control structure **100** is not accidentally displaced during use of the instrument. In alternative embodiments, and as mentioned above in relation to **FIG. 7C****,** in some embodiments, the pivot control structure **100** may include arms **97** which act as catch bars or detent elements **192.**

As shown, the pivoting member **184** of the pivot control structure **100** includes a curved inner shield **194.** The inner shield **194** is tiered below the slider **186,** and under the handle housing when assembled. A post **196** may span the distance between the top face of the inner shield **194** and the bottom face of the slider **186.** In some embodiments, the catch bars **192** may be located on the top of the inner shield **194.** In such embodiments, the ridges **94** described above may be located on the interior wall of the housing of the handle distal section **30** such that the ridges **94** may form detents for the catch bars **192** on the inner shield **194.** As described above, this allows the pivot control structure **100** to be "parked" in a desired position.

Extending from the bottom face of the inner shield **194** may be a pivot arm **198.** In the example embodiment, the pivot arm **198** includes two mechanical cable attachment points or holes **202.** One hole **202** is situated on one side of a pivoting shaft **204,** while the second hole **202** is situated on the other side of pivoting shaft **204.** In the illustrated embodiment, forward movement of slider **186** causes a mechanical cable connected to the lower hole **202** to be retracted proximally, while aft movement of slider **186** causes a mechanical cable connected to the upper hole **202** to be retracted proximally. In order to accommodate a relatively unimpeded passage of a fiberoptic or electrical cable from the proximal end of the handle to the distal end of the handle, the pivot arm **198** may be, for example, notched over its pivot shaft **204,** so that a passing cable may rest freely on the pivot shaft **204** (or a concentric sleeve or hub surrounding the shaft **204**). Such an arrangement would allow passage with minimal displacement laterally or vertically.

Referring now to both **FIGS. 12** and **14****,** the pivot arm **198** is constructed to have a laterally displaced section **199** encompassing pivoting region **200** and pivot shaft **204.** Thus a hub or sleeve encompassing pivot shaft **204** (when assembled) is shown to serve as a bearing surface upon which a passing cable **250** may rest. A lower portion of pivot arm **198** extends downward from a location beneath the hub or sleeve of pivot shaft **204.** In some embodiments, the lower portion of pivot arm **198** optionally may be vertically aligned with the upper portion of pivot arm **198,** so that mechanical cables connected to points or holes **202** are also aligned vertically. In other embodiments, one or more cables (e.g., cable **250**) may travel around (or through) a hub of pivot shaft **204** in a variety of other ways, so that its path is minimally obstructed by the pivot arm **198** of the pivot control structure **100.**

Optionally, a secondary pass-through seal provides an additional barrier between fluid that may infiltrate into the housing of the handle distal section **30** and the housing of handle proximal section **16,** in which electronics section **80** may be housed. The seal may include orifices, holes or slits through which components such as though not limited to, fiberoptic bundle, electronic cable and/or fluid conduit tubing may pass. The holes or slits may be sized to provide a snug fit over these components as they pass through the seal. In an embodiment, the secondary pass-through seal is formed from a rubber or other elastomeric material to enhance its fluid sealing characteristics. In some embodiments, for example, those which include a pass through barrier **159** which includes a flexible member **153,** no secondary pass-through seal may be included. In such embodiments, the electronics section **80** and the external handle section **82** may be the same volume or connected volumes.

**FIG. 13** shows an example embodiment of a secondary seal, i.e. sealing member **210.** The sealing member **210** may be roughly rectangular in shape as shown in **FIG. 13****.** As shown in **FIG. 13****,** one end of sealing member **210** may be of a first (e.g. a rectangular) shape, while a second end of sealing member **210** may be of a second shape (e.g. have rounded edges or be rounded). This may provide an advantage during assembly to ensure that the sealing member **210** is mounted in the proper orientation. The sealing member **210** may include a number of orifices. In the example embodiment, the sealing member **210** includes a fiberoptic bundle (e.g., an illumination fiber) orifice 212, a flex cable (i.e., electronic cable) orifice **214,** and a fluid tubing (e.g., an irrigation line) orifice **216.** In the example embodiment shown in **FIG. 13** the illumination fiber orifice **212,** flex cable orifice **214,** and irrigation line orifice **216** extend through the entire sealing member **210.** The illumination fiber orifice **212** has a relatively small diameter to match the diameter of a fiber bundle or light pipe. The flex cable orifice **214** is a slit, matching the size and shape of an electronic flex cable. The irrigation line orifice **216** is cylindrical and has a diameter larger than that of the illumination fiber orifice **212.** The illumination fiber orifice **212,** flex cable orifice **214,** and irrigation line orifice **216** extend through the sealing member **210** at an angle that is substantially perpendicular to the front face (relative to **FIG. 13**) of the sealing member **210.** In alternative embodiments, the orifices in the sealing member **210** may differ in number, size or shape. In some embodiments, the sealing member **210** may include an additional hole for wiring to the button **90,** for example.

As shown in the example embodiment in **FIG. 13****,** the sealing member **210** may also include a number of gasket arms **218.** In the example embodiment in **FIG. 13****,** the gasket arms **218** project away from the top and bottom faces of the sealing member **210** near the back edge of the sealing member **210.** As shown, there may be two gasket arms **218.** In some embodiments, the gasket arms **218** may be straight. In the example embodiment, the gasket arms **218** include two straight sections connected by an arcuate section which bends the gasket arms **218** away from the sealing member **210.**

**FIG. 14** shows an example embodiment of one half (**30a**) of the handle distal section **30.** As shown, the inner sheath mount **160,** pivot control structure **100** and the sealing member **210** are assembled and placed within the shown half (**30a**) of the handle distal section **30.** A flex cable **250** (e.g., flexible electronic communications/power cable) is also shown. In the example embodiment shown in **FIG. 14****,** the distal section **161a** of the inner sheath mount **160** includes a sheath mounting hub **252.** The sheath mounting hub **252** extends distally along the same axis as the utility hole **168** (see **FIG. 11A**). In the example embodiment, the sheath mounting hub **252** may be hollow and substantially cylindrical. The inner diameter of the sheath mounting hub **252** optionally may be approximately equal to or somewhat larger than the diameter of the utility hole **168.** In the example embodiment, a sheath mount mounting tab **254** projects superiorly from the outer surface of the sheath mounting hub **252.** The sheath mount mounting tab **254** is located next to the face of the insertion side piece **160a** from which the sheath mounting hub **252** projects. The mounting tab **254** may serve to properly orient a sheath (e.g. inner sheath **312** shown in **FIG. 17**) as it is mounted onto the sheath mounting hub **252,** and optionally may also serve as a locking member to secure a sheath to the sheath mounting hub **252** and sheath mount **160.**

In other embodiments, the sheath mount tab **254** may be disposed on the inside surface of the sheath mount hub **252.** This may be desirable because it obviates the need to nest the inner sheath mount hub **252** inside of a sheath removing a restriction in the diameter of the conduit of the sheath. Consequentially, a higher flow rate through such a conduit may be achieved. Alternatively, a sheath mount nub **254** may not be included in some embodiments. The sheath may instead be oriented and secured to a sheath mount hub 252 in any suitable fixture (not shown).

As shown the flex cable **250** extends through the inner sheath mount **160.** The flex cable **250** passes through the sheath mounting hub **252** into the distal section **161a** of the inner sheath mount **160.** The flex cable **250** is also routed through the sheath mount slit **176** of the proximal section **161b.**

The proximal section **161b** of the inner sheath mount **160** includes a fluid conduit attachment site or port **256.** The fluid conduit attachment site **256** may be a hollow, roughly cylindrical projection which extends toward the right of the page (in relation to **FIG. 14**) from the proximal section **161b** of the inner sheath mount **160.** Tubing of an irrigation line **434** (see **FIG. 96**) may be slid over the outer surface of the fluid conduit port **256,** which optionally may be barbed to aid in retaining an installed section of tubing. As shown, the right edge of the fluid conduit port **256** may be chamfered in a manner to also facilitate ease of installation of a tubing segment to the port **256.** Additionally, as shown in **FIG. 14****,** the proximal end of the fluid conduit port **256** tapers to a slightly larger diameter than the rest of the port **256** surface. This may act as a barb and help ensure that once attached, the tubing of an irrigation line **434** (see **FIG. 96**) is not easily dislodged. In an alternative embodiment, the conduit port **256** may extend and be fitted into an irrigation line orifice **216** of a sealing member **210.** The barbed portion/attachment site for an irrigation line **434** may then be placed on the sealing member **210.**

The pivot control structure **100** may be pivotally coupled into the handle distal section **30** as shown in **FIG. 14****.** As shown, the pivot shaft **204** extends through the pivot shaft hole **200** in the pivot arm **198** of the pivot control structure **100.** The end of the pivot shaft **204** (or of a surrounding hub) inserted into the far wall of the handle distal section **30** may be seated in a pivot bearing **260** projecting from the inner wall of the handle distal section **30.** When fully assembled, the opposite end of the pivot shaft **204** may similarly be seated in a pivot bearing **260** projecting from the inner wall of the other half (**30b**) of the handle distal section **30.**

As shown in **FIG. 14****,** the slider **186** and inner shield **194** of the pivot control structure **100** may be offset from each other by the post **196** a distance slightly larger than the thickness of the walls of the handle distal section **30.** The post **196** may extend through the pivot control structure notch **96** described above. The curvature of the slider **186** and inner shield **194** may be selected such that the slider **186** and inner shield **194** may freely move fore and aft with input from a user without interfering with the walls of the handle distal section **30** housing. The length of the pivot control structure notch **96** may determine the amount of pivotal displacement a user may create with input to the pivot control structure **100.**

In some embodiments, the walls of pivot control structure notch **96** may exert a frictional force against the post **196.** In such embodiments, this frictional force may allow the pivot control structure **100** to be "parked" in a position. In such embodiments, the walls of the pivot control structure notch **96** may be made of a high friction material such as rubber or other elastomeric material. In such embodiments, the pivot control structure **100** may not need to include the catch bars **192** or the ridges **94** described above.

The endoscope **10** may also include mechanical pivot actuators in the form of pull cables or wires, belts, or pushrods. An actuator may be any elongate member, solid, braided, or otherwise extending from the handle of the endoscope **10** to a movable element at the distal end of the insertion section. The elongate member may be flexible or substantially rigid. The elongate member may be round (as in the example of a cable), ovoid, relatively flat, or may have any other shape or cross section. In some embodiments, the actuator may be a belt.

In an endoscope having a pannable camera or camera mount at or near the distal end of the shaft or insertion section, the pannable camera or camera mount may be rotated using pull wires or pushrods. In a pull wire embodiment, panning cables may be attached or connected to, or looped through the cable attachment holes **202.** In some embodiments, two panning cables may be attached to each cable attachment hole **202.** In a preferred embodiment both ends of a single panning cable are attached to each cable attachment hole **202** creating a loop. Alternatively, a single cable may be looped through the cable attachment hole **202** at about its midpoint, the ends of the cable then being connected distally to the rotatable camera or camera mount. The panning cables may extend from the cable attachment holes **202** in the pivot arm **198** and be routed through one or more orifices **178** in the proximal section **160b** of the inner sheath mount **160.** The panning cables may then extend through the utility hole **168** and through the conduit formed by the inner sheath, optionally alongside the length of an electronic flex cable **250** and/or fiberoptic bundle. By pivoting the pivot control structure **100,** the panning cable or cables connected to one of the cable attachment holes **202** will be pulled, while the cable(s) connected to the other attachment hole **202** will slacken. By attaching the panning cable or cables associated with one cable attachment hole **202** to one side of a pivot point and attaching the panning cable or cables associated with the other cable attachment hole **202** to the opposite side of the pivot point, the pivot control structure **100** may be used to selectively rotate a pivoting object distally in the insertion section of the endoscope. In other embodiments, a similar cabling mechanism may be used to actively flex a flexible distal segment of the insertion section.

In some embodiments, the pivot arm **198** of the pivot control structure **100** may be pivoted via gearing. In such embodiments, the finger contact **98,** finger contact post **188** (see **FIG. 12**), slider **186,** vertical post **196,** and inner shield **194** may not be needed. At least a portion of a user input gear contained in the handle distal section **30** may project out of the handle raised section **34.** The user input gear may be rotated about a pivot axis disposed within the handle distal section **30.** This rotation may be user-initiated via, for example, a user's finger or thumb. The user input gear may mesh with a pivot shaft gear disposed about the pivot shaft **204** for the pivot arm **198** of the pivot control structure **100.** In such embodiments, as the user input gear is rotated, the pivot shaft gear and pivot arm **198** are also caused to rotate, acting on the pivot actuators (e.g. panning, actuating or pull wires) as described above. In some embodiments, there may be an intermediary gear or any number of intermediary gears between the user input gear and the pivot shaft gear to provide any desired gear reduction to meet precision-of-movement and ergonomic requirements.

In other embodiments, the pivot arm **198** may be caused to rotate via an electric motor (e.g., brushless motor, stepper motor, etc.). Rotation via the motor may be controlled by one or more user input means such as a button **90.** In embodiments including at least one button **90,** the button **90** or buttons **90** may control the speed and direction of movement of the pivot arm **198.**

In some embodiments, the pivot shaft **204** may project to the outside of the handle distal section **30.** In such embodiments, the pivot shaft **204** (or an overlying hub or sleeve) may be directly rotated by the user. In some embodiments, the portion of the pivot shaft **204** projecting out of the handle distal section **30** may include a knob, dial, crank, etc. so that a user may easily rotate the pivot shaft **204** by grasping and rotating the knob, dial, crank, etc.

As shown in **FIG. 14****,** the sealing member **210** is positioned in a gasket recess **270.** The gasket recess **270** may include gasket arm recesses **272.** Various components may pass through the sealing member **210** as mentioned above. As shown, a flex cable **250,** connected to a printed circuit board **430a** (see, for example, **FIG. 96**) in the electronics section **80** housed in the handle proximal section **16** may pass through the flex cable orifice **214** of the sealing member **210** and extend beyond the sealing member **210** through the housing of the handle distal section **30** and sheath mount **160,** ultimately to travel distally in the insertion section of the endoscope. The irrigation line **434** (see **FIG. 96**) and fiberoptic bundle (e.g., illumination fibers **364,** see **FIG. 96**) may pass through their respective irrigation line orifice **216** and fiberoptic bundle orifice **212** and extend through the housing of the handle distal section **30** similar to the flex cable **250.** In some embodiments, a sealing member **210** may not be included. Instead the electronics section **80** may not be partitioned from the rest of the handle **12.** In such embodiments, the enclosed printed circuit board **431** (see, e.g. **FIG. 15**) may be coated or encased in a protective coating or layer, such as potting. In embodiments including a sealing member **210** a printed circuit board (see, e.g. **FIG.** 15) may still be encased in a protective coating or layer. Additionally or alternatively, the inner sheath mount **160** may include a flexible member **153** similar to that shown in **FIG. 11B-C** which forms a seal around and displaces as any pass-through components (e.g. flex cable **250,** actuators/cables, illumination fibers, etc.) running through the inner sheath mount **160** are displaced.

Only one half of the gasket recess **270** is shown in **FIG. 14****.** The other half of the gasket recess **270** may be located on the other, not shown half (**30b,** see **FIG. 8****,** for example) of the handle distal section **30.** When fully assembled, the sealing member **210** is captured between the two halves of the gasket recesses **270.** When fully assembled the sealing member **210** may ensure that fluid which may be present in the handle distal section **30** may be inhibited from infiltrating into the handle proximal section **16,** which contains electronics components comprising electronics section **80.** The sealing member **210** may be made of suitably compliant (e.g., elastomeric) material or other suitable gasket material and may be pressed into the gasket recesses **270** to ensure a tight seal. In some embodiments, the sealing member **210** may be held in place using an adhesive.

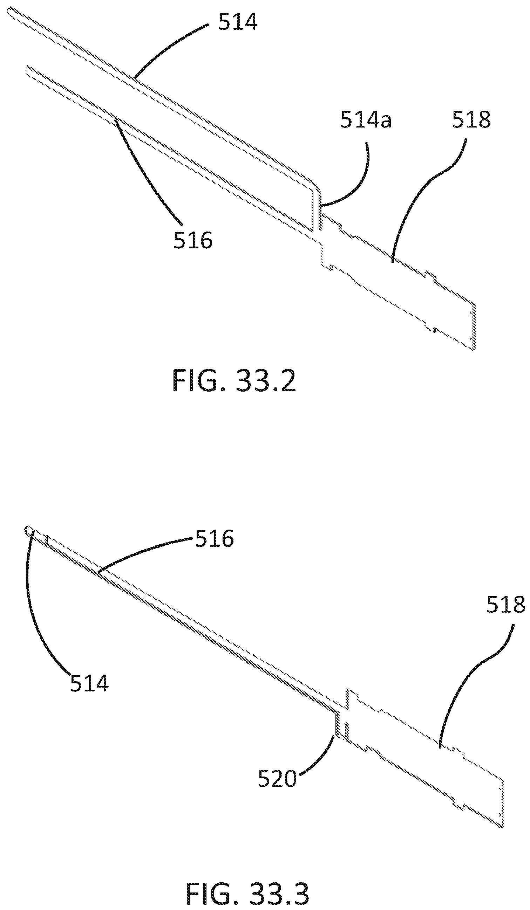

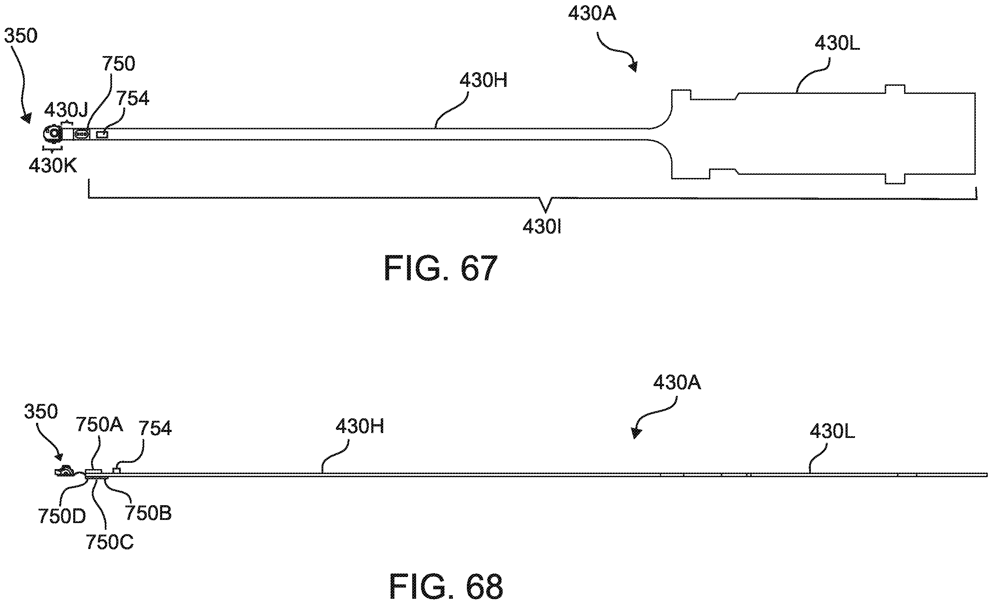

**FIG. 15** shows another example embodiment of one half (**30a**) of the handle distal section **30.** As shown, a pass-through barrier **159** and a pivot control structure **100** are assembled and placed within the shown half **(30a**) of the handle distal section **30.** An enclosure **431** of a printed circuit board which has been encased in a protective material **752** is also shown in place within part **30a** of the handle distal section **30.** In some embodiments, a projecting portion **430h** may be a ribbon or flex cable **250** (see, e.g., **FIG. 14**). The printed circuit board may communicate with components at the distal end of the shaft 14 through one or more ribbon cables that pass through the bulkhead or pass-through barrier 159. These cables may or may not need to slide somewhat back and forth through the bulkhead, to accommodate rotational movement of the sensor or camera housing at the distal end of the endoscope shaft, or to accommodate flexion and extension of the shaft 14 if it is a flexible shaft. Alternatively, the PCB may include an extension **430h** of the PCB itself which extends into the shaft or insertion section **14** through the pass-through barrier **159.** In some specific embodiments the printed circuit board and its extension **430h** may be similar to that shown and described in relation to FIG 33.2 and 33.3, or **FIG. 67****.** A sealing agent **151** is shown in place around the projecting portion **430h.** The pass-though barrier **159** may include a peripheral gasket **163** (see, e.g. **FIG. 11D**) in some embodiments. The pass-though barrier **159** may be coupled to the handle distal section **30** in any number of ways, including but not limited to, adhesive, epoxy, glue, solvent bonding, press fit, etc.

The pivot control structure **100** of FIG. 15 is similar to that shown in **FIG. 12** and **FIG. 14****.** However, the pivot control structure **100** may include arms **97** which interface with ridges **94** described above in relation to **FIG. 7C****.** Additionally, in this particular embodiment, the pivot arm **198** of the pivot control structure **100** does not include pull-wire attachment holes **202** (see, e.g. **FIG. 14**). Instead, fasteners **203** or a similar structure including eyelets **201** may be attached to or provided as part of the pivot arm **198.** Pull-wires may be attached to the pivot arm through the eyelets **201** and the pivot control structure **100** may be used to actuate the pull-wires, e.g. to bend a flexible shaft or insertion section **14** or to rotate a camera assembly in an insertion section **14.** The pull-wires may pass through the flexible member **153** of the pass-though barrier **159** to actuate components in the insertion section **14.**

As shown in **FIG. 15****,** the pass-through barrier **159** may be the only barrier separating the insertion section **14** from electronic components housed in the handle distal section **30** and proximal section 16. A sealing member **210** (see, e.g. **FIG. 14**) may not be included. In some embodiments, an electronics section **80** may not be partitioned from or fluidically isolated from the rest of the handle **12.** As mentioned above in relation to **FIG. 11C****,** a pass-through barrier **159** may include a peripheral gasket member **163** in some embodiments to provide an additional seal.

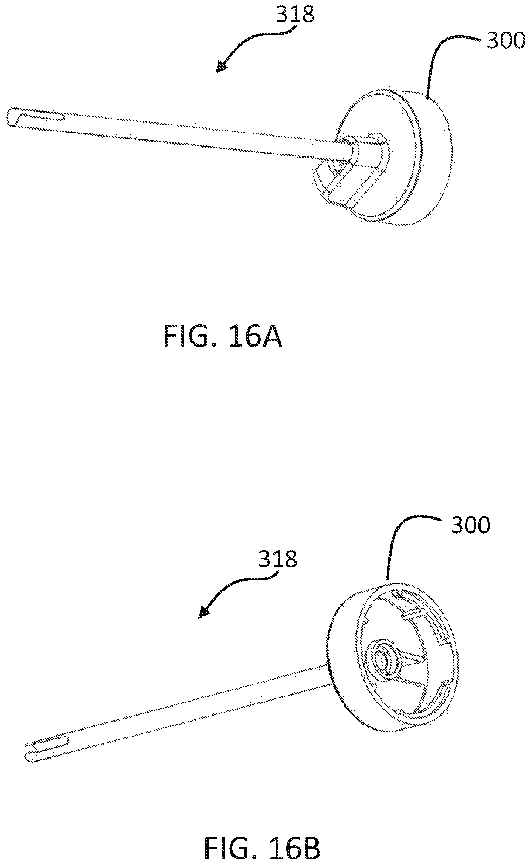

**FIG. 16** shows an example embodiment of an outer sheath or cannula mount **300.** As shown in FIG. 16A and FIG. 16B, an outer sheath or cannula **318** may be employed to provide additional protection to components in the distal end of the insertion section, or to allow a user to withdraw the insertion section of the endoscope while leaving the cannula **318** in situ, to allow later re-insertion of an insertion section of the endoscope. As shown, the cannula mount **300** may have a frustoconical shape, with the larger diameter section proximally forming a connector (e.g. bayonet mount) for the mounting of a cannula **318** over an inner sheath **312** (see, for example, **FIG. 17**). A cannula mount hole **302** may extend through the cannula mount **300** to merge with a cannula channel. The cannula or outer sheath mount hole **302** may be configured to accept and retain a cannula **318.** The cannula **318** may be configured to act as a sleeve over an inner sheath **312** of the insertion section.

As shown, the female bayonet mount portion **304** includes two slots **306.** The slots **306** optionally may have different dimensions to ensure proper orientation of the cannula **318** with respect to a mating (male) connector on a distal portion of the handle distal section **30.** In some embodiments, the slots **306** of the female bayonet mount portion **304** may include a serif into which the male bayonet mount portion **308** may be spring loaded using, for example, a Belleville washer. In such embodiments, a spring-loaded connection may help ensure the two pieces (cannula **318** and handle distal section **30**) are more securely locked together.

In some embodiments, an alignment feature may be included on the cannula mount **300** in order to properly orient the cannula **318** with the cannula mount **300** during assembly, and ultimately with the inner sheath **312** (see, for example, **FIG. 17**) when installed over the inner sheath **312** of the insertion section. In the example embodiment in **FIG. 16****,** an outer sheath mount tab **310** may project from the inner wall of the outer sheath mount hole **302.** The outer sheath mount tab **310** may extend from a distal face of the female bayonet mount portion **304,** which may then be used to align the bayonet mount **300** with a cannula **318** having a mating slot during assembly. Alternatively, the need for such a feature may be removed by coupling the outer sheath or cannula **318** and cannula mount **300** in a suitable fixture.

**FIG. 17** shows a partial cutaway view of an example embodiment of the distal face of the handle distal section **30.** An inner sheath **312** is mounted on the sheath mounting hub **252** of the inner sheath mount **160.** The inner sheath **312** includes a sheath mount notch **314.** The inner sheath mount notch **314** may be dimensioned to accept the sheath mounting tab **254** on the sheath mounting hub **252.** In such embodiments, the sheath mounting tab **254** and inner sheath mount notch **314** may ensure that the inner sheath **312** is correctly oriented on the endoscope **10.**

The inner sheath **312** (and/or the outer sheath or cannula **318,** see **FIG. 16**) may be formed from steel, any of a number of hardened plastics or other rigid, durable material. Alternatively, the inner sheath **312** or a portion thereof may be flexible, allowing the insertion section of the endoscope to bend as needed for insertion into a non-line-of-site target area. In these embodiments, a user may forgo the use of an outer sheath or cannula **318,** or the cannula **318** itself may also be constructed of a similarly flexible material.

The male bayonet mount portion **308** is also visible in the example embodiment shown in **FIG. 17****.** The male bayonet mount potion **308** may include two prongs **316.** The prongs **316** may be sized to fit in the legs of the L-shape slots **306** of the female bayonet mount portion **304** referring now also to **FIG. 16****.** The outer sheath **318** and cannula mount **300** may be coupled to the handle distal section **30** by aligning the prongs **316** with the slots **306,** pressing the bayonet mount over prongs **316,** and then turning the bayonet mount to lock it into position. As shown, optionally the two prongs **316** are dimensioned differently such that the outer sheath mount **300** may only have one possible orientation when coupled onto the handle distal section **30.**

Still referring now to both **FIGS. 16-17****,** an outer sheath or cannula **318** may be slid over the inner sheath **312,** forming a sleeve. The inner diameter of the outer sheath **318** may be only slightly larger than the outer diameter of the inner sheath **312** to ensure a snug fit. The outer sheath **318** may include an outer sheath notch **320.** The outer sheath notch **320** may be dimensioned to accept the outer sheath mount tab **310** when the endoscope **10** is fully assembled. In some embodiments, the outer sheath **318** may be friction fit, glued or otherwise fused or attached to the wall surrounding the outer sheath mount hole **302.** The outer sheath mount tab **310** may help to ensure correct orientation of the outer sheath **318** when the endoscope **10** is fully assembled.

When the shaft or insertion section **14** (see **FIG. 3**) of the endoscope **10** is inserted into a target region, the outer sheath **318** and outer sheath mount **300** may be uncoupled from the rest of the endoscope **10** as mentioned above. This may allow the outer sheath **318** to be used as a cannula, remaining in situ to permit the endoscope **10** to be reintroduced into the target region.

Shown in FIG. 17A is a trocar or obturator 319 adapted for use with the outer sheath 318. During introduction of the endoscope into the surgical field, the trocar may be inserted into an outer sheath 318 to facilitate entry of the outer sheath 318 into the desired location, after which the trocar can be withdrawn and the inner sheath 312 of the shaft 14 can be inserted. If the shaft 14 needs to be substantially repositioned within the surgical field during an operation, the endoscope shaft 14 can be withdrawn from the patient while keeping the outer sheath 318 in place, the trocar can be introduced into the outer sheath 318, and the trocar/outer sheath assembly can be repositioned as needed. Once in the proper location, the trocar can be withdrawn from the outer sheath 318, and the endoscope shaft with inner sheath 312 can then be re-inserted into the outer sheath 318. In the example shown, the trocar 319 includes a solid shaft portion 321 with pointed or blunted end 323, and a base portion 325. The base portion 325 is optionally equipped with a locking mount that matches that of the distal handle section 30 of the endoscope handle, so that the trocar can be secured to the outer sheath 318 when in use. If desired, the outer sheath or cannula **318** may be used as a conduit through which other instruments may be introduced into the target region. The outer sheath **318** may also function as a conduit through which fluid may be introduced or withdrawn from the target region.

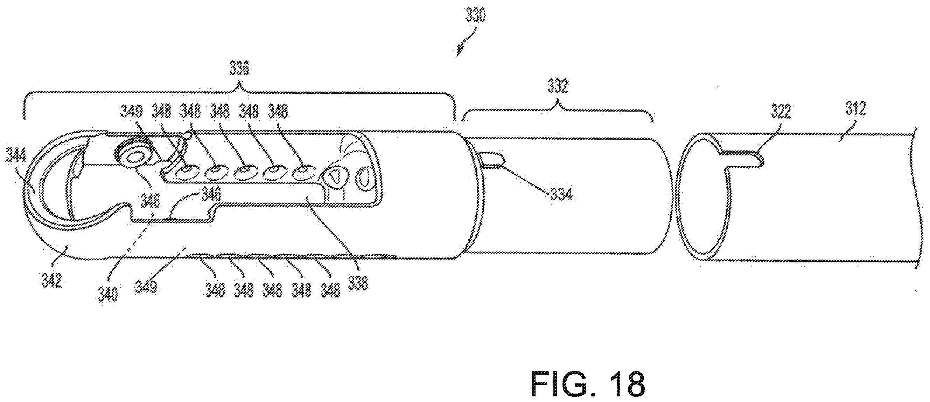

A camera assembly housing **330** or distal working section is shown in **FIG. 18****,** separated from a distal end of an inner sheath **312.** In this embodiment, the distal working section of an insertion section of an endoscope may be constructed separately from the inner sheath **312,** and subsequently mated to a distal end of the inner sheath **312** during assembly. In other embodiments the inner sheath **312** may be constructed as a single piece, incorporating a distal working section. In embodiments where the distal working section is constructed separately, the distal working section may be made from a material different from that of the inner sheath **312.** Additionally, it may be constructed from a number of assembled parts.

In the example embodiment in **FIG. 18****,** the distal edge of the inner sheath **312** includes an inner sheath distal notch **322.** The camera assembly housing **330** may include a nested segment **332,** shaped and having an outer diameter suitable for insertion into the distal end of inner sheath **312** during assembly of the endoscope **10.** The nested segment **332** may include a nested segment tab **334** or other alignment feature. The nested segment tab **334** may be dimensioned so that it may be mated to the inner sheath distal notch **322** when the endoscope **10** is assembled. The nested segment tab **334** and inner sheath distal notch **322** may help ensure that the camera assembly housing **330** is properly oriented and aligned when the endoscope **10** is assembled.

The camera assembly housing **330** may additionally include a working segment **336.** As shown, the working segment **336** in **FIG. 18** may include a top void **338** with or without a bottom void **340.** The top void **338** and bottom void **340** may extend along most of the working segment **336** of the camera assembly mount **330.** A rounded tip **342** may be included at the distal end of the working segment **336** of the camera assembly mount **330.** As shown, the rounded tip **342** may optionally include an embrasured opening **344.** The edges of the embrasured opening **344** may be beveled, chamfered or rounded. In the example embodiment, the embrasured opening **344** is continuous with the top void **338.** In some embodiments, the top void **338** and bottom void **340** may be similarly embrasured.

A rounded tip **342,** such as the rounded tip **342** shown in **FIG. 18** may provide a number of benefits. A rounded tip **342** may facilitate the insertion of the insertion section 14 into a target region of a patient. In some cases, this may eliminate the need for a trocar. In arthroscopic applications, the contours of the rounded tip **342** allow the endoscope **10** to be maneuvered into tight spaces within a joint. A rounded tip **342** additionally may allow a surgeon to exert pressure atraumatically on tissues within a target region. The rounded tip **342** may also serve as a guard feature for a camera assembly **350.**

As shown in **FIG. 18****,** the interior walls of the working segment **336** of the camera assembly housing **330** include two camera mount pivot bearings **346.** In the example embodiment shown in **FIG. 18****,** the camera pivot bearings **346** project substantially perpendicularly from the inner side walls of the camera assembly mount **330.** The camera assembly housing **330** may be made of steel, any number of hardened plastics, or any other suitably strong, rigid material.

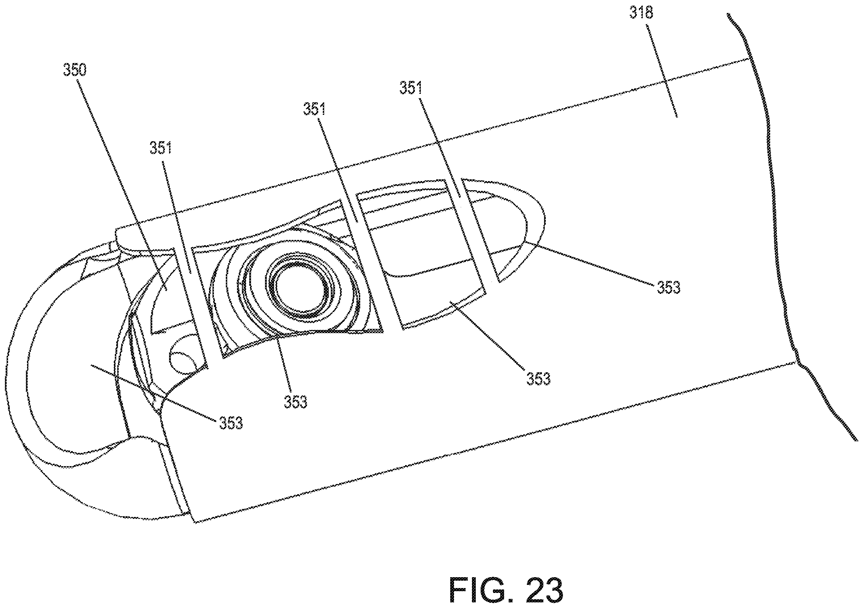

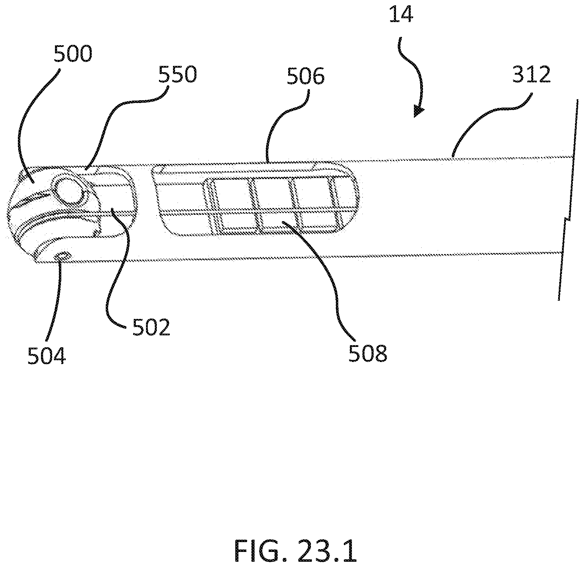

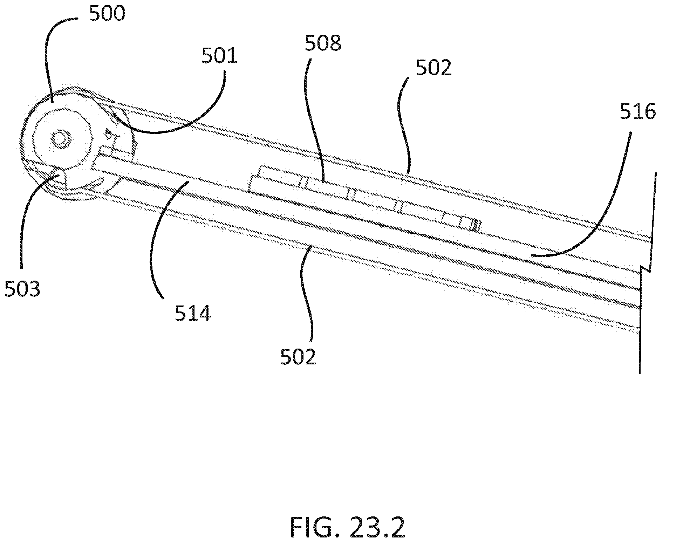

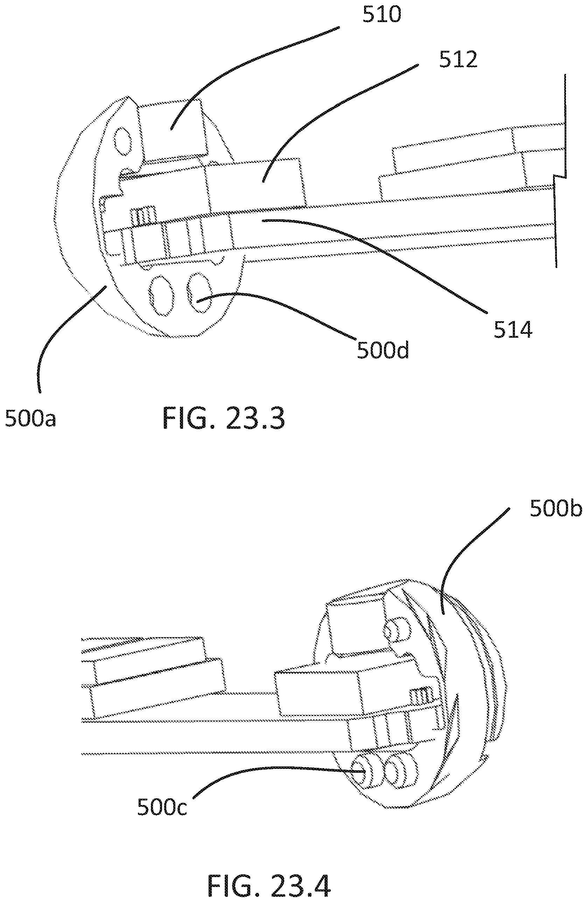

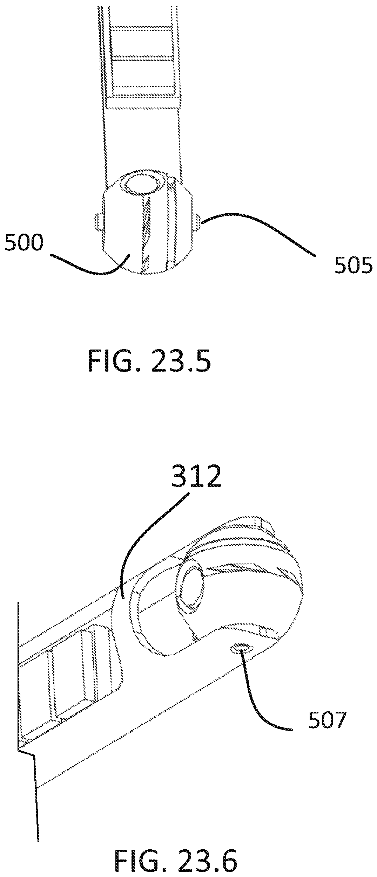

In the example embodiment shown in **FIG. 18****,** the interior walls of the working segment **336** of the camera assembly housing **330** include a number of cable guide holes **348.** In a preferred embodiment, there may only be two cable guide holes **348.** One cable guide hole **348** may be located on one side wall while another cable guide hole **348** may be located on an opposing side wall. Preferably, the cable guide holes **348** may be disposed below the camera mount pivot bearings **346,** so that the distal end of a control cable may form an angle with respect to a camera, camera mount, or camera assembly **350** (see, for example, **FIG. 23**) to which it is connected. The camera assembly housing **330** may also include one or a number of constraining features. In the example embodiment shown in **FIG. 16****,** there are two restraining notches **349.** One restraining notch **349** is located on one side wall and the other restraining notch **349** is located on an opposing side wall. As shown in **FIG. 16****,** the restraining notches **349** are roughly in line with the cable guide holes **348.** The cable guide holes **348** and restraining notches **349** will be described further below.

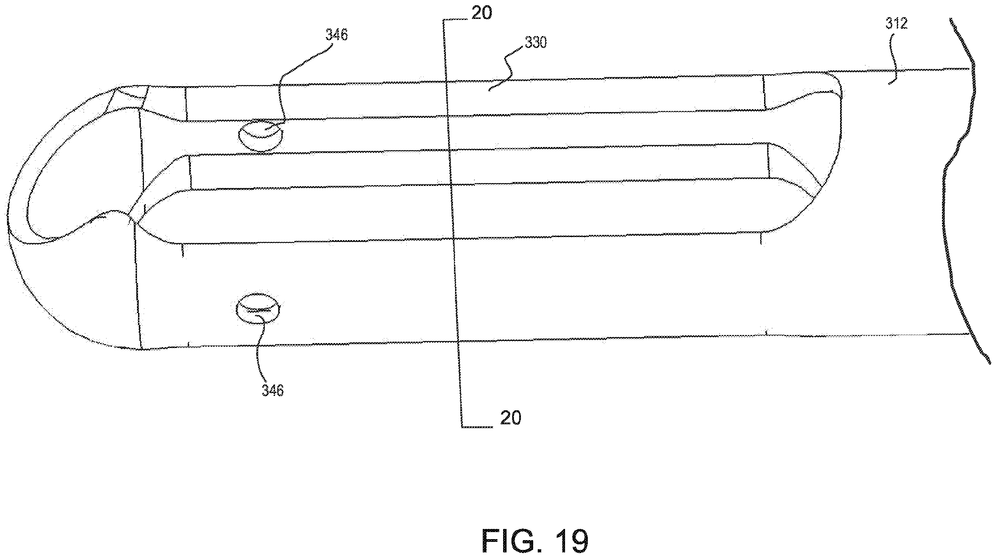

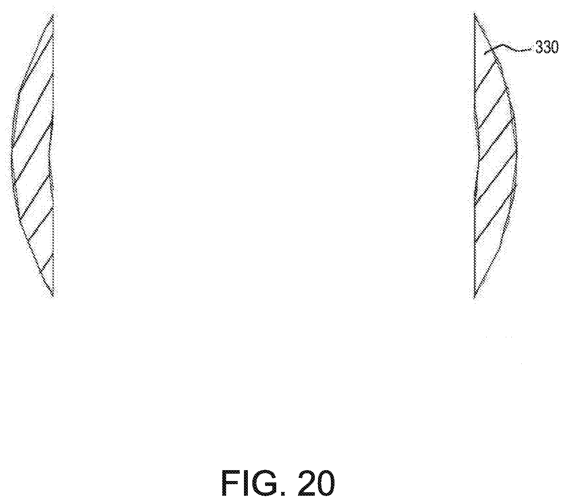

**FIG. 19** depicts an embodiment of a distal working section or camera assembly housing **330** and inner sheath **312** which are constructed as a single part. Referring also to **FIG. 20****,** a cross section taken at line **20-20** of the camera assembly housing **330** in **FIG. 19** is shown. In embodiments where the distal working section or camera assembly housing **330** and the inner sheath **312** are constructed as a single part, they may be made from steel. In such instances the tip shape of the inner sheath **312** and camera assembly housing **330** may be created via a rolling process. Various voids, openings, and other features, for example those described above, may then be post machined into the part. In the example embodiment in **FIG. 19****,** the camera assembly housing **330** includes only the camera mount pivot bearings **346.**

It may be advantageous to create the inner sheath **312** and the camera assembly housing **330** as a single part. Among the advantages, the part may be stronger. Another advantage is that the need for a nested portion is removed. Consequently, a "choke point" in cross-sectional area at the junction of the inner sheath **312** and camera assembly housing **330** is removed. This may provide a number of benefits. Removing such a choke point allows more room for various components, such as utility components within the inner sheath **312** and camera assembly housing **330.** Moreover, removal of such a choke point allows for increased flow of irrigation fluid within the inner sheath **312** and camera assembly housing **330.** Alternatively or additionally, the overall diameter of the inner sheath **312** and camera assembly housing **330** may be decreased. The inner sheath **312** and camera assembly housing **330** may also be thickened. This helps to strengthen the part. Since thickening will strengthen the part, it may also allow an outer sheath or cannula **318** to be made thinner. A thinner outer sheath or cannula **318** in turn may allow for a larger diameter inner sheath **312** and camera assembly housing **330.** That is, without increasing the overall diameter of an insertion section **14** (comprised of an outer sheath **318,** inner sheath **312** and camera assembly housing **330**), the cross-sectional area of a conduit within the insertion section **14** may be made larger. Thickening furthermore enables the camera mount pivot bearings **346** to have a larger bearing surface allowing pressure exerted against the bearing to be spread over a larger area.

**FIG. 21** shows an assembled view of the tip of the insertion section **14** (best shown in **FIG. 3A**). The camera assembly housing **330,** camera assembly **350,** and outer sheath or cannula **318** are visible in **FIG. 21****.** As shown, the rounded tip **342** of the camera assembly housing **330** projects past the distal end of the outer sheath or cannula **318.** A viewing notch **352** is recessed into the top of the outer sheath **318.** The camera assembly **350** may be pannable throughout the viewable range as defined by the opening created by the combination of the embrasured opening **344** and the viewing notch **352.** In some embodiments the pannable range may be approximately 180°. When panning, the camera assembly **350** may pivot on the camera pivot bearings **346** (see, for example, **FIG. 18**). Panning actuation will be described further below.

In some embodiments, the outer sheath **318** may be rotated to an insertion position (not shown) when the insertion section **14** (see **FIG.** 3) of the endoscope **10** is being inserted into the target region. In the insertion position, the viewing notch **352** may not be aligned with the embrasured opening **344** and top void **338.** This may help protect the camera assembly **350** during insertion, and in medical applications may reduce the risk of damage to tissue upon insertion of the insertion section **14.** After insertion, the outer sheath **318** may be rotated back to a position in which the viewing notch **352** is aligned with the embrasured opening **344** and top void **338** so that the full viewable range is again available.

In some embodiments, a cap or window material may cover or be placed in the openings defining the viewing notch **352** and embrasured opening **344** to protect the camera assembly **350.** In some embodiments, the distal edge of the outer sheath **318** and the viewing notch **352** may be embrasured, rounded, beveled, etc. to help prevent damage that might result from having sharp edges.

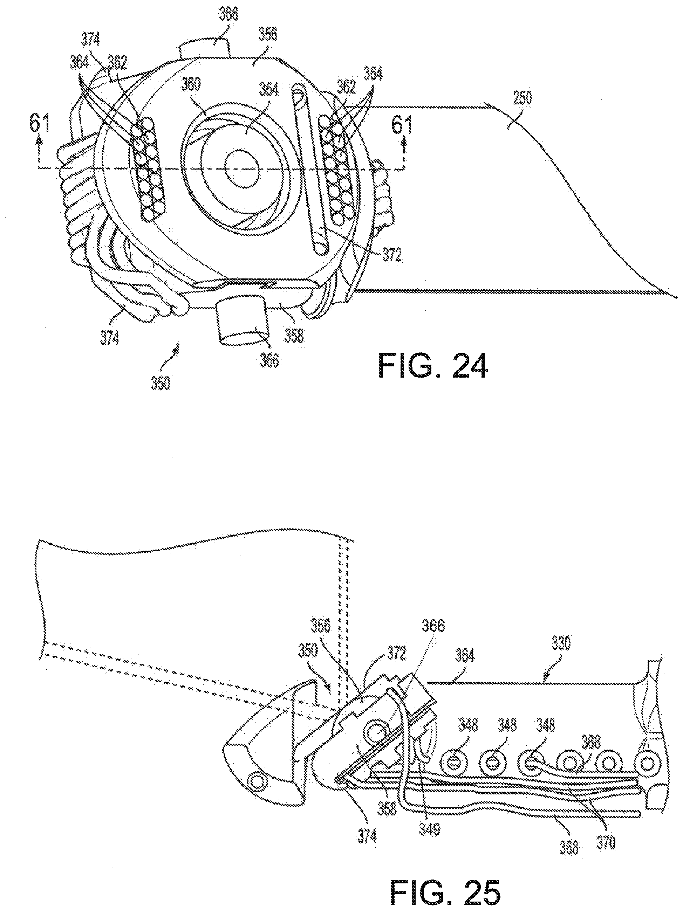

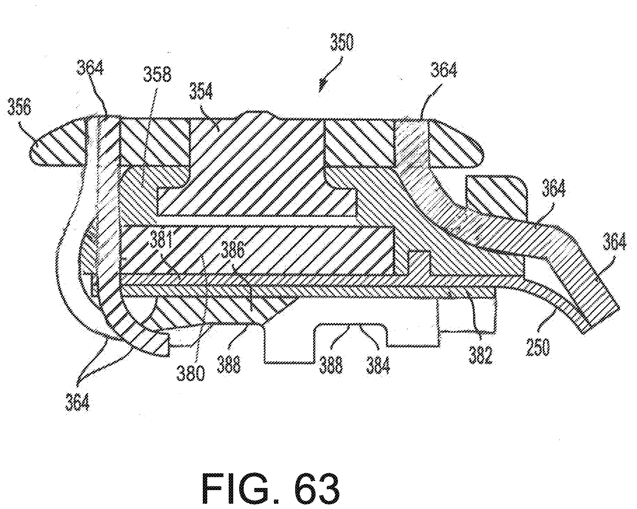

In the example embodiment, a cap or window is not used. Such an arrangement provides a number of benefits. For example, by not using a cap or window at the tip of the insertion section **14,** the cost of the endoscope may be reduced because no expensive scratch and wear resistant materials such as sapphire, specialized glass, etc. are used. Not having a cap or window may also eliminate any undesirable reflections from the surface of the cap or window, which could otherwise affect the clarity of any image captured by a camera. Moreover, by not using a cap or window, irrigation of the target area may be conducted through the conduit of the inner sheath **312** (see **FIG. 15**) of the endoscope **10.** This enables the total diameter of the insertion section **14** to be kept small while retaining irrigation capabilities. Furthermore, irrigation flow within the inner sheath **312** may help to clear/clean any debris or material away from the camera assembly **350** and any associated lens or lenses. In one example, a user may be able to effectively irrigate the camera assembly **350** by panning the camera assembly **350** during irrigation so that the irrigation flow washes over a lens assembly **354** (see, for example, **FIG. 24**) of the camera assembly **350** and carries away the debris or unwanted material. As an added benefit, the irrigation flow may also help to cool an image sensor **380** (see, for example, **FIG. 63**) associated with the camera assembly **350.**

As shown, the embrasured opening **344** and viewing notch **352** may be dimensioned in order to protect the camera assembly **350** without the need for a cap or window. In the example embodiment in **FIG. 21****,** the embrasured opening **344** and viewing notch **352** partially envelop the camera assembly **350,** which is recessed from the outer surfaces formed by the embrasured opening **344** and viewing notch **352.** Thus the embrasured opening **344** and viewing notch **352** define the edges of a guard for the camera assembly **350.** The partial envelopment helps to protect movable components of the camera assembly **350** and any associated components (e.g. control, electric, information cables, etc.) from contact with external objects either during insertion of the insertion section into the target region, or during use of the instrument once in the target region. The embrasured opening **344** and viewing notch **352** provide the camera assembly **350** an unrestricted view while exposing only a small part of the camera assembly **350** to possible damage from objects external to the insertion section (such as, e.g., a medical instrument such as a shaver). This helps to ensure that the camera assembly **350** is not damaged during insertion or during a procedure.

As the camera assembly **350** rotates, the distance between the camera assembly **350** and the outer sheath **318** will change. As a consequence, the amount of the outer sheath **318** which falls into the field view of the camera assembly **350** will also change. The greater the distance from the camera assembly **350** to the inner sheath **318,** the greater the amount of the outer sheath **318** which will be in the field of view of the camera assembly **350.** Thus, an optimized amount of protection while still affording the camera assembly **350** and unrestricted view may be achieved by varying the width of a viewing notch **352.**

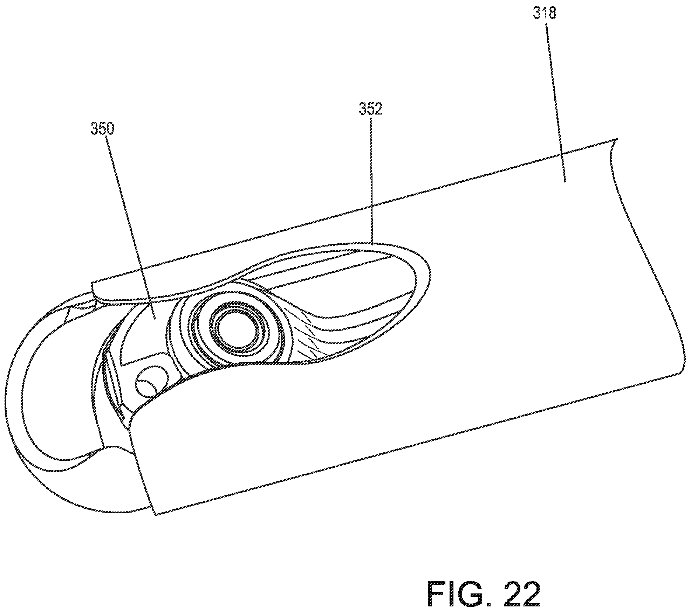

**FIG. 22** depicts an alternate assembled view of the tip of an insertion section **14** (best shown in **FIG. 3A**) in which the viewing notch **352** has a varying width. The width of the viewing notch **352** varies such that the viewing notch **352** is just outside of the field of view of the camera assembly **350** in any angular orientation of the camera assembly **350.** This allows for a greater degree of envelopment of a camera assembly **350** by an outer sheath **318.**

**FIG. 23** depicts another alternate embodiment of a tip of an insertion section **14** (best shown in **FIG. 3A**) in which a number of openings **353** separated by bars **351** are included in place of a viewing notch **352** like that shown in **FIG. 20****.** Such an arrangement may provide additional protection to a camera assembly **350.** To minimize the amount that the bars **351** obscure the field of view of the camera assembly **350,** the bars **351** may be made of a transparent material. In other embodiments the bars **351** may be made of an opaque material, for example, the same material as the outer sheath **318.**

Alternatively a cover member (not shown) which partially covers a viewing notch **352** (see **FIG. 22**) or one or more openings **353** (see **FIG. 23**) may be mounted to the distal tip of a shaft or an insertion section **14** (see, for example, **FIG. 1**) Such a cover member may for example be a cage which allows a substantially clear field of view for the camera assembly **350** while providing additional protection for the camera assembly **350.** In some embodiments, the cover member may include an optically clear partial covering.

In another embodiment, the camera assembly may be mounted at the distal end of an endoscope shaft without a protective tip structure 342. Although a tip structure 342 may provide some protection to a camera assembly, it may also inhibit a full field of view of the camera in all positions within its range of motion. An example of an alternative arrangement is shown in **FIG. 23****.****1****.** In this example, the sensor or camera housing 500 itself is constructed to provide adequate protection to an enclosed camera assembly (e.g., lens and sensor assembly). For example, the camera housing 500 may be at least partially constructed of steel or similarly strong material; at least an outer shell of the housing can be so constructed to withstand physical abuse when the insertion end of the endoscope is introduced or repositioned. The exposed portion of the housing 500 preferably has an outer spherical, spheroid (oblate, prolate, etc..) or dome shape - or an otherwise rounded shape providing rounded edges to help prevent damage to tissues as the endoscope shaft is inserted or moved within the operative field. Placing the camera assembly in a reinforced and at least partially rounded housing 500 at the distal end or tip 550 of the endoscope shaft 14 provides an unobstructed view of a greater portion of the surgical field, without placing the camera assembly or nearby tissues at risk of damage. In this example, the sensor or camera housing 500 can be rotated using pull wires 502 , cables or bands about an axis 504 so that an optical axis of the camera assembly (lens and sensor assembly) can be aimed from less than zero degrees to more than ninety degrees with respect to the long axis of the distal end 550 of the endoscope shaft 14. If the sensor or camera assembly is arranged to have a wide field of view, then the range of motion of the camera housing can be arranged to provide an optical axis range of motion of between about 35 degrees and about 115 degrees with respect to the long axis of the endoscope shaft at its distal or insertion end. In this arrangement, the operator can still view the operative field directly opposite the distal end of the endoscope shaft, yet be able to view a region of the surgical field behind the tip of the endoscope. This arrangement may also allow the operator to irrigate the surface of the camera assembly to remove any accumulated surface debris by rotating it to a position equal to or greater than 90 degrees.

A camera assembly **350** is shown in isolation in **FIG. 24****.** This arrangement is more suited to the insertion section or shaft shown in Fig. 23, because of the physical protection offered by the rounded tip 342 of the working end of the distal endoscope shaft shown in Figs. 18 - 23. As shown, a ribbon or flex cable **250** is coupled into the camera assembly **350** and may provide power and data communication paths to and from the camera assembly **350.** The camera assembly **350** may be any suitable structure configured to support the camera of the endoscope **10.** In embodiments where the camera assembly **350** may be panned, the camera assembly **350** may include pivot actuator attachment features.

As shown, the camera assembly **350** may include a lens assembly **354.** As shown, the lens assembly **354** may be held in place between a camera housing top **356** and a camera housing bottom **358.** When assembled, the camera housing top **356** and camera housing bottom **358** may be coupled together by any suitable means, such as, but not limited to glue, adhesive, ultrasonic welds, press fit of cooperating features, etc. In the example embodiment in **FIG. 24****,** the lens assembly **354** projects through a lens opening **360** in the camera housing top **356** such that it may have a clear view of the target anatomical area. In some embodiments, at least a portion of the lens assembly **354** may be proud of the camera housing top **356.**

The camera housing top **356** may include a number of other voids. In the exemplary embodiment shown in **FIG. 24****,** the camera housing top **356** includes two elongate light projection voids **362** disposed on the right and left (relative to **FIG. 24**) flanks of the lens opening **360,** the voids **362** being designed to accommodate terminal elements of optical fibers (or optionally other light sources such as LEDs) to project light onto a target area coinciding with the direction at which a camera lens or lens assembly **354** may be aimed. In the example shown, the right elongate void **362** is trapezoidal in shape while the left elongate void **362** is rhomboid in shape. In alternative embodiments, the shape of the voids **362** may differ, for example, both may be ovoid. In alternative embodiments, there may be additional voids **362.** For example, in some embodiments, there may be three voids **362** arranged in a triangular configuration around the lens opening **360.** In some embodiments there may be four voids **362** arranged in a rectangular, square, circular, or ovoid configuration around the lens opening **360.**

One or more illumination sources for the endoscope **10** may be included at least partially within the endoscope **10.** The illumination source or sources may illuminate the field of view of the camera of the camera assembly **350** regardless of its panned position. In some embodiments, the illumination source may be in the camera assembly **350.** In the example embodiment in **FIG. 24****,** the illumination source is a number of optical fibers (e.g. fiberoptic fibers) **364** which may transmit light from a lighting element (not shown) external to the endoscope **10.** The optical fibers **364** may be routed and coupled into the voids **362** in the camera housing top **356.** In the example embodiment, 28 optical fibers **364** are routed into the voids **362** of the camera housing top **356.** The number of optical fibers **364** may differ in alternate embodiments. The light emitting ends of the optical fibers **364** may be roughly flush with the top face of the camera housing top **356.** In some embodiments, other illumination sources, for example LEDs, may be used. The optical fibers **364** or other illumination source may be configured to supply any desired color or intensity of light at a pre-determined light projection angle.

As shown in the example embodiment in **FIG. 24****,** the camera assembly **350** may include pivot pins **366.** The pivot pins **366** may be pivotally coupled into the pivot pin bearings **346** in the camera assembly housing **330** (see **FIG. 18**). The pivot pins **366** may project substantially perpendicularly from the long axis of the insertion section. The pivot pins **366** may allow the camera assembly **350** and optical fibers **364** (or other illumination source) to pivot in tandem with one another.

The camera assembly **350** may also include a pivot actuator attachment feature as mentioned above. In the example embodiment in **FIG. 24****,** the camera assembly **350** includes a top cable attachment feature or anchor point **372** and a bottom cable attachment feature or anchor point **374.** The top cable attachment feature **372** and bottom cable attachment feature **374** will be further discussed below.

As mentioned above, the endoscope **10** may also include a pivot actuator or actuators. A pivot actuator may be an elongate member used to pull on or push the camera assembly **350** via a pivot attachment feature. In the illustrated examples, the pivot actuators are mostly pull cables or wires, but these examples should not be construed as strictly limiting pivot actuators to a cable-like structure. The elongate member may be flexible or substantially rigid. The elongate member may be round (as in the example of a cable), flat, or may have any other shape or cross section. In some embodiments, the pivot actuator may be a belt routed around a cooperating attachment feature frictional engaged or otherwise meshed with features on the inner circumference of the belt. In a preferred embodiment, the pivot actuator may be used to only supply a pulling force. Such an arrangement allows for a smaller diameter insertion section **14** (see **FIG. 3A**) because the pivot actuator does not have to be sufficiently thick or cross-sectionally strengthened, or confined within a supporting track to prevent substantial lateral displacement within the insertion section **14** in response to a pushing force against the pivot actuator. A pull-wire or pull-cable arrangement also allows a greater range of materials to be used in constructing the pivot actuator because the material only needs to have tensile strength, rather than compressive stiffness.

As shown in **FIG. 25****,** panning cables may be attached to the camera assembly **350** above and below the pivot pins **366.** In the example embodiment, the panning cables are shown as relatively slack for ease of illustration. In operation one or more panning cables on one side of the pivot pins **366** would be under tension, while one or more panning cables on the other side of the pivot pins **366** would be slack. As detailed above and referring now also to **FIG. 14****,** the panning cables may be attached proximally to the cable attachment holes **202** of the pivot control structure **100** (see **FIG. 14**). In some embodiments, two panning cables may be attached to each cable attachment hole **202.** The panning cables may extend from the cable attachment holes **202** in the pivot arm **198** and be routed through one or more orifices **178** in the proximal section **161b** of the inner sheath mount **160** (see **FIG. 11A**). The panning cables may then extend through the utility hole **168** alongside the flex cable **250.** Since the cable attachment holes **202** are located on opposite sides of the pivot point of the pivot arm **198,** pivoting the pivot control structure **100** may cause the panning cables attached to one of the cable attachment holes **202** to slacken and panning cables attached to the other to become taut. By attaching the panning cables associated with one cable attachment hole **202** to the camera assembly **350** on one side of the pivot pins **366** and attaching the panning cables associated with the other cable attachment hole **202** to the opposite side of the pivot pins **366,** the pivot control structure **100** may be used to selectively rotate the camera assembly **350.** In some embodiments, pushing the pivot control structure **100** forward may pan the camera assembly **350** forward while pulling the pivot control structure **100** aft may pan the camera assembly **350** backward. In some embodiments, when assembled, all of the panning cables may be under tension.

In a preferred embodiment, only a single panning cable may be attached to each cable attachment hole **202** on the pivot control structure **100** pivot arm **198** (see **FIG. 14**). In such embodiments, there may be a top panning cable **368** and a bottom panning cable **370.** The top panning cable **368** and bottom panning cable **370** may extend as described above to the camera assembly **350.** The top panning cable **368** may wrap around a top cable attachment feature **372** on the camera assembly **350** and return back to the same cable attachment hole **202** on the pivot arm **198** from which it originates. The bottom panning cable **370** may wrap around a bottom cable attachment feature **374** on the camera assembly **350** and return back to the same cable attachment hole **202** from which it originates. Alternatively, the panning cable may be looped through attachment hole **202,** with both ends of the cable terminating on the cable attachment feature distally.

In the example embodiment, the top cable attachment feature **372** (best shown in **FIG. 24**) includes two holes in the camera housing top **356.** The top cable attachment feature **372** additionally includes a recess that connects the two holes. The top panning cable **368** may enter one of the holes, follow the recess, and exit the other of the two holes to return to the cable attachment hole **202** (see **FIG. 14**) in the handle. The bottom cable attachment feature **374** (best shown in **FIG. 24**) includes two attachment points or hooks which project off opposite sides of the camera housing bottom **358.** The bottom cable attachment feature **374** is on the opposite side of the pivot pins **366** than the top cable attachment feature **372.** The bottom panning cable **370** may be wrapped around one attachment point or hook of the bottom cable attachment feature **374,** strung over to the second attachment point or hook of the bottom cable attachment feature **374** and from there return to its cable attachment hole **202** on the pivot arm **198** of the handle. In alternate embodiments, the top cable attachment feature **372** and/or bottom cable attachment feature **374** may comprise, for example, eyelets, prongs, pegs, etc.

The top panning cable **368** and bottom panning cable **370** may be made from any suitable cable or wire-like material, either metallic or synthetic polymer, either braided or monofilament. The top panning cable **368** and bottom panning cable **370** may, for example, be metal or plastic strips or bands that are laterally flexible. In a preferred embodiment, the top panning cable **368** and bottom panning cable **370** are made from a material which is resistant to stretching under tension. Wrapping a single panning cable from each cable attachment hole **202** on the pivot arm **198** (see **FIG. 14**) around a pivot actuator attachment feature on the camera assembly **350** may be desirable because it ensures that the side of the panning cable running to the camera assembly **350** is under the same tension as the side of the panning cable returning from the camera assembly **350;** any stretching of some portion of the cable over time or use will have an equal effect on both halves of the cable.

In a preferred embodiment, the top panning cable **368** may be run through one of the cable guide holes **348** on each interior wall of the camera assembly mount **330.** As shown in **FIG. 25****,** the top panning cable **368** is threaded through one of the cable guide holes **348** and continues extending toward the camera assembly **350** along the exterior of the camera assembly housing **330.** In some embodiments, there may be a depression or trough recessed into the exterior of the camera assembly housing **330** along the path taken by the top panning cable **368.** In such embodiments, the depression or trough may serve as a guide. The depression or trough may also help to ensure that the top panning cable **368** is roughly flush to exterior surface of the camera assembly housing **330.** This may help to ensure that the outer sheath **318** (see **FIG. 21**) does not impinge on the top panning cable **368** to impair its movement during the use of a fully assembled endoscope **10.**

As shown in **FIG. 25****,** the top panning cable **368** is strung through the constraining notch **349** as it re-enters the interior of the camera assembly housing **330.** The top panning cable **368** then runs to the top cable attachment feature **372** as describe above. On return to the cable attachment hole **202** (see **FIG. 14**), the top panning cable **368** runs from the top cable attachment feature **372** to the constraining notch **349** on the opposite wall (see **FIG. 18**) of the camera assembly housing **330.** The top panning cable **368** then runs along the exterior surface of the front wall of the camera assembly housing **330** and optionally along a depression or trough in the wall. The top panning cable **368** then re-enters the interior volume of the camera assembly housing **330** and travels back to the cable attachment hole **202** in the handle as described previously.

A terminal segment of a pivot actuator (such as a wire or cable) proximal to its connection to a pivoting assembly at the distal end of the insertion section may be constrained at a fulcrum or support point to re-direct the actuator so as to form an angle with respect to the long axis of the insertion section or shaft. For example, by running the top panning cable **368** through the cable guide holes **348** and the constraining or re-directing notches **349,** and then angling it up to the top cable attachment feature **372** on the other side of pivot pin **366,** an increased pivotal range for the pivoting camera assembly **350** may be achieved. Thus an image sensor having a pre-determined or fixed angular field of view may be rotated to allow for a rotatable field of view, so that the viewable area can be increased to a range of up to 180 degrees. In other embodiments, an image sensor may be rotated so as to achieve a viewable area that exceeds 180 degrees. As shown in **FIG. 25****,** having the cable routed as described places the cable at a more acute angle of incidence to its attachment point **372,** and thus permits a greater degree of back-rotation of the camera assembly **350.**

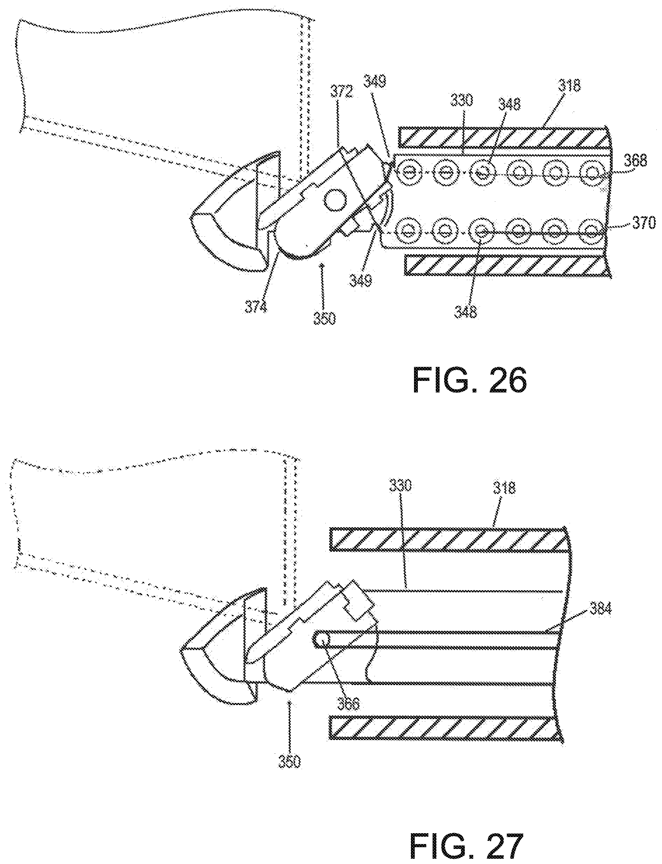

In some embodiments, and referring now also to **FIG. 26****,** the camera assembly **350** may be capable of rotating a full 180 degrees or more, because of the presence of two sets of cable guide holes **348:** a lower set of guide holes **348** to control the camera housing top section, and an upper set of guide holes **348** to control the camera housing bottom section. The degree to which the camera assembly **350** can be rotated is a function of the angle that the terminal portion of the panning cable makes with respect to the proximal portion of the panning cable or the longitudinal axis of insertion section (or endoscope shaft) **14** (see **FIG.1**). The greater the angle the terminal portion of the panning cable makes as it re-enters the exterior of the camera assembly housing **330** in relation to the longitudinal axis of the insertion section **14,** the greater the range of motion it can induce in the camera assembly **350.** In a preferred embodiment, the re-entry surface or re-directing guide of the camera assembly housing **330** is positioned to provide for an angle of the terminal portion of the panning cable to be within a range of about 30 - 90 degrees with respect to the long axis of insertion section **14.** In other embodiments, the rotational range of motion of the camera assembly **350** may be improved while limiting the frictional resistance of the panning cable by positioning the cable re-entry surface or guide to achieve an angle of the terminal portion of the panning cable to be within a range of about 45 - 80 degrees. Such an embodiment, as described above, only requires a pulling force on either of a pair of complementary cables **368, 370** one angled up at a distal or terminal location in insertion section **14** to attach to the top cable attachment feature **372,** and one angled down at a distal or terminal location in insertion section **14** to a corresponding bottom cable attachment feature **374.** With this arrangement, neither actuating cable is required to move laterally or transversely within most of the length of insertion section **14,** which allows the internal space within insertion section **14** to be narrower, helping to minimize its overall diameter.

In some embodiments, a constraining or re-directing notch **349** may not be used. Some embodiments may use a different type of constraint or re-directing element incorporated into a wall at the distal end of the insertion section. In some embodiments, a pulley or an eyelet may be used as a constraint. A pin, peg, post, etc. may also be used as a constraint or re-directing element. In some embodiments, a curved finger or prong may be formed in the side walls of the camera assembly housing **330.** The curved finger may extend into the interior volume of the camera assembly housing **330** such that there is a space between the interior wall of the camera assembly housing **330** and the curved finger. The top panning cable **368** may be run through this space so that it is constrained by the curved finger. In most embodiments, it may be desirable that the point of contact between the constraint and the cable has a smoothness or radius of curvature sufficient to minimize the potential for frictional damage to the panning cable during operation of the endoscope. In some cases, the constraint may be coated with a material having a low coefficient of friction such as Teflon.

In some embodiments, the bottom panning cable **370** instead of the top panning cable **368** may be constrained similarly to the preceding description to enable a greater pivotal range of the camera assembly **350** in one direction of rotation over another. As shown in **FIG. 26****,** in some embodiments, both the bottom panning cable **370** and top panning cable **368** may be constrained or redirected, allowing for even greater pivotal ranges.

In **FIG. 26****,** the outer sheath **318,** camera assembly housing **330,** and camera assembly **350** are shown. There are two sets of cable guide holes **348.** One set is above the longitudinal axis of the camera assembly housing **330** and the other is below the longitudinal axis of the camera assembly housing **330.** There are also two constraining notches **349.** One of the constraining notches **349** is located above the longitudinal axis of the camera assembly housing **330** and the other is located below the longitudinal axis of the camera assembly housing **330.**

An improved mechanical advantage of the panning cables may be obtained by positioning the re-directing element (e.g. notch) on one side of (e.g., below) the pivoting axis of the camera assembly **350,** while attaching the terminal end of the panning cable to a point on the camera assembly **350** located on the opposing side of (e.g. above) the pivoting axis of the camera assembly **350.**

As shown, the top panning cable **368** is run through one of the cable guide holes **348** below the longitudinal axis, and re-enters the camera assembly housing **330** at the constraining notch **349** below the longitudinal axis. The top panning cable **368** then redirects up to the top cable attachment feature **372** on the camera assembly **350.** In **FIG. 26****,** the bottom panning cable **370** is run through a cable guide hole **348** above the longitudinal axis of the camera assembly housing **330.** The bottom panning cable **370** then re-enters the camera assembly housing **330** through the constraining notch **349** above the longitudinal axis of the camera assembly housing **330.** The bottom panning cable **370** then redirects down to the bottom cable attachment feature **374.** The top panning cable **368** and bottom panning cable **370** may wrap around a portion of the camera assembly **350** depending on where the camera assembly **350** has been pivoted to. In **FIG. 26** the bottom panning cable **370** is shown wrapping around a portion of the camera assembly **350.**

Some embodiments may make use of a belt **384** as a pivot actuator. An embodiment which includes a belt **384** as a pivot actuator is shown in **FIG. 27****.** As shown, the belt **384** wraps around one of the pivot pins **366** of the camera assembly **350.** In some embodiments, the pivot pins **366** may be elongated such that a portion of at least one of the pivot pins **366** extends from the pivot bearings **346.** In such embodiments, the belt **384** may be wrapped around this portion of the pivot pins **366** as shown in **FIG. 27****.** In some embodiments, the shape of the camera assembly **350** may differ such that the belt **384** may wrap around the camera assembly **350.** For example, the camera assembly **350** may be a substantially cylindrical shape. The substantially cylindrical shape of the camera assembly **350** may be coaxial with the pivot pins **366.** In such embodiments, the belt **384** may be wrapped around the circumference of camera assembly **350.**

In some embodiments, the surface over which a belt **384** is wrapped may be recessed (e.g., V-shaped) in relation to the surfaces which flank it. This may help to keep the belt **384** in place during operation. In other embodiments, any other type of guide may be used. For example, the surface over which a belt **384** is wrapped may be flanked by two walls which keep the belt **384** in place during operation.

A belt **384** may be made of a high friction material so that the belt **384** does not slip over the surface which it wraps around as the belt **384** is driven. In some embodiments, the belt **384** may have a coarse surface, or may be toothed to aid in its ability to grip or positively engage a camera assembly pivot pin **366** (which may be geared). Use of a belt **384** may allow for a wide range of pivoting of the camera assembly **350** without the need for a pull-cable pivot actuator to be redirected laterally within the insertion section **14** to achieve an equivalent range of motion of the camera assembly **350.** This allows the insertion section **14** to be made with a smaller diameter.

In embodiments using a belt **384,** the belt **384** may be configured to be driven by displacement of the pivot control structure **100** (see **FIG. 14**). In some embodiments, the opposite end of the belt **384** from that which wraps around the camera assembly **350** or pivot pins **366** may wrap around the pivot shaft **204** of the pivot control structure **100.** In such embodiments, rotation of the pivot shaft **204** may drive the belt **384.** The portion of the pivot shaft **204** which the belt **384** wraps around may have a relatively large diameter. This may be desirable so that only a small pivotal displacement of the pivot shaft **204** is needed to drive the belt **384** a relatively large amount. In embodiments where the belt **384** includes teeth, the teeth of the belt **384** may interdigitate with a gear located on the pivot shaft **204** of the pivot control structure **100.** In such embodiments, rotation of the pivot shaft **204** and gear on the pivot shaft **204** may drive the belt **384.** As the belt **384** is driven, the movement of the belt **384** will exert a driving force on the camera assembly **350** causing the camera assembly **350** to pivot.

In other embodiments, the pivot actuator may be the rack of a rack and pinion arrangement. In such embodiments, the pivot pins **366** of the camera assembly **350** may include a toothed portion. The toothed portion of the pivot pins **366** may be the pinion gear that interdigitates with the rack of the pivot actuator. As the rack displaces longitudinally within the insertion section **14,** this motion is translated into rotation of the camera assembly **350** via the toothed, pinion portion of the pivot pins **366.** While such an embodiment does not solely rely on a pulling force to rotate the camera assembly **350,** the pivot actuator still does not require lateral displacement of the actuator within the insertion section **14.** In some specific embodiments, a push-pull rack-type actuator may nevertheless require features (e.g., rigidity, thickness) or may otherwise be constrained within a track to prevent lateral or side-to-side flexion during the application of a compressive force on the rack

In yet another arrangement using one or more panning cables, a similar pivotal range may be achieved without requiring any routing of a panning cable through various features included in a camera assembly mount **330.** This may be desirable because it may allow the diameter of an insertion section **14** (see **FIG. 1**) to be made smaller. Additionally, a camera assembly mount **330** for such an embodiment would not require any fenestrations (e.g. the cable guide holes **348** of **FIG. 18**) or re-directing elements/constraints (e.g. the constraining notch **349** of **FIG. 18**) thus simplifying manufacture of a camera assembly mount. Such an embodiment may for example use the camera assembly mount **330** and inner sheath **312** shown in the example embodiment in **FIG.** 1**9**.

In such an embodiment, a camera assembly **350** may include one or more spooling features or surfaces **1400.** The spooling feature is configured to at least partially wind the terminal portion of a panning cable around the housing of the camera assembly **350.** A connection or attachment point for the terminal end of the panning cable may be situated on the camera assembly housing distal to the spooling feature. The spooling feature preferably has a curved, somewhat recessed surface, which may partially or completely wrap around a portion of the camera assembly housing. Thus, in various embodiments, a panning cable may wind around the housing only partially, or in one or more complete loops around the housing. A longer spooling feature provides for a more extensive range of rotation of the camera assembly. During actuation, an associated panning cable may be wound or unwound from the spooling feature **1400.** Spooling feature **1400** may increase the pivotal range of a camera assembly **350.** Spooling feature **1400** may allow a more consistent torque to be applied to a camera assembly **350** during rotation. Spooling feature **1400** may be constructed to create a moment arm of desired or varying length. Additionally, positioning the spooling feature **1400** radially apart from the axis of rotation of the camera assembly may help a panning cable to generate rotational torque more efficiently.

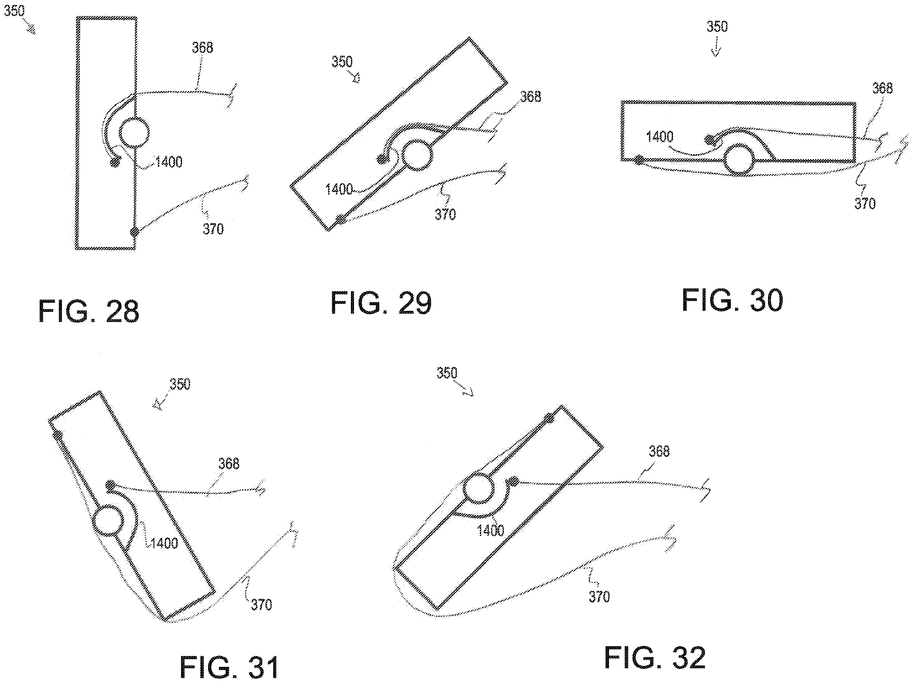

The progression of **FIGS. 28-32** conceptually illustrate a camera assembly **350** including a spooling feature **1400** in a number of rotational positions. As shown, the spooling feature **1400** may include an arcuate portion and a straight portion. The arcuate portion is shaped such it has a radius of curvature which extends from the pivot axis of the camera assembly **350.** The straight portion of the spooling feature **1400** is angled such that is serves as a torque increasing feature. Additionally, the straight portion of the spooling feature **1400** allows the camera housing **355** to be made with more material (which would otherwise need to be removed to continue the arcuate section) and thus increases the structural integrity of the camera housing **355.** This may be particularly important in embodiments where the camera assembly **350** is designed to fit in a very small space and thus must be made with a very small form factor.

As shown in **FIG. 28** the top panning cable **368** may be wound around the spooling feature **1400.** A pulling force exerted by the top panning cable **368** would create a torque about the pivot axis of the camera assembly **350** causing the camera assembly **350** to rotate in a clockwise direction. Additionally, the straight portion of the spooling feature **1400** creates a longer moment arm thus increasing the torque generated for a given amount of pulling force.

As the camera assembly **350** rotates to the position shown in **FIG. 29****,** the top panning cable **368** begins to unwind from the spooling feature **1400.** As force continues to be applied and the camera assembly continues to rotate, the top panning cable **368** will continue to unwind from the spooling feature as shown in **FIG. 30****.** When sufficiently unwound, the point at which the top panning cable **368** leaves the spooling feature **1400** will be located on the arcuate section of the spooling feature **1400** (as shown in both **FIG. 29** and **FIG. 30**). In an embodiment, all points on the arcuate section of the spooling feature **1400** may be located an equal distance from the pivot axis.

In an exemplary embodiment, as a pulling force continues to be exerted by the top panning cable **368,** the camera assembly **350** will continue to rotate until the top panning cable **368** no longer contacts the surface of the spooling feature **1400** as shown in **FIG. 31****.** The camera assembly **350** may then continue to rotate until the pulling force of the top panning cable **368** approaches coincidence with the axis of rotation of the camera assembly **350.** This position is depicted in **FIG. 32****.** As would be understood by one skilled in the art, a panning cable **368** may be wound around a spooling feature **1400** one or more times to increase the amount of rotation which may be created using the panning cable **368.** The degree to which a panning cable **368** winds around a contact surface on the camera assembly **350** allows for a range of rotation of the camera assembly **350** that exceeds 90 degrees. The degree of rotation of the camera assembly **350** would then be limited only by the amount of slack and the flexibility of the attached electronic flex cable and/or the optical fiber bundle.

In an embodiment, the panning cable and spooling surface are arranged to permit the camera assembly **350** to rotate to a position between about 90 degrees to about 120 degrees of the long axis of the distal endoscope shaft, orienting the lens surface of the camera assembly at least partially in the direction of the proximal end of the endoscope shaft. In this position, any debris or other contamination of the lens surface may be washed away by irrigation fluid traveling distally in the endoscope shaft.

To rotate the camera assembly **350** from its position in **FIG. 32** to the position shown in **FIG. 30****,** a pulling force may be exerted via the bottom panning cable **370.** In some embodiments, the bottom panning cable **370** may also be associated with a spooling feature. For example, the corners or edges of the camera assembly **350** around which the bottom panning cable **370** may wrap may be rounded.

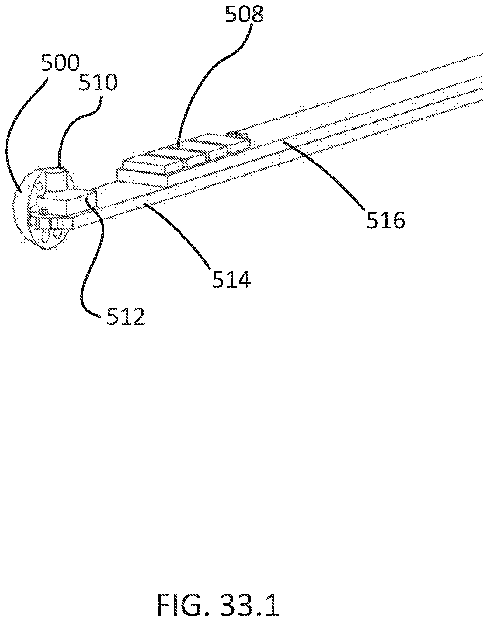

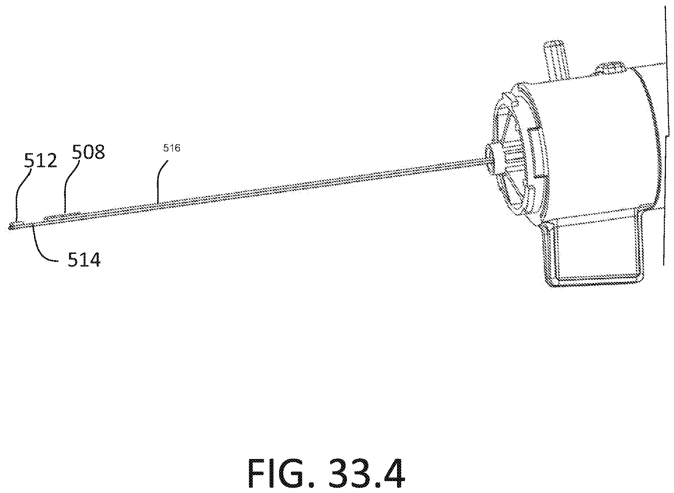

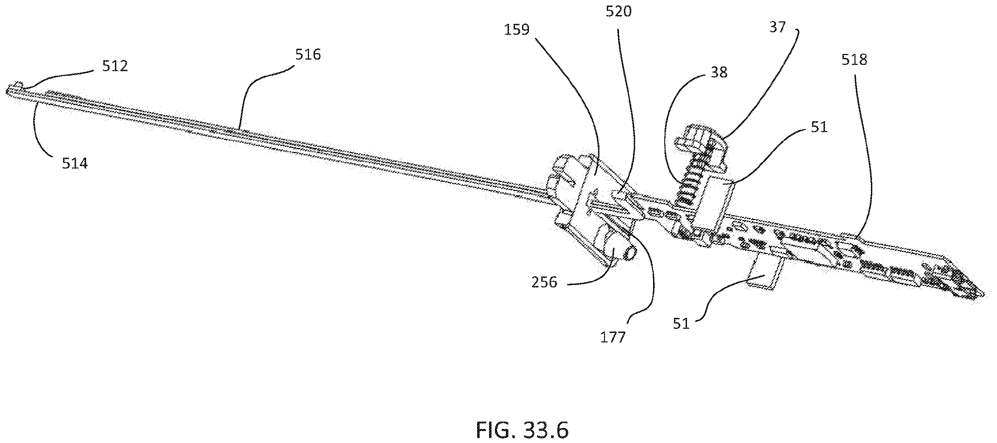

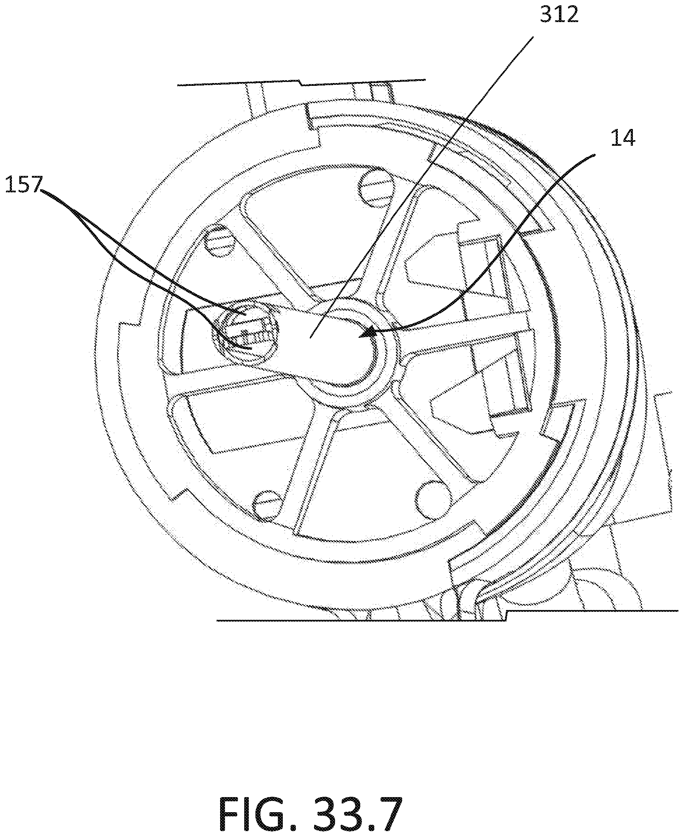

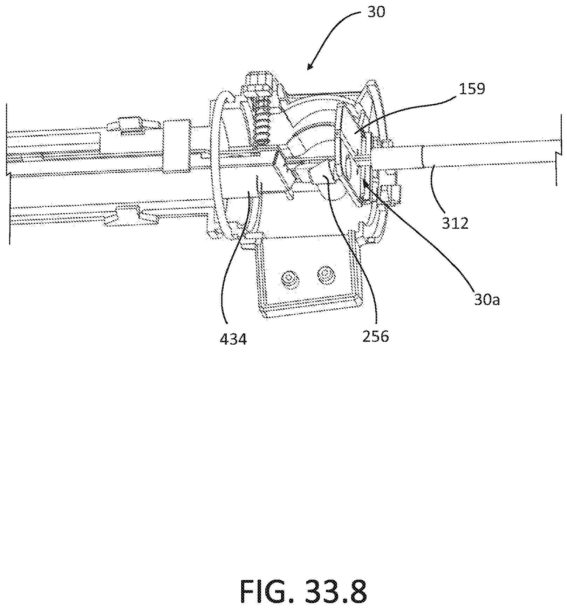

**FIG. 33-34** depicts a top perspective view of a specific example embodiment of a camera assembly **350** which includes a spooling feature **1400.** The camera assembly **350** includes a lens assembly **354.** The lens assembly **354** is disposed inside of a camera housing **355.** The spooling feature **1400** may be recessed into a side of the camera housing **355** as shown. The spooling feature **1400** in the example embodiment includes an arcuate portion and a straight portion. The arcuate portion of the spooling feature **1400** is shaped such it has a radius of curvature which extends from the center of the pivot pins **366** or pivot axis.

As best shown in **FIG. 33****,** the wall into which the spooling feature **1400** is recessed may include a first void **1402.** The camera housing **355** may also include a second void **1404.** The second void **1404** may pass through the top face of the camera housing **355** to the bottom face of the camera housing **355.**

As shown, only a single panning cable **1406** may be used. The panning cable **1406** may extend through both the first void **1402** and the second void **1404** in the camera housing **355.** One end of the panning cable **1406** may be attached to a cable attachment hole **202** on the pivot arm **198** (see **FIG. 14**). The other end of the panning cable **1406** may be attached to the other cable attachment hole **202** on the pivot arm **198.** In some embodiments, the panning cable **1406** may be fixedly attached to the camera housing **355** at one or more points. For example, an adhesive of glue may be placed into one of the voids **1402** or **1404.** This may ensure that the panning cable **1406** does not slip or move over the surface of the camera housing **355** during actuation. Additionally, in some embodiments, the panning cable **1406** may be knotted in one or more location. For example, the panning cable **1406** may be fed through one of the voids **1402** or **1404,** knotted, and then fed through the other of the voids **1402** or **1404.** Preferably, the width of the knot may be sufficiently wide so as to not fit through either of the voids **1402** or **1404.** Such a knot may again help to keep the panning cable **1406** from slipping or moving over the surface of the camera housing **355** during actuation.

As would be appreciated by one skilled in the art, the embodiment shown in **FIG. 33-34** may easily be modified to use two panning cables. One panning cable may terminate and be fixedly attached to the camera housing **355** in or at the location of the first void **1402.** A second panning cable may terminate and be fixedly attached to the camera housing **355** in or at the location of the second void **1404.**

In an alternative example, FIG. 23.2 shows how a pull wire 502 operated by the pivot control structure 100 may be wrapped around and/or attached to a rounded or dome-shaped sensor or camera housing 500 at the distal end of the endoscope shaft 14. The inner sheath 312 has been removed for clarity. In this example, camera housing 500 includes a nearly circumferential slot 501 offset to one side of the housing 500 so as not to interfere with the lens/camera assembly contained within the housing 500 (which is preferably positioned at about the center of two assembled halves of the housing). The pull wire 502 is retained within slot 501, and may be secured to the housing 500 at recess 503. A knot placed in the pull wire 502 may be embedded in recess 503 to act as an attachment point that causes the housing 500 to rotate when the pull wire 502 is moved back and forth along the endoscope shaft. Optionally, a small amount of adhesive may be used to provide added attachment security during assembly of the endoscope. In a preferred embodiment, the pull wire 502 comprises a Kevlar thread, which provides substantial longitudinal strength and resistance to stretching. Other wire types may include steel (braided or single-strand), nylon, or other materials of suitable strength and resistance to stretching.

The rounded sensor housing 500 shown in FIG. 23.1 and 23.2 can enclose a more simply constructed lens 510 and image sensor 512, and the source of light for illumination of the operative field can be located near the sensor/camera housing 500 without having to be mounted to the housing itself. FIG. 23.3 and FIG. 23.4 show how a rounded or dome-shaped sensor/camera housing 500 can be constructed from two sections 500a and 500b. Each of these sections can be molded or machined to have internal cutouts for placement of the distal end of a PCB extension 514 or flex cable, its associated sensor 512 (e.g. CMOS or CCD) and a suitable lens 510. The two sections 500a and 500b can be joined over these components by a number of methods. In the example shown, one or more pins 500c of section 500b can be mated with a corresponding arrangement of matching recesses 500d of section 500a. As shown in FIG. 23.5, a pivot shaft 505 on the outer side of each section can be inserted into corresponding holes, bearings or bushings 507 of the distal end of the inner sheath 312 of the endoscope shaft, which may be resiliently flexible to allow for assembly. An assembled housing 500 can be pressed or snap-fit into place within the holes 507, which may help to keep the two sections 500a and 500b joined together. Optionally, an adhesive may also be used to securely join the pins 500c to their corresponding recesses 500d. Although the illustrated sensor/camera housing 500 does not include a light source, this is an option; a suitably sized LED or group of LED's can be included around the periphery of the lens, or the terminus of a fiberoptic cable can be installed in a similar arrangement, as discussed below.

In an exemplary arrangement, one or more LED's 508 can be positioned along an open portion 506 of the inner sheath 312 of endoscope shaft 14. FIG. 33.1 shows a sensor 512 and lens 510 assembly within sensor housing 500 (half of the housing having been removed for clarity). Both the lens 510 and sensor 512 (e.g. a CMOS or CCD sensor) are suitably sealed to prevent liquid from entering between them. The sensor 512 may be connected to a ribbon or flex cable running through the endoscope shaft to the PCB in the endoscope handle. Similarly, the light source/LED's may be connected to the ribbon or flex cable to receive electrical power, or may be connected to a separate ribbon or flex cable adjacent to the sensor communications cable. In an alternative example, the main PCB in the endoscope handle can be manufactured with an elongate extension arranged to extend through the endoscope shaft to one or more components at the distal end of the shaft. The PCB may be constructed to have a flexible component sandwiched together with a rigid component. The flexible component may emerge from the main PCB to form a PCB extension for the endoscope shaft. The rigid component may similarly emerge from the main PCB to form a PCB extension for the endoscope shaft. Either or both of these extensions may alternatively be combined with a ribbon or flex cable coming from the main PCB to provide power or communications to components at the distal end of the endoscope shaft. For example, sensor 512 can be mounted to the distal end of a flexible PCB extension 514 from the main PCB in the endoscope handle 12. The sensor PCB extension 514 is flexible and has sufficient slack to permit rotation of the sensor 512 and lens 510 as the sensor housing 500 is rotated. Also, the light source in this example comprises one or more LED's 508 mounted to either the sensor PCB extension, or preferably mounted to a separate light source PCB extension 516. Because of the power requirements of the light source, mounting the power supply wires on a separate flex cable or PCB extension 516 may increase the reliability of the endoscope. Also, if the endoscope shaft 14 is rigid (e.g. as would be the case for most arthroscopes), the light source power wires can be mounted to a rigid PCB extension, further enhancing the overall robustness of the endoscope. In the example shown in FIG. 33.1, a flexible sensor PCB extension 514 has been folded over at its base and placed against a rigid light source PCB extension 516, both extensions merging into the main PCB within the handle 12 of the endoscope (see below).

The form factor for the printed circuit board (PCB) for the embodiment shown in FIGs. 23.1 and 33.1 is shown in FIG. 33.2. Note that the form factor shown in FIG. 33.2 and 33.3 may also be used to represent how a ribbon or flex cable can be folded over to run adjacent a companion ribbon or flex cable, or adjacent a companion PCB extension through the bulkhead and along the endoscope shaft to connected components at the distal end of the shaft. In one example, the rigid main PCB 518 (on which a number of electronic processing components are mounted) comprises a composite of both rigid and flexible components sandwiched together, the main PCB 518 being located within the endoscope handle. A flexible PCB extension portion 514 emerges from the composite main PCB 518 at an angle to the direction of a rigid extension portion 516, so that a proximal leg 514a of the flexible extension portion 514 can be folded over at point 520 (FIG. 33.3) to run adjacent to the rigid portion 516, as shown in FIG. 33.3. In the example shown, the proximal leg 514a of the flexible board extension is at an angle of approximately 90 degrees with respect to the rigid board extension. In some embodiments, the angle may be less than or more than 90 degrees, due to the flexibility of the flexible board extension permitting the proximal leg 514a to accommodate an imperfectly aligned fold. The flexible PCB extension 514 is shown running adjacent to the rigid PCB extension 516 in FIG. 33.4 along the endoscope shaft (inner sheath, sensor housing and lens removed for clarity). In this manner, both PCB extensions can pass through an elastomeric slot in a bulkhead or fluid barrier, such as the slot 177 shown in FIG. 11B or 11C.

FIG. 33.5 shows a cutaway view of an exemplary endoscope. The main PCB 518 is shown in relation to the handle proximal section 16, the camera control button 37 the bulkhead or pass-through barrier 159, and pivot control structure 100. Rotational position sensor magnets 51 are shown both in relation to the handle proximal section 16 and the main PCB 518. An exemplary fluid conduit 434 (for irrigation or suction) is shown approaching the bulkhead 159. It may be connected to bulkhead 159 through a barb fitting 256 as shown in FIG 33.6, or through any other suitable means of secure connection. In this case, the inner sheath 312 has been removed for clarity of the description.

FIG. 33.6 shows the PCB of the endoscope without the handle, inner sheath, pivot control structure and fluid conduit, as its extensions pass through the bulkhead or pass-through barrier 159. In addition, the rotation position sensing magnets 51 and the camera control button 37 and shaft 38 (with embedded magnet) are shown in relation to the main PCB 518 to provide an indication of where on the PCB 518 the respective Hall effect sensors for those magnets can be located. As shown, the fold point 520 of the flexible PCB extension is located proximal to the bulkhead 159, so that the combined adjacent flexible and rigid PCB extensions may pass through the bulkhead at slot 177. The slot in this case can be sealed securely against fluid infiltration, because a sufficient amount of slack in the flexible PCB extension 514 can be provided distally near its termination at the sensor 512.

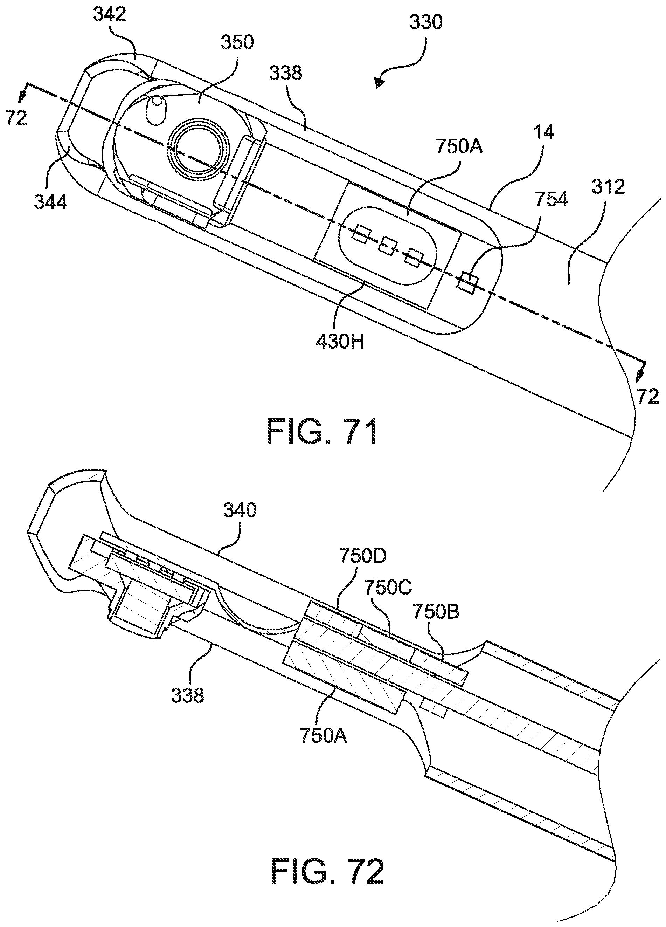

As shown in FIG. 33.7, a fluid or air carrying lumen 157 shares space within the endoscope shaft 14 with the PCB extensions 514, 516 (or alternatively with one or two ribbon or flex cables), which supply power to the light source and communications with the sensor/camera located at the distal end of the endoscope shaft 14. The sensor/camera housing has been removed for clarity. (Note that the inner sheath 312 of the shaft 14 has a cutout or opening proximal to the sensor/camera housing, above to provide illumination by the light source or LED's, and optionally below to improve fluid flow around the sensor/camera housing. This is shown, for example in FIGs. 23.1 and 72.6).

FIG. 33.8 shows a cutaway view of the interior of the handle distal section 30 of the above endoscope. The bulkhead or fluid barrier 159 separates a relatively dry region (in which the pivot control structure, camera control button, and distal end of the main PCB are located) from a wet section 30a. A fluid or air conduit 434 runs from the outside of the endoscope handle, through the handle proximal section 16, and connects to a port 256 of the bulkhead 159. In this example, fluid passing through the conduit 434 and port 256 communicates with the lumen 157 of the endoscope shaft 14 via the space occupied by the wet section 30a. Appropriate seals can be used to contain this fluid so that fluid or air does not leak out between the distal section housing and the proximal end of the endoscope inner sheath 312.

Referring now back to **FIGS. 7C** and **14****,** the pivot control structure **100** may be capable of being "parked" in detents defined by ridges **94** in the slide button recess **92** of the handle raised portion **34** or another portion of the handle **12.** In some embodiments, the ridges **94** may be spaced such that the detents formed by the ridges **94** may correspond with specific angular orientations of the camera assembly **350.** In some embodiments, the detents formed by the ridges **94** may be spaced such that their location corresponds to specific angular increments (e.g. 30°) of the camera assembly **350.**

As mentioned above (see **FIG. 7A**), the handle distal section **30** may be rotatable relative to the handle proximal section **16.** Such rotation would also cause the longitudinal axis of the insertion section **14** to rotate as well. In turn, the camera assembly **350** may rotate with the insertion section **14.** This may allow a user to get a near-global view of the anatomical area in question with minimal to no angular repositioning of the endoscope **10.** A user may need only to pan the camera assembly **350** and rotate the handle distal section **30** relative to the handle proximal section **16** to obtain a desired field of view within an anatomical area.

Repeated contortion and bending of optical fibers such as the optical fibers **364** may lead to fracturing or failure of one or more fibers. In the instance of the optical fibers **364,** this leads to light and illumination loss which increases as more optical fibers **364** become compromised. Such bending may occur if the optical fibers **364** terminate and are attached or fused to a portion of a pivoting camera assembly **350** as described above. If the endoscope **10** is designed to be disposable, then any decrement in the integrity or performance of the optical fibers **364** may be within acceptable limits relative to the intended lifespan of the instrument. Consequently, in some embodiments the optical fibers **364** may be attached or fused to a pivotal camera assembly **350** with minimal concern for optical fiber **364** breakage and resultant light loss. A terminal illuminator, light projection element or light emitter associated with the optical fibers **364** may, in some embodiments, be advantageously mounted to the camera assembly **350** in order to project light at whatever target or field of view a lens assembly **354** of the camera assembly **350** has been rotated or panned to. Such an arrangement helps to ensure that the field of view (shown with dashed lines in **FIG. 23-25**) for the lens assembly **354** is always illuminated by the optical fibers **364** regardless of where the in the camera assembly's **350** pannable range the camera assembly **350** has been rotated to.

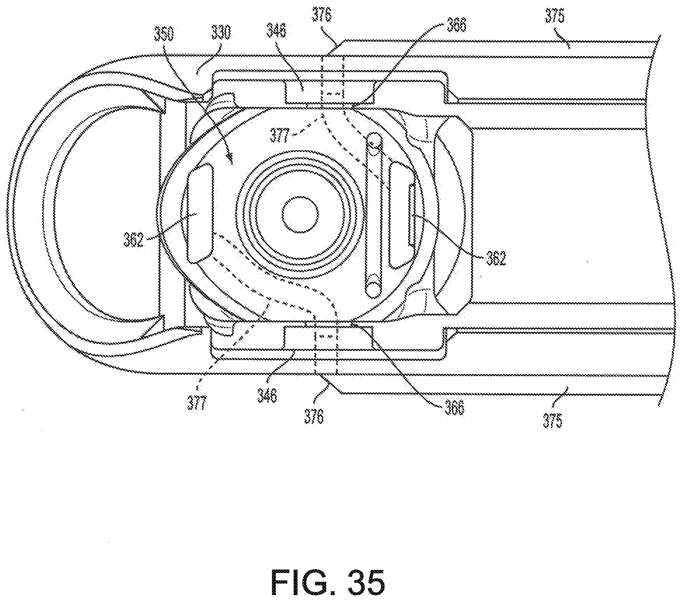

In some embodiments, the illumination system may include a light guide or light pipe **375.** In some embodiments, the optical fibers **364** may comprise a light guide or light pipe **375** (see, for example, **FIG. 35**) along at least a part of the path of the illumination system. The terms "light guide" and "light pipe" are herein used interchangeably. When an optical fiber is relatively straight, light loss is relatively small because the angle of incidence of the light within the fiber is shallow enough to facilitate near total reflection within the optical fiber. Bending the optical fiber, however, may alter the angle of incidence to the point where some transmission of light out of the fiber is possible. Bends of a light pipe or guide may, however, be controlled. For this reason, use of a light guide **375** where feasible may help to minimize light loss in an illumination system comprising optical fibers **364** or may replace the optical fibers altogether. A light guide **375** may also provide a number of other benefits. For example, a light guide **375** may aid in assembly and shorten assembly time for a device. The light guide **375** may be of the types described herein or may be any suitable type light guide known to those skilled in the art.

**FIG. 35** shows an example embodiment of an endoscope **10** utilizing light pipes **375.** Two larger diameter light pipes **375** may extend along one or more sections of the wall of the inner sheath **312** (see **FIG. 18**) to the camera assembly housing **330** and then bend or curve into one of the camera assembly pivot bearings **346.** The bent section of each light pipe **375** may be coated with a highly reflective material **376** in order to minimize loss of light out of the light pipe **375** as it changes direction. Any suitable highly reflective material **376** known to one skilled in the art may be used. In such embodiments, the camera assembly **350** may also have built-in camera assembly light pipes **377** that are formed in a junction with the light pipes **375** at the pivot bearings **346.** The light carried by the light pipes **375** may be transferred to the camera assembly light pipes **377** at the junction. The camera assembly light pipes **377** may extend from each of the pivot pins **366** into the camera assembly **350.** The camera assembly light pipes **377** terminate in the light projection voids **362** so that the field of view of the camera assembly **350** will be illuminated regardless of the rotational position of the camera and lens assemblies. In such an embodiment, any bends taken by the camera assembly light pipes **377** may be coated with a highly reflective material **376** as described above. In some embodiments, the highly reflective material **376** may be included on other portions of the light pipes **375** and camera assembly light pipes **377** in addition to the bends of the light pipes **375** and camera assembly light pipes **377.**

Creating a light pipe junction coinciding with the pivoting region of the camera assembly **350** may be desirable because it avoids the bending or twisting of optical fibers **364** as the camera assembly **350** is rotated, removing the risk of damage to the optical fibers **364.** Such a design can be adapted for use in either a reusable or disposable endoscope **10.** This arrangement may also reduce the manufacturing or assembly costs of the endoscope **10.**

In another example embodiment (not shown) which uses light pipes **375,** a larger diameter light pipe **375** may extend substantially along the path of the flex cable **250.** The end of the light pipe **375** nearest the inner sheath mount **160** may form a junction with the optical fibers **364** or be arranged to draw in light from another illumination source. The end of the light pipe **375** nearest the camera assembly **350** may also form a junction with illumination fibers **364** which extend to the camera assembly **350.**

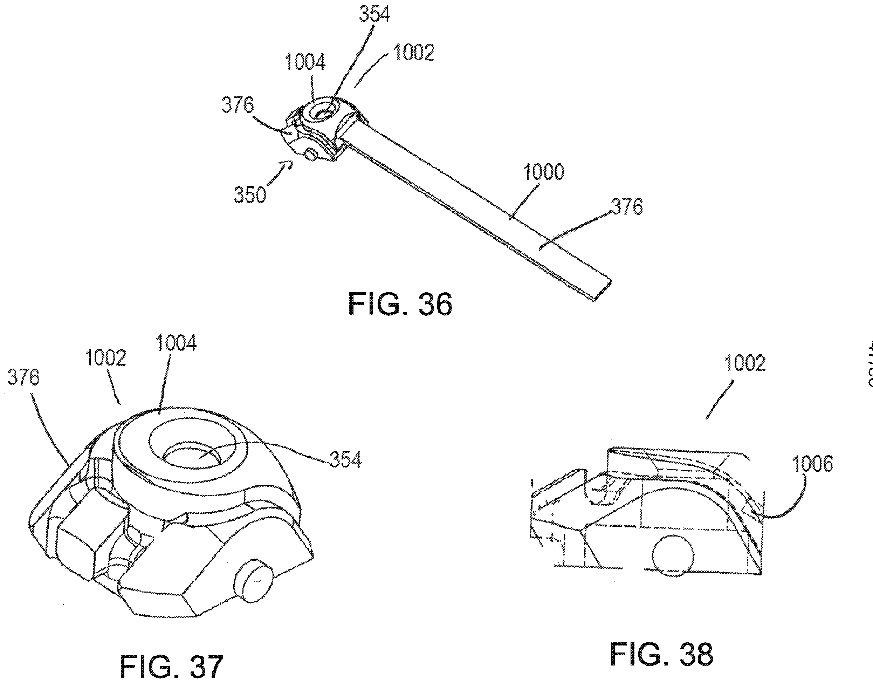

In some embodiments, the optical fibers **364** to the camera assembly **350** may be arranged to form a flexible ribbon **1000,** creating a linear array of fibers that can be terminated into a light projection element with minimal bending or bending in only one dimension (see, e.g., **FIG. 36**). Alternatively, the flexible ribbon **1000** need not be a linear array of fibers and instead may, in some embodiments, be a single, ribbon-like, flexible piece of light guide material. In some embodiments there may be two flexible ribbons **1000** each extending to one of the light projection voids **362** in the camera assembly **350.** In some embodiments, the flexible ribbons **1000** may be coated with a reflective material **376** to maximize the amount of light at the camera assembly **350.** In some embodiments, a flexible ribbon **1000** may form a junction with a light pipe.

In some embodiments, a camera housing top **356** may comprise a light piping material to serve as a light projection element or illuminator. In this case, light may be emitted from most of the camera housing top **356** and into the viewing field of the camera assembly **350.** In some embodiments, some areas of the camera housing top **356** may be blacked out or masked so that light is only emitted from a desired region or regions of the camera housing top **356.** In some embodiments, some regions of the camera housing top **356** may be coated with a highly reflective material **376** to prevent the unwanted emission of light from those areas.

**FIG. 36** shows an embodiment in which the optical fibers **364** are incorporated into a flexible ribbon **1000,** which optionally may be coated in a highly reflective material **376.** As shown, the flexible ribbon **1000** extends to the camera assembly **350.** The flexible ribbon **1000** may be over-molded to, potted into, fused with or otherwise coupled to the camera assembly **350.**

In the example embodiment in **FIG. 36** the camera assembly **350** comprises a monolithic camera housing **1002.** An example monolithic camera housing **1002** without an attached flexible ribbon **1000** is shown in greater detail in **FIG. 37****.** In the example embodiment, the monolithic camera housing **1002** is made from a light piping or transmitting material and functions as a light projection element. The monolithic camera housing **1002** in the example embodiment may be nearly entirely coated with a highly reflective material **376** to maximize light output from the non-coated or non-masked regions of the monolithic camera housing **1002.** A light projection or illumination surface **1004** having a shape suitable for placement adjacent a lens and image sensor assembly on the monolithic camera housing **1002** may be constructed by masking the area during application of a highly reflective material **376** (or alternatively a simple dark mask). In the example embodiment, the light projection surface **1004** has the shape of a ring. In other embodiments, the light projection surface **1004** may be crescent-shaped, semi-circular, or may have any other desired shape. Light may be emitted from the light projection surface **1004** of the monolithic camera housing **1002** to illuminate the field of view of the lens assembly **354.** As in the above-described embodiments, the field of illumination preferably pivots with the camera assembly **350,** ensuring that the field of view of the lens assembly **354** is always illuminated.

**FIG. 38** shows another example embodiment of a monolithic camera housing **1002.** As shown in outline form, the monolithic camera housing **1002** includes a coupling recess **1006.** The coupling recess **1006** may allow a flexible ribbon **1000** to be suitably coupled into the monolithic camera housing **1002.** In some embodiments, the coupling recess **1006** may allow a flexible ribbon **1000** to be coupled, for example, via snap fit into the monolithic camera assembly **1002.** In some embodiments, the coupling recess **1006** may accommodate optical fibers **364** not formed in a flexible ribbon **1000.** Similar to **FIG. 37****,** in **FIG. 38****,** the monolithic camera housing **1002** may function as a light projecting element. The monolithic camera housing **1002** may also be similarly coated and/or masked as the monolithic camera housing **1002** described in relation to **FIG. 37****.**

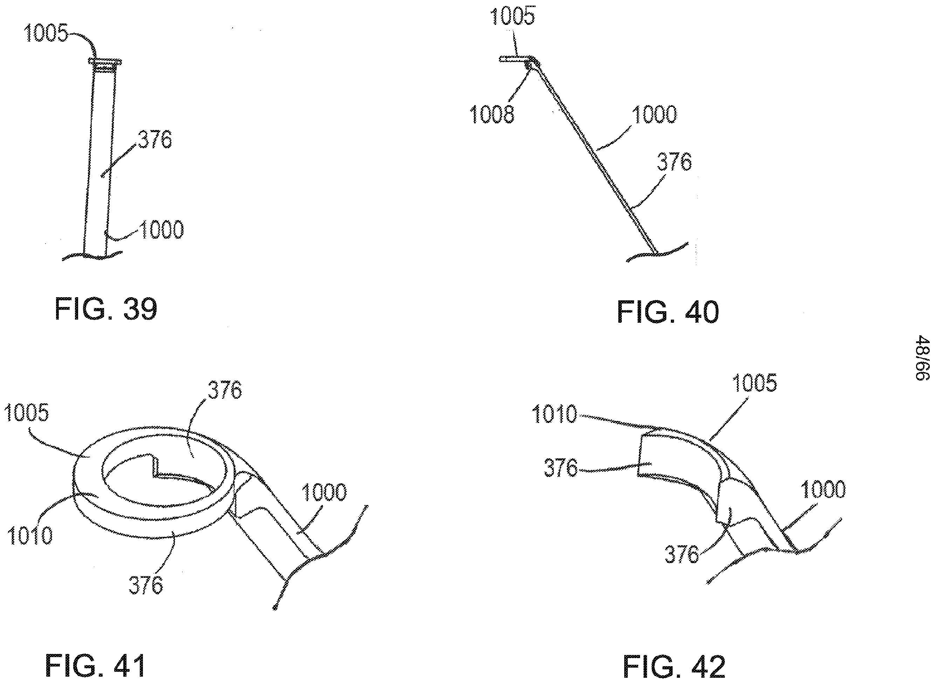

**FIGS. 39** and **FIG. 40** show an embodiment in which a light projection element **1005** is incorporated in an end of a flexible ribbon **1000.** The light projection element **1005** may be formed from a light piping material, which in some embodiments may be a fusion of a group of fibers into a shape suitable for projecting light from a fiberoptic bundle or flexible ribbon **1000** in a desired manner. In some embodiments, the light projection element **1005** and flexible ribbon **1000** may be two separate parts fused together (e.g., by heating or by chemical means). In other embodiments the light projection element **1005** and flexible fiberoptic ribbon **1000** may be a single molded part. In some embodiments the light projection element **1005** may be created as described in relation to **FIGS. 49-62.**

Still referring to **FIG. 39** and **40****,** the flexible ribbon **1000** may be coated with a highly reflective material **376.** The bottom and side walls of the light projection element **1005** may also be coated with a highly reflective material **376.** This may ensure that light is only emitted from the non-coated top of the light projection element **1005** and into the field of view of the lens assembly **354.** As show in **FIG. 40****,** the light projection element **1005** or the flexible ribbon **1000** may include a coupling feature **1008.** The coupling feature **1008** may allow the light projection element **1005** and flexible ribbon **1000** to be coupled onto or into a camera assembly **350.** The coupling feature **1008** may be an integral part of the light projection element **1005.**

**FIG. 41** and **FIG. 42** depict two example embodiments of a flexible ribbon **1000** which include light projection elements **1005,** which may be formed from a light piping material. The light projection element **1005 in** **FIG. 41** has a generally ring-like shape while the light projection element **1005 in** **FIG. 42** is generally crescent shaped, although other shapes may be selected as desired. In the example embodiments in **FIGS. 41** and **42** only the top surfaces of the light projection elements **1005** are left uncoated with a highly reflective material **376.**

A light projection element **1005** may comprise one or a number of textures **1010** that help to direct the light emitted from the light projection elements **1005.** In some embodiments, the texture **1010** or textures **1010** may be included to encourage light to be emitted in a diffuse manner. The texture **1010** or textures **1010** may be created, for example, during molding of the light projection element **1005,** or alternatively, the light piping material forming the light projection element **1005** may include a fill material that encourages light to be emitted from the light projection element **1005** in a diffuse manner.

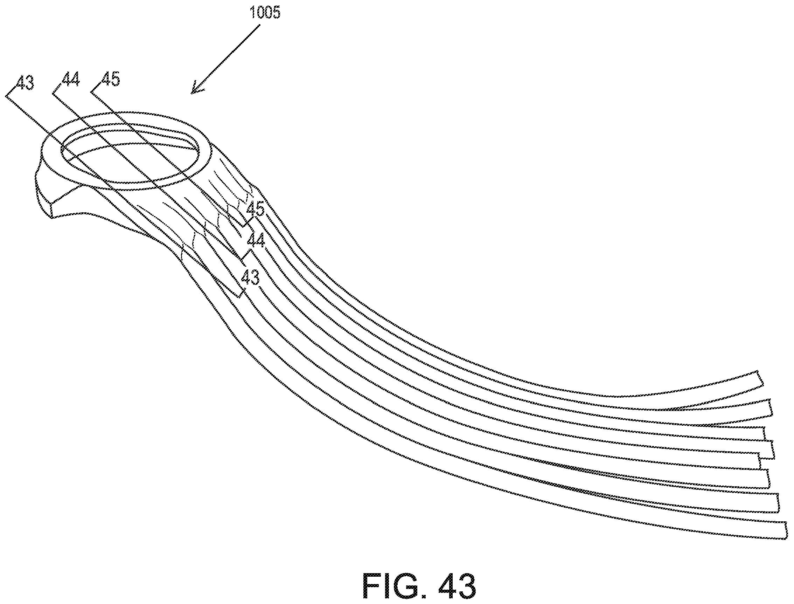

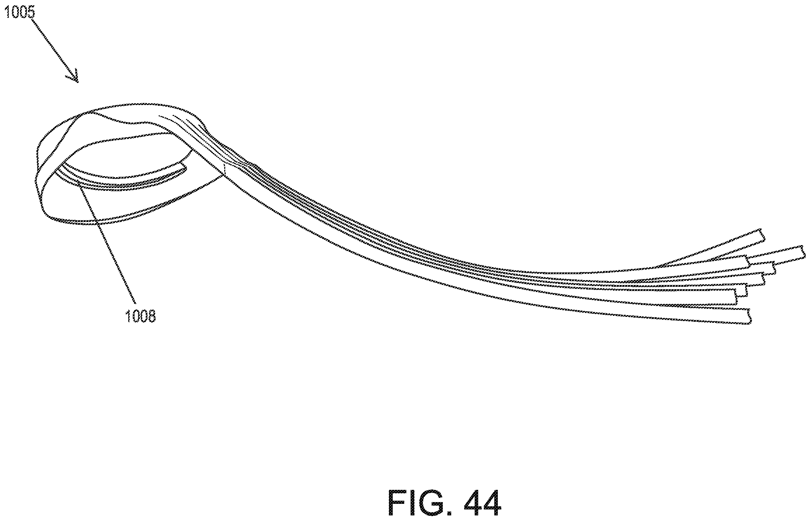

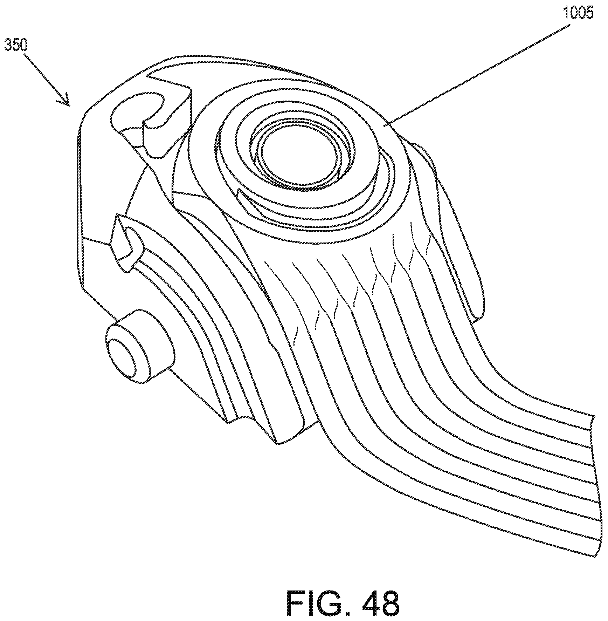

**FIG. 43** and **44** respectively depict top and bottom perspective views of another example embodiment of a light projection element **1005.** As shown, the light projection element **1005** is ring-like in shape. The light projection element **1005** also includes a coupling feature **1008** as shown in bottom perspective view in **FIG. 44****.** The coupling feature **1008 in** **FIG. 44** is an integral part of the light projection element **1005.** In the example embodiment, the coupling feature **1008** is a ledge or shelf. The ledge coupling feature **1008** may help to locate and/or align the light projection element **1005** on another component such as a camera assembly **350.** Additionally, in some embodiments, adhesive or glue may be placed along the ledge coupling feature **1008** to fix the light projection element **1005** to another component such as a camera assembly **350.** The light projection element **1005** is shown attached to an example camera assembly **350 in** **FIG. 48****.**

The light projection element **1005** shown in **FIGS. 43-44** does not include a highly reflective coating or material **376** (see, for example. **FIG. 39**). The need for such a highly reflective coating or material **376** may be minimized by dimensioning the light projection element **1005** to increase or maximize the total internal reflection of light entering and within the light projection element **1005** where the emission of light is not desired. This may be done by ensuring any bend or bends have a large radius in areas of the light projection element **1005** where the emission of light is undesired. Additionally, this may be done by dimensioning a light projection element **1005** such that thickness variations throughout the light projection element **1005** do not introduce changes in the angle of incidence of light within the light projection element **1005** which would make the angle of incidence less than the critical angle. It may be desirable that the thickness of the light projection element **1005** does not decrease to less than the thickness of the optical fibers or flexible ribbon to which the light projection element is attached **1005.** It may also be desirable that the surface of the light projection element **1005** be smooth in areas where the emission of light is not desired.

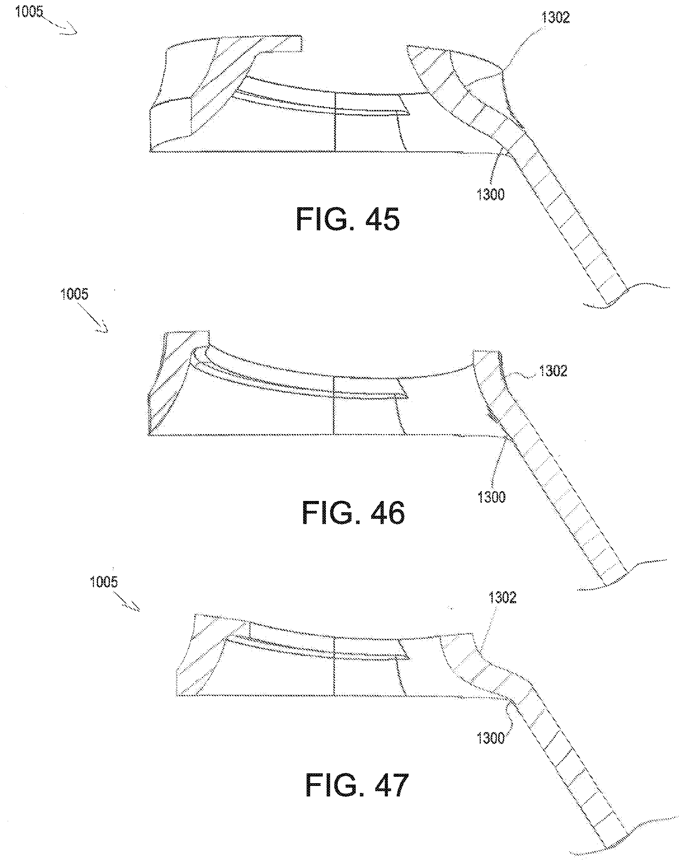

**FIGS. 45, 46,** and **47** depict a number of cross sections of the light projection element **1005** depicted in **FIGS. 43-44****.** The cross sections are respectively taken at lines **43-43, 44-44,** and **45-45** of **FIG. 43****.** As shown, light entering the light projection element **1005** must traverse a first bend **1300** and second bend **1302** before being emitted out of the top surface of the light projection element **1005.** As shown in **FIGS. 45-47****,** the light projection element **1005** may be shaped such that the radii of these bends vary depending on the plane of the light projection element **1005.** The radius of each of these bends **1300** and **1302** may be chosen so as to be as gradual as possible in the available space in a given plane. Also as shown, the thickness of the light projection element **1005** is kept generally constant. This ensures that changes in angle incidence due to thickness variation are minimized.

The light projection element **1005** shown and described in relation to **FIGS. 43-47** is attached to an example camera assembly **350** in **FIG. 48****.** As shown the light projection element **1005** is arranged such that it projects light into a primary illumination field (the surrounding area around this primary illumination field also may be illuminated due to diffusion and reflection of the emitted light) which is substantially coincident with the field of view of the lens assembly **354.**

Exemplary methods for constructing a fiberoptic light projecting element are disclosed in U.S. Patent Application Serial No. 14/170,080 (US Application Publication No. 2014/0221749), filed Jan. 31, 2014, and incorporated herein by reference in its entirety.

**FIG. 63** shows a cross sectional view of an exemplary camera assembly including a lens assembly **354** taken at the cross-sectional plane represented by line **61-61** of **FIG. 24****.** The lens assembly **354** is shown housed between the camera housing top **356** and camera housing bottom **358** as in **FIG. 24****.** As shown, the lens assembly **354** is positioned to project an image onto the plane of an image sensor **380.** The type of image sensor **380** may include, for example, a CCD image sensor, CMOS image sensor, etc. Preferably, the image sensor **380** may be housed in a sealed section of the camera assembly **350** to guard against fluid exposure. In a disposable endoscope, a less costly process may be used to seal the image sensor against fluid exposure (e.g., using a clear epoxy compound), because the assembly would not then be designed to withstand the rigors of sterilization and reuse.

As shown in **FIG. 63** the image sensor **380** may be electrically coupled to a flex board **381** of the flex cable **250.** In some embodiments, a conformal coating material may be used to give added protection against moisture, and optionally may be constructed to support the joints of a ball grid array mounting for the image sensor **380.** The flex cable **250** may provide power to the image sensor **380,** as well as the means of conveyance of data and/or commands from/to the image sensor **380.** In some embodiments, a stiffener **382** may be included in the camera assembly **350.** In the example embodiment shown in **FIG. 63****,** a stiffener **382** is positioned to strengthen the structure on which the image sensor **380** is supported, which may help to protect the physical integrity of the image sensor **380.** The stiffener **382** may comprise, for example, a thin aluminum backing (which in an exemplary embodiment may be about 0.002 inch thick).

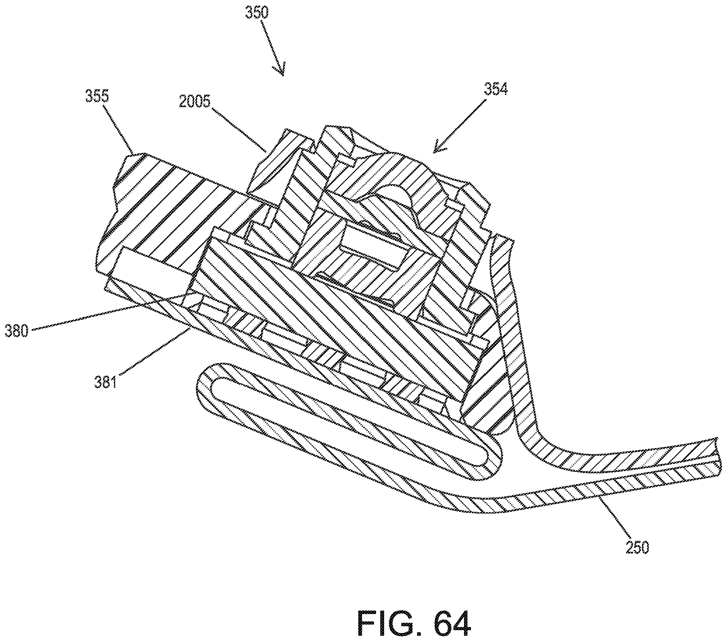

The camera assembly **350** may also include one or a number of fiber guides **384.** In the example shown in **FIG. 63****,** a fiber guide **384** is coupled to the bottom face of the camera housing bottom **358.** The example fiber guide **384** includes a guide trough **386.** The back wall of the guide trough **386** of the fiber guide **384** may be seen projecting toward the bottom of the page in **FIG. 63****.** The fiber guide **384** may also be or include a number of directing notches or channels **388** which in the example fiber guide **384** shown in **FIG. 63** are recessed into the back wall of the guide trough **386.** In some embodiments, including the exemplary embodiment in **FIG. 63****,** directing notches or channels **388** may be formed in one or both of the camera housing top **356** and camera housing bottom **358.** The fiber guide **384** may help to route the illumination fibers **364** during assembly of the endoscope **10.** The fiber guide **384** may also act to keep the illumination fibers **364** in place during operation of the endoscope **10.** The location, shape, number, size, etc. of the fiber guides **384** may vary depending on the specific configuration of the endoscope **10.** In some embodiments, glue, epoxy or another suitable adhesive or agent may be used in addition to the fiber guides **384** to help keep the illumination fibers **364** in the desired location. In some cases, for example, in which light guides or light projection element (as shown, e.g., in **FIG. 35-42** or as shown in **FIG. 64**) are used, fiber guides **384** may not be used in an assembly.

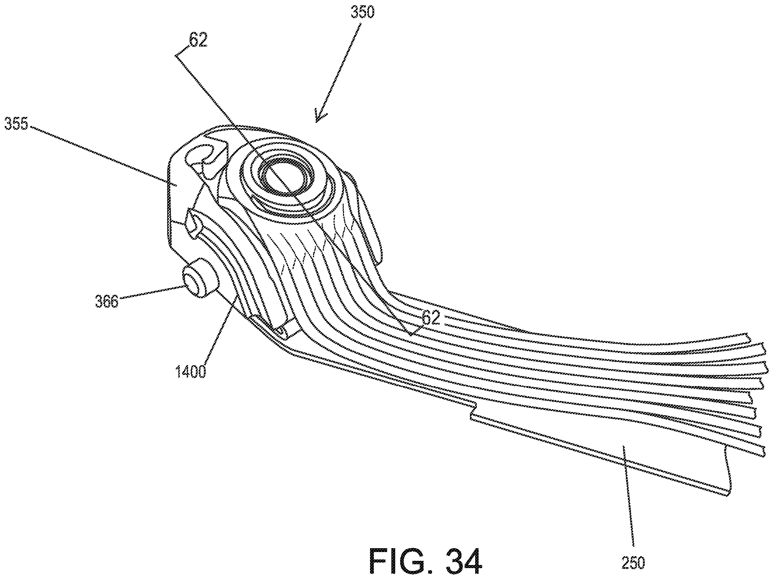

**FIG. 64** depicts a cross section of the camera assembly **350** depicted in **FIG. 34** taken at line **62-62** of **FIG. 34****.** As shown, a lens assembly **354** is shown in place in the camera housing **355.** An image sensor **380** is also shown in place within the camera housing **355.** The lens assembly is positioned to project an image to the image sensor **380.** As above, the image sensor **380** may be any type of image sensor (e.g. CCD, CMOS, etc.) and may be sealed against fluid exposure. Also as above, the image sensor **380** is coupled onto a flex board **381** attached to a flex cable **250**. The camera assembly **350** shown in **FIG. 64** does not include a fiber guide **384** (see **FIG. 63**). Instead a light projection element or light emitter **2005** is in place on the camera assembly **350** in **FIG. 64****.**

As shown, the flex cable **250** is doubled back upon itself in the example embodiment. This may be accomplished by bending the flex cable **250** and then maintaining the bend by applying glue or another fixative to the affected areas of the flex cable **250.** Double-looping the flex cable **250** below the camera assembly **350** may be advantageous in embodiments in which the camera assembly **350** is enclosed in a confined space. For example, confining the camera assembly **350** to the space within an inner sheath **312** as shown in **FIG. 22** may limit the amount of flex cable **250** available for bending. The flex cable **250** may then have to bend over an undesirably small radius in certain rotation positions of the camera assembly **350.** Such a small bend radius can be detrimental to a flex cable **250** especially if it occurs repeatedly. This problem becomes more of an issue as the diameter of the inner sheath **312** decreases. By arranging the flex cable **250** to double back upon itself, however, a greater length of flex cable **250** is available for repeated bending upon rotation of the camera assembly **350** and a larger minimum bend radius may be obtained. Thus, this may allow the inner sheath **312** to then be made with a smaller diameter without concern for the integrity of the flex cable **250** due to the repeated bending and unbending over a small radius.

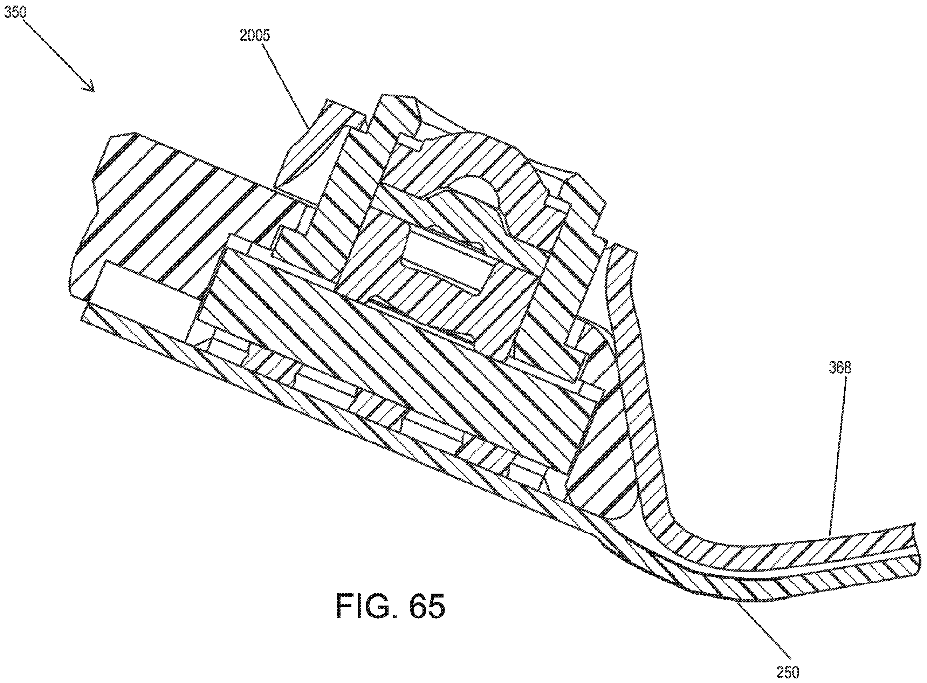

Both the flex cable **250** and the optical fibers **364** leading the light projection element **2005** exhibit some resistance to bending. Additionally, both can exert a restoring spring force when bent. This resistance to bending may increase the camera assembly's **350** resistance to rotation. As shown in **FIG. 65****,** the flex cable **250** and the optical fibers **364** may be angled toward one another. Such an arrangement may leverage the stiffness of the flex cable **250** against the optical fibers **364** or vice versa to assist in rotating camera assembly **350.** To best illustrate this concept, the flex cable **250** is not doubled back upon itself in **FIG. 65****.**

In some embodiments, at least one illumination source for a camera assembly **350** may be positioned to project light in a direction other than into the field of view of the camera assembly **350.** That is, the direct illumination field of an illumination source may be oriented such that it is outside or not coincident with a field of view of a camera assembly. Such an illumination source may be referred to as an indirect lighting source whereas illumination sources which project light directly into a field of view of a camera assembly **350** may be referred to as direct lighting sources. An indirect lighting source may, for example, be oriented such that it emits light behind a camera assembly **350** or in a direction substantially opposite that of the field of view of the camera assembly **350.** For example, instead or in addition to a light projecting element **2005** which couples around a lens assembly **354** or lens and projects light into a field of view of the lens assembly **354** or lens, a light projecting element **2005** may be attached to part of the camera assembly **350** opposite the lens assembly **354** or lens.

Though counter-intuitive, projecting light outside of the field of view of the camera assembly **350** (e.g. behind a camera assembly **350** in a direction opposite the field of view) provides increased image quality and reduces the need for image processing. For example, such an illumination arrangement may help to provide greater depth perception in endoscopic procedures as shadowing of areas which would otherwise be directly illuminated may be maintained. By emitting light from a light projecting element **2005** or other illumination source to points outside the field of view of a camera assembly **350,** hot spots or areas which appear washed out and dark spots or areas which appear underexposed may be mitigated. Such an illumination arrangement may help to provide more uniform lighting within the field of view of the camera assembly **350.**

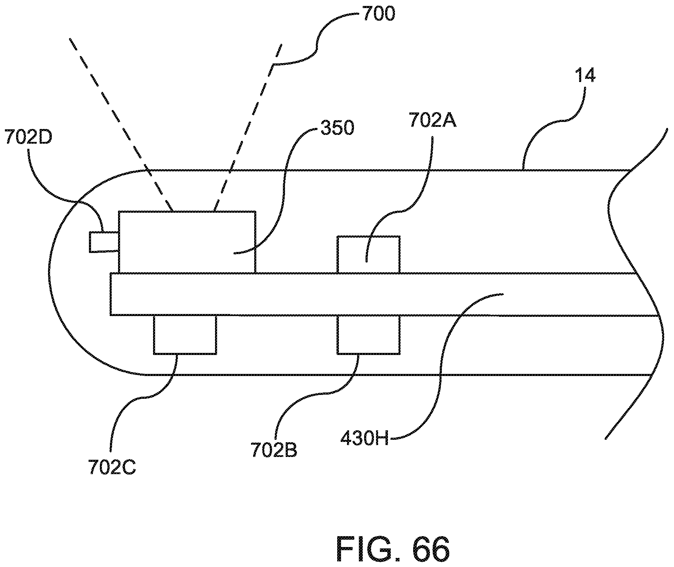

A representational embodiment of an illumination arrangement in which light may be emitted from a number of light sources **702a-d** to areas inside and outside of a field of view **700** of a camera assembly **350** is shown in **FIG. 66****.** As shown, an extension **430h** of a printed circuit board in the endoscope handle is shown extending to the camera assembly **350.** The printed circuit board extension **430h** may provide power and data communication pathways to various components in the insertion section **14.** In some embodiments, a ribbon or flex cable **250** (see, e.g., **FIG. 14**) may be used instead of a printed circuit board extension **430h.** A number of components are mounted to the printed circuit board extension **430h.** These components may be any or a variety of different components such as a sensors, light emitters, etc. In the example embodiment, the components are described as light sources **702a-d.** The light sources **702a-d** may be any suitable light source, such as but not limited to, fiber optic cables, light projecting elements 2005 (see, e.g. **FIG. 62**), LEDs, or arrays of LEDs.

Use of LEDs may be desirable in some specific embodiments for a variety of reasons. For example, use of LEDs may obviate the need for a bundle/ribbon of illumination fibers, some specific embodiments. Some optical fibers may degrade when subjected to prolonged bending during usage and may be prone to light loss when bent. LEDs are long lasting and do not require a lengthy bundle of fibers which a subject to bending. Use of LEDs may also minimize the number of pass-through elements between a dry section (e.g. a handle **12**) of an endoscope and a wet section of an endoscope (e.g. insertion section **14**). Additionally, by omitting optical fibers less of the cross-sectional area of a fluid conduit in an insertion section **14** may be obstructed or filled. This may allow for increased flow rates of irrigation fluid through an insertion section **14.** LEDs may also help to simplify or increase ease of manufacturing.

As shown, a first light source **702a** is disposed such that it may project light generally toward the field of view **700** of the camera assembly **350.** Such a light source **702a** may generally provide direct lighting of the field of view **700.** In some embodiments, a direct light source **702a** may be omitted or may be accompanied by one or more other indirect lighting sources, such as, for example, any or a combination of light sources **702b-d.** In embodiments where a direct lighting source **702a** is included in conjunction with one or more indirect lighting source, the direct lighting source **702a** may provide light at a lower intensity than one or more of the indirect lighting sources. In some embodiments, a direct lighting source **702a** may provide light in a different spectrum or a subset of the spectrums emitted by an indirect lighting source(s). For example, a direct lighting source **702a** may be a RBG LED array while indirect lighting sources may emit white light.

A number of other lighting sources **702b-d** are also shown in **FIG. 66****.** These lighting sources are shown together for illustrative purposes, and need not all be present in any given embodiment. Any number of lighting sources **702a-d** may be included in various embodiments. Some of the lighting sources **702a-d** may be omitted in various embodiments. Each of the light sources **702b-d** is an indirect lighting source is arranged such that they do not emit light directly into the field of view **700** of the camera assembly **350.** Light sources **702b-d** are arranged to emit light in a direction substantially opposite the field of view **700** of the camera assembly **350.** Light source **702d** may be attached to the camera assembly **350** and may be arranged to emit light from a side of the camera assembly **350.** In a preferred embodiment, backlighting provides the only source of illumination of the surgical field. In this case, an illumination source for the distal end of the endoscope may only comprise one or more LED's located at position 702B, for example; that is, projecting light from a side of the endoscope shaft that is away from the side from which most of the field of view of the camera assembly is directed. The light thus provided illuminates the space viewed by the camera assembly more indirectly and diffusely, preventing bright reflections of light aimed directly at the camera or reducing the casting of shadows, and improving the operator's view of the field. On the other hand, placing the LED's at position 702A may improve illumination of the area of the surgical field toward which the camera field of view 700 is directed.

The camera assembly **350** may include one or more optical filters to selectively use the wavelengths emitted from one or more of the light sources **702a-d.** For example, a polarizing filter or band-gap filter may be used to enhance the imaging by the camera assembly **350.**

Depending on the embodiment, the camera assembly **350** may be rotatable within the insertion section **14.** In such embodiments, the lighting sources **702a-d** may remain stationary as the camera assembly **350** is rotated. Alternatively, one or more lighting source **702a-d** may rotate with the camera assembly **350** (e.g. a light source **702a-d** may be attached to the camera assembly **350** and thus rotate with the camera assembly **350**). A light source **702a-d** may be positioned such that it does not emit light directly into the field of view **700** in any or a large percentage (e.g. from around 70% to 100%) of the possible rotational positions of the camera assembly **350.** In some embodiments, the entire distal end of the shaft or insertion section **14** (or the entire insertion section **14**) may be transparent such that the camera assembly **350** may be rotated therein to obtain many viewing angles through the transparent portion or portions; In this specific embodiment, the distal portion of the insertion section **14** optionally may be fluidly sealed from the surrounding space, the camera and light source relying on the transparency of the distal end of the shaft.

Additionally, in some embodiments, a controller monitoring the rotational position of the camera assembly **350** may increase or decrease the intensity of light emitted by various light sources **702a-d** depending on the position of the field of view **700.** For example, a controller may monitor displacement of a pivot control structure **100** (see, e.g. **FIG. 14**) via a sensor such as a rotational potentiometer attached to a pivot shaft **204** (see, e.g. **FIG. 14**). Based on data collected from the sensor, a controller may determine if a light source **702a-d** has shifted from a direct light source to an indirect light source or vice versa. The controller may then adjust the intensity of light produced by that light source **702a-d** accordingly. Based on the sensor reading, the controller may, for example, determine which light sources **702a-d** are emitting light into the field of view **700** of the camera assembly **350** and decrease the intensity of light produced by those light sources **702a-d.** A controller may use the sensor reading to determine if the camera assembly **350** has been rotated such that a light source **702a-d** which was previously emitting light directly into the field of view **700** is now acting as an indirect light source. Upon determination that a light source **702a-d** has shifted from a direct light source to an indirect light source the intensity of light produced by that light source **702a-d** may be increased. The adjustment of the emitted light may be done in a continuous, gradual fashion, step wise fashion, or binary manner (e.g. switching between a preset direct and indirect intensity level).

An example of a printed circuit board **430a** which includes a number of LED light sources **750** and a sensor **754** on an extension **430h** of the printed circuit board **430a** is shown in **FIGS. 67-68****.** A sensor or camera assembly **350** is also shown at the end of the PCB extension **430h.** **FIG. 67** depicts a top down view of the printed circuit board **430a,** while **FIG. 68** depicts a side view of the printed circuit board **430a.**

The printed circuit board **430a** may include a main portion **430L** from which a shaft portion **430H** extends. The main portion **4301** of the printed circuit board **430a** may be housed in the handle **12** (see, e.g. **FIG. 3A** or **3B**) of an endoscope **10.** In the example embodiment shown in **FIGS. 67-68****,** the main portion **4301** is not shown populated with electronic components for sake of simplicity. An example printed circuit board **430a** populated with exemplary components **430b-f** is depicted in **FIG. 96****.** In some embodiments, and as shown in **FIG. 68****,** at least a portion of the main portion **430L** of the printed circuit board **430a** may be coated or encased in a protective coating or layer of material. The protective material may be any of a variety of potting material or conformal coating materials. Acrylic, epoxy, polyurethane, silicones, parylene, thermo-setting plastics, rubber, or any other potting material or conformal coating material may be used. Preferably, the protective material is biocompatible and provides waterproofing characteristics. A transparent protective coating may also be placed over the LEDs **750** and sensor **754** on the projecting portion **430h.**

The PCB extension (or optionally ribbon cable) **430h** may extend through a pass-through barrier **159** (see, e.g. **FIG. 15**) and along the axis of an insertion section **14** (see, e.g. **FIG 15**) of an endoscope **10.** The projecting portion **430h** may provide power and data communication pathways to various components (e.g. a camera assembly **350** and/or LEDs **750**) within the insertion section **14** (see, e.g., **FIG. 15**). The projecting portion **430h** may be divided into a number of different parts. In the example shown, the projecting portion **430h** includes a first portion **430i,** second portion **430j,** and a third portion **430k.** (In other embodiments, the projecting portion **430h** may be divided into a different number of components). Each part of the projecting portion **430h** may possess different characteristics. For example, each part of the projecting portion **430h** may have differing levels of flexibility. Certain parts may be rigid circuit board, while other parts are flex cable. Additionally, each part of the circuit board may have different number of layers, different widths, different numbers of traces on each layer, etc. It may be desirable that at least one part of the projecting portion **430h** of the printed circuit board **430a** be a flex cable or be otherwise flexible. This may help to facilitate rotation of the camera assembly **350.**

In some embodiments, the first portion **430i** may be a six layer rigid circuit board. The second portion **430j** may be a 2 layer flex cable. The third portion **430k** may be a 4 layer rigid circuit board. Each section may transition into the next section or connectors may be used between one or more section of the projecting portion **430h.** To simplify manufacturing and to reduce costs, it may be preferable that the communications and power lines in the endoscope shaft comprise flexible and/or rigid extensions of the main PCB located in the endoscope handle. The entire PCB with extension(s) can be manufactured as shown in FIG. 33.2, and any flexible PCB extension can be folded over as shown in FIG. 33.3 to run adjacent to a companion PCB extension (rigid or flexible) during assembly of the endoscope.

The sensor **754** may be any of a variety of sensors. In some embodiments, the sensor **754** may be a temperature sensor such as a thermistor, thermocouple, or resistance temperature detector. In some specific embodiments, the sensor **754** may be thermistor. A temperature sensor **754** may be used to monitor the temperature of the environment near or at the LEDs **750.**

During an endoscopic procedure, irrigation fluid may flow over the LEDs **750.** This fluid may help to cool the LEDs **750** keeping the temperature of the LEDs **750** within a desired temperature range. In the event that an endoscope **10** is outside of a patient and irrigation fluid is not running, a controller may monitor data from the sensor **754** to determine if the environment near the LEDs **750** is becoming hotter than a desired temperature range. In the event that temperature exceeds the temperature range, the controller may command current flow to the LEDs **750** to be lowered or the controller may command the LEDs **750** off. A temperature sensor may also be used as an irrigation fluid flow sensor. During an endoscopic procedure, the flow rate of irrigation fluid may be sufficient to dissipate heat produced by the LEDs **750** via convection such that the area surrounding the LEDs **750** is within a desired temperature range. In the event that the flow rate decreases beyond a certain amount, the temperature in the area proximal to the LEDs **750** may rise. This rise may, in some embodiments, be interpreted by a controller as a decrease in irrigation fluid flow rate. In response, the controller may generate a notification to user to this effect.

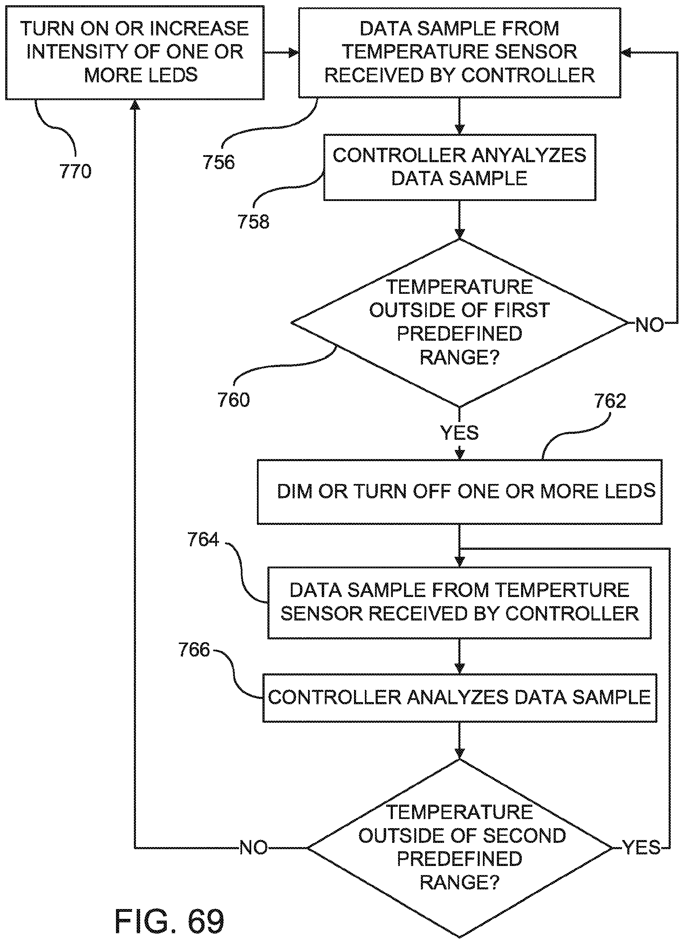

**FIG. 69** depicts a flowchart detailing a number of example steps which may be used by a controller to control LEDs in an insertion section **14** based on a sensed temperature. In step **756,** a controller receives a data sample from a temperature sensor near the LEDs. The controller then analyzes the data sample in step **758.** If **760** the temperature is not outside of a first predefined range, step **756** may be repeated. If **760** the temperature is outside of the first predefined range, the controller may transition one or more LED from a first state to a second state in step **762.** The temperature range may have a high bound of between 40-50 degrees Celsius (e.g. 50 degrees Celsius). The first state may be a high light output intensity on-state and the second state may be an off-state. In the example flowchart, the second state is a dimmed or off-state. The controller receives a data sample from the temperature sensor in step **764** and analyzes the data sample in step **766.** If **768** the data sample indicates the temperature is outside of a second predetermined range, step **764** may be repeated. If **768** the data sample indicates that the temperature is within the second predefined range, at least one LED may be turned on or commanded to increase the intensity of light it produces in step **770.**

The first and second predefined ranges may be the same, or the first and second predefined ranges may differ, with the second predefined range being less than the first predefined range. This may ensure that the controller does not rapidly cycle between turn on/off or dimming and increasing the intensity of light from the LEDs. In some embodiments, after reaching step **762,** a timer may be started. The timer may be a minimum dim or off time timer for the at least one LED whose brightness has been modified. In the event that the minimum off or dim timer has not elapsed, the at least one LED may not be turned back or commanded to increase light output intensity even if the temperature is within the second predefined range. This may again help to prevent rapid cycling between LED states.

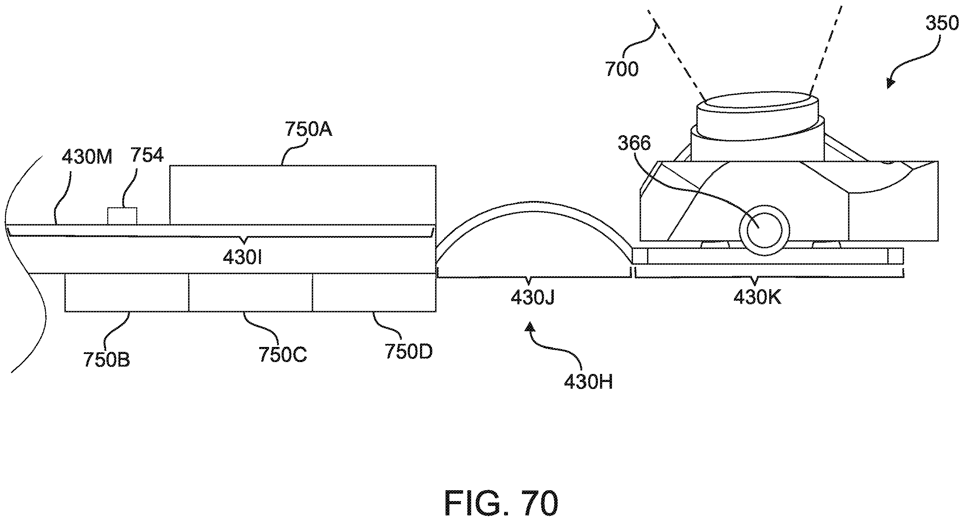

**FIG. 70** depicts a close-up side view of an example of the end of the projecting portion **430h** of the printed circuit board **430a.** As shown, the projecting portion **430h** includes a first portion **430i,** second portion **430j,** and third portion **430k.** A camera assembly **350** is attached to the third portion **430k.** A sensor **754** is mounted on the first portion **430i** in the vicinity of a number of LEDs **750a-d.** The sensor **754** may be a temperature sensor such as a thermistor and may be used to aid in control of the LEDs **750a-d.** Sensors for detecting characteristics of a fluid environment other than temperature (e.g., conductivity, pH, etc..) may also be used.

LED **750a** may be a direct lighting source and generally projects light into the field of view **700** of the camera assembly **350.** LED **750a** is mounted to a first side **430m** of the first portion **430i** of the projecting portion **430h.** LEDs **750b-d** are mounted on a second side **430n** of the first portion **430i** which is opposite the first side **430m.** These LEDs **750b-d** may be indirect light sources and in some embodiments may not project light directly into the field of view **700** of the camera assembly **350** in any rotational orientation of the camera assembly **350.** In some embodiments, LED **750a** may be a RBG LED array. LED **750a** may be adjusted to provide light at a spectrum which may help to modify the color of the image produced by an image sensor of the camera assembly **350** in a desired manner. For example, LED **750a** may be adjusted to correct a color bias of an image sensor of the camera assembly **750a.** LEDs **750b-d** may be white light LEDs. As shown, the first portion **430i** of the projecting portion **430h** may be a thicker portion of printed circuit board relative to other portions **430j, 430k** of the projecting portion **430h.** This may allow the power demands of the LEDs **750a-d** to be easily accommodated.

The first portion **430i** may transition into the second portion **430j** of the projecting portion **430h.** The second portion **430j** may be a relatively thin flex cable. The second portion **430j** may facilitate rotation of the camera assembly **350** about the axis of the pivot pins **366** of the camera assembly **350.** As shown, the second portion **430j** may be of a length such that when the first portion **430i** and third portion **430k** are parallel to one another, the second portion **430j** is bowed or arched. This may help to allow for increased range of motion of the camera assembly **350.**

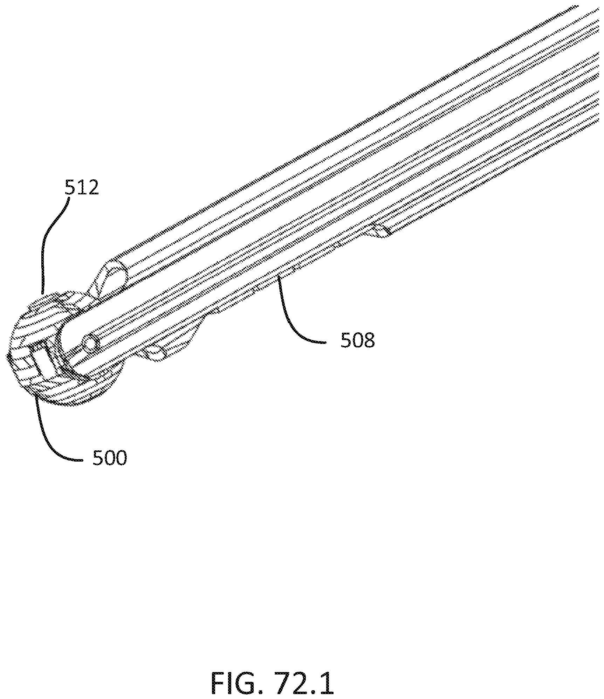

**FIG. 71** depicts an example embodiment of a camera assembly housing **330** of an insertion section **14.** **FIG. 72** depicts a cross-sectional view of the camera assembly housing **330** taken at line **72-72** of **FIG. 71****.** The camera assembly housing **330** is continuous with the inner sheath **312** and both can be formed as a single part. As shown in **FIG. 71-72** a projecting portion **430h** of a printed circuit board **430a** (see, e.g., **FIG. 68**) is shown within the camera assembly housing **330.** The projecting portion **430h** is similar to that shown in **FIG. 70** and includes a camera assembly **350,** a number of LEDs **750a-d** and, optionally, a sensor **754.** As shown, the LEDs **750a-d** are arranged such that they may emit light out of the top and bottom openings **338, 340** of the camera assembly housing **330.** The camera assembly **350** may be panned such that its field of view can be swept from the embrasured opening **344** at the rounded tip **342** through the opening provided by the top void **338.** When fully assembled an outer sheath **318** (see, e.g. **FIG. 22**) may be placed over the camera assembly housing **330** and inner sheath **312** to protect the camera assembly **350** while still providing an unobstructed field of view.

**FIG. 23****.****1** shows a perspective view of the distal end of the shaft of an endoscope (or arthroscope) 14 in which the sensor or camera housing 500 is positioned at the tip of the shaft. In this case the inner sheath 312 has no distal-most protective guard, shield or tip structure. At least a portion of the rotatable sensor or camera housing 500 (i.e. the distal-most portion) forms the distal-most element of the endoscope insertion end. Thus the sensor housing 500 is preferably constructed to withstand repeated contact with anatomical structures including soft tissue, bone, cartilage or articular surfaces. In an embodiment, as described above for camera housing 350, the rounded or dome-shaped camera housing 500 may also include a light source, movable with the rotatable camera housing, and continuously directed toward the field of view of the camera housing 500. The light source may comprise, for example, one or more LED's, or the terminus of a fiberoptic bundle. In the example shown in FIG. 23.1, a light source 508 (in this case a bank of LED's) is positioned on a side of the shaft just proximal to the location of the rotatable sensor or camera housing 500. In the illustration, the light source 508 is on the side that directs illumination toward the general field of view of the camera/lens assembly 500 when rotated to approximately 90 degrees with respect to the long axis of the endoscope shaft 14. (Alternatively, the light source may be arranged to be opposite the side on which the camera assembly - lens and sensor - can be oriented). In the arrangement shown, the optical axis of the camera assembly can be directed generally toward the direction of light projected from a first side of the insertion end of the endoscope. In other arrangements, the light source is positioned on a second opposing side of the insertion end, projecting light away from the second side of the insertion end of the endoscope, while the optical axis of the camera assembly can be directed generally toward a field of view opposite the first side of the insertion end of the endoscope. **FIG. 72****.****1** shows a perspective view of this latter arrangement, in which the light emitted by the light source 508 (LED's in this example) is directed to a region generally away from the field of view of the sensor and lens 512. The alternative second arrangement is intended to provide indirect illumination (or backlighting) of the field of view of the camera assembly, relying on the ambient light generated by the illumination source in the operative field of the endoscope. The illumination source or LED's are mounted to be flush with or recessed from the outer surface of the inner sheath or shaft of the insertion end. If recessed, there is less likelihood that heat generated by the LED's will directly touch or injure nearby tissues in the operative field.

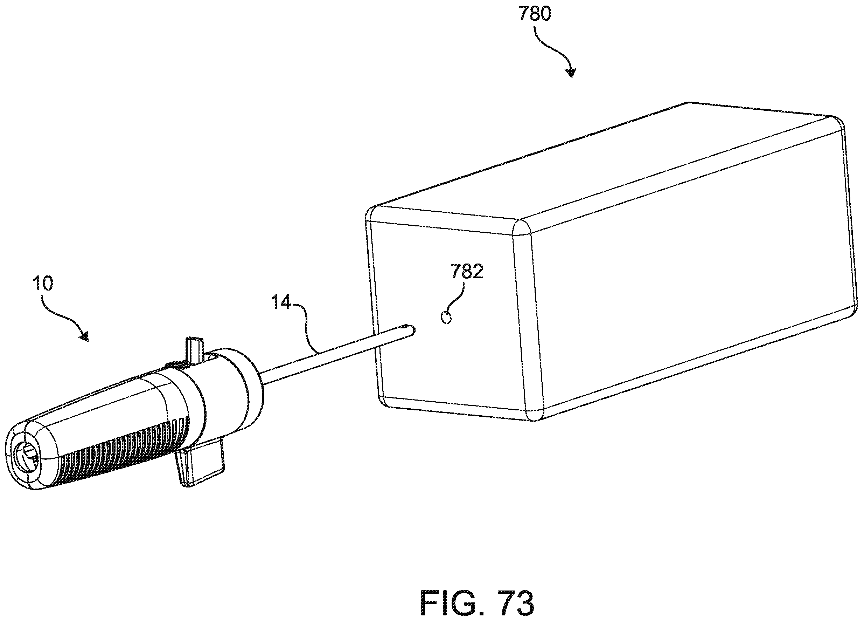

Referring now to **FIG. 73****,** in embodiments which include at least one variable illumination source which may produce light at different intensities and/or spectrums, the endoscope **10** may be placed in a calibration fixture **780** to aid in setting various lighting parameters. An embodiment with one or more white LED and one or more colored LED (e.g. a RBG LED array) may for example be placed in a calibration fixture prior to usage to adjust light output intensity of its LEDs and adjust the color output of the one or more colored LED. This may help to ensure more uniform image quality and minimize differences between endoscopes **10** which may be attributable to variation between their LEDs and/or image sensors.

The calibration fixture **780** may be a light-tight box or other volume including an opening **782** sized to fit the insertion section **14** of the endoscope **10.** This opening **782** may be gasketed such that a light-tight seal is formed against the insertion section **14** of the endoscope **10** when the endoscope **10** is installed in the calibration fixture **780.** The interior of the calibration fixture **780** may include one or more target(s) with known characteristics placed within the field of view of a camera assembly **350** (see, e.g. **FIG. 71**) of the endoscope **10.** For example targets with known color characteristics may be placed within the calibration fixture **780.**

A controller may monitor characteristics of the one or more target(s) in the image captured by an image sensor of a camera assembly **350.** Since the characteristics (e.g. color) of the targets is known, the controller may adjust the lighting provided by the variable light source(s) until the characteristics of the target(s) in the captured image match or are within a range of the target's or targets' known characteristics. For example, the intensity of light and/or spectrum of light produced by a number of LEDs **750a-d** (see, e.g. **FIG. 70**) included in the insertion section **14** may be adjusted.

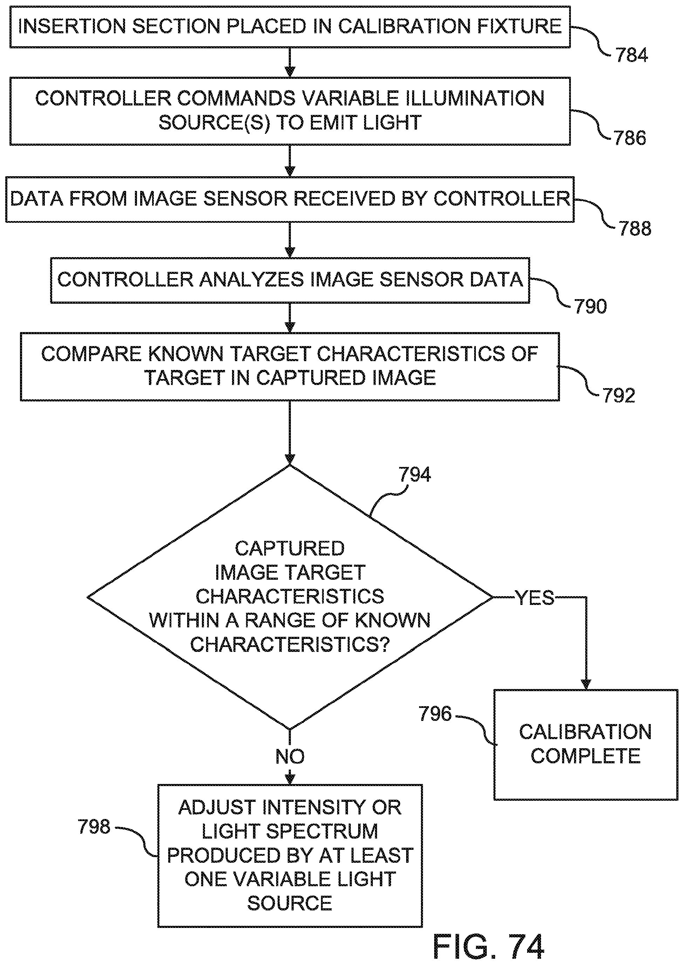

**FIG. 74** depicts a flowchart detailing a number of example steps which may be used to calibrate one or more lighting parameter of at least one variable light source included in an endoscope. In step **784** a portion of the insertion section of an endoscope including a camera assembly may be placed in a calibration fixture. Once inserted, a controller may command variable illumination sources in the endoscope to emit light in step **786.** The light may be emitted based on default parameters (e.g. light intensity and color parameters). In step **788,** a controller may receive image date from an image sensor of an endoscope's camera assembly. This data may be analyzed by a controller in step **790.** The image data may, for example, be analyzed to determine one or more characteristics of interest of a target or targets in the captured image. These characteristics may be compared, in step **792,** to the known or expected characteristics of the target or targets which have been imaged. If **794** the characteristics of the target in the captured image are within a range of the known or expected characteristics, the calibration may be considered complete in step **796.** If **794** the characteristics of the target or targets in the captured image are not within a range of the known or expected characteristics, a controller may adjust one or more illumination parameter of at least one variable illumination source of the endoscope in step **798.** In the example embodiment, the intensity and/or spectrum of light produced by at least one variable light source is adjusted in step **798.** Step **788** may repeated after adjustment. Images may continue to be compared and analyzed and illumination parameters may be adjusted until the characteristics of targets in the captured image are within range of their known or expected values.
Various methods may be used in the construction and assembly of a lens or group of lenses to focus images onto the sensor or camera at the distal end of the endoscope shaft. Examples of such methods and techniques are disclosed in US Patent Application Serial No. 14/170,080 (US Application Publication No. 2014/0221749), filed Jan. 31, 2014, and incorporated herein by reference in its entirety.

**FIG. 96** shows another example embodiment of the endoscope **10.** An outer sheath **318** is shown installed on the endoscope in **FIG.** 96. Additionally, only the bottom half-shell **22** of the handle proximal section **16,** and half (**30a**) of the handle distal section **30** are visible for clarity of this description. As shown, the endoscope **10** includes a handle-enclosed printed circuit board **430a** (also referred to herein as handle or main PCB **430a**). An electronic cable (such as, e.g., a power/HDMI cable) **432,** optical fibers **364,** and irrigation/suction line **434** are also shown. **FIG. 96** shows example routing pathways for the power/HDMI cable **432,** optical fibers **364,** and irrigation line **434.** As shown, the electronic cable **432,** optical fibers **364,** and irrigation line **434** enter the endoscope **10** through an opening **60** at the rear or butt end of the handle proximal section **16.** This entry point may be more advantageous than a handle side-entry point because it reduces the potential of various cords and cables to get tangled as the insertion section is rotated relative to the handle proximal section **16.**

In some embodiments, the electronic cable **432,** optical fibers **364** (if present), and irrigation line **434** may enter the endoscope **10** at an angle with respect to the rear handle opening **60.** Such an arrangement would afford an ergonomic benefit to the user by allowing the user to grasp a greater portion of the rear portion of the handle proximal section **16.**

As shown, the electronic cable **432,** optical fibers **364** (if implemented in the endoscope), and irrigation line **434** extend over a portion of the handle PCB **430a** after entering the handle proximal section **16.** The electronic cable **432** plugs into a connector **430b** (such as, e.g. a power/HDMI connector) on the handle PCB **430a.** The electronic cable **432** may provide power to the endoscope **10.** Image data may pass to the handle PCB **430a** via the flex cable **250.** The electronic cable **432** may transmit visual data collected by the endoscope **10** to an external graphical user interface display (not shown). The optical fibers **364** (if implemented in the endoscope) and irrigation line **434** extend under the handle PCB **430a** and follow the pathways previously described. In embodiments in which the endoscope **10** is disposable, the electronic cable **432,** optical fibers **364,** and irrigation line **434** may all be included as disposable components to ensure sterility each time an endoscope is used, or to save on the costs of sterilization and packaging for re-use.

In this example, a control wire **91** for button **90** is also shown in **FIG. 96****.** As shown, the control wire **91** passes through an orifice in the sealing member **210.** The control wire **91** is in communication with the handle PCB **430a.** Also as shown in **FIG. 96** the handle PCB **430a** includes a handle PCB flex cable **430e.** The handle PCB flex cable **430e** connects to a handle PCB portion **430f,** permitting PCB portion **430f** to be oriented at an angle (e.g., perpendicular) to the rest of the handle PCB **430a.** When assembled, the flex attached handle PCB portion **430f** may be disposed between the two potentiometers **122** of the example rotation sensing assembly **150** (see **FIG. 8**).

In some embodiments, the handle PCB **430a** may include an image or graphic processing unit **430c.** Preferably, however, the image processing unit **430c** is located external to the endoscope **10.** The image processing unit **430b** may function as an electronic righting mechanism for the endoscope **10.** The image processing unit **430c** may receive the image captured by the image sensor **380** which is sent from the image sensor **380** to the handle PCB **430a** via the flex cable **250.** In a preferred embodiment, the image captured by the image sensor **380** is then transmitted to the image processing unit **430c** external to the endoscope **10** via the electronic cable **432.** The image processing unit **430c** may also receive a signal from the rotation sensing assembly **150.** In some embodiments, an analog to digital converter **430d** may be included on the handle PCB **430a** to convert the signal from the rotation sensing assembly **150.** The image processing unit **430c** may use the signal from the rotation sensing assembly **150** to electronically "right" the image to a desired orientation. In some embodiments, image may be rotated by the image processing unit **430c** so that the image is displayed as if it were captured from the user's point of view. In some embodiments, the image processing unit **430c** may also correct for the effects of lens distortion.

Unless the orientation of an image displayed on a graphical user interface is first corrected, the displayed image may be disorienting to the user. By defining a direction according to the user's point of view, the image processing unit **430c** may use data from the rotation sensing assembly **150** to automatically rotate images so that images correspond with the user's point of view.

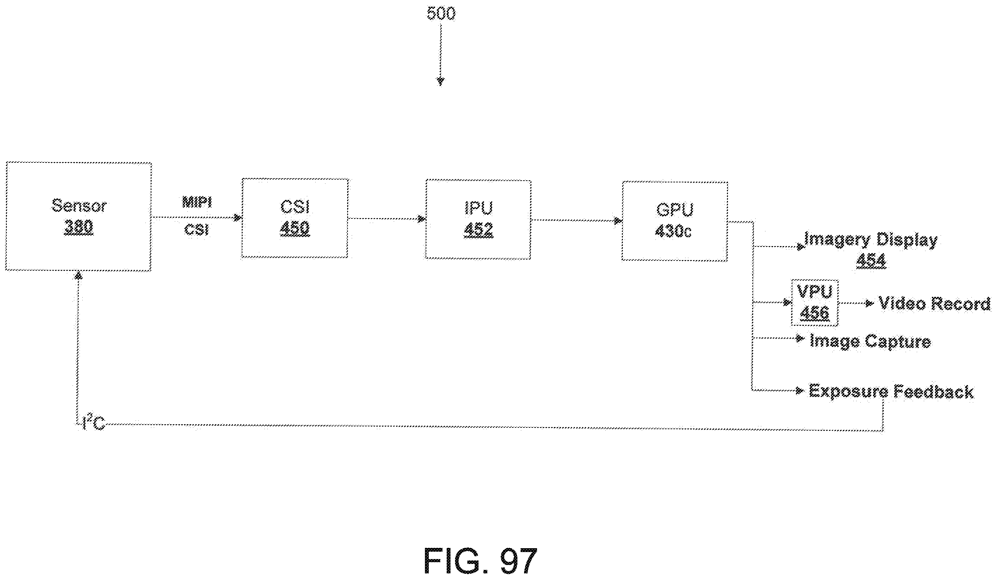

**FIG. 97** shows an example block diagram of an imaging system. As shown, the imaging system includes an image sensor **380** that captures an image. The image captured by the image sensor **380** may be passed via a camera serial interface **450** (for example a MIPI camera serial interface) to an image processing unit **452.** The image processing unit **452** (IPU) may then move image frames to other hardware components in the imaging system. Other hardware components may include, but are not limited to, a memory device and a graphical processing unit **430c** (GPU). The graphical processing unit **430c** may correct any distortion caused by the lens assembly **354.**

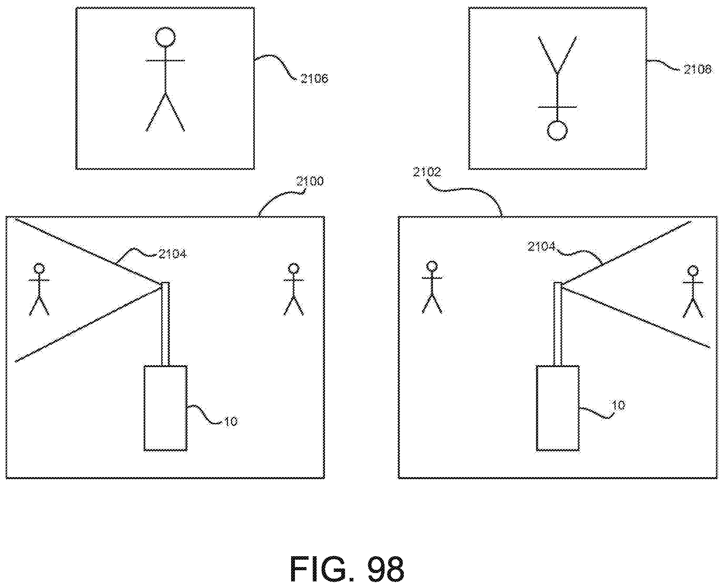

In some embodiments, the graphical processing unit **430c** may correct this distortion by representing the image as a texture on a surface that has been loaded into the graphical processing unit **430c.** This may cause the image to be adjusted or stretched in a manner which corrects and/or removes the distortion introduced by the lens assembly **354.** In embodiments where the image is righted, the graphical processing unit **430c** may then rotate the corrected image via input from a rotation sensing assembly **150** (see, for example, **FIG. 8**). For example, the measurement from a rotation sensing assembly **150** may be passed to the graphical processing unit **430c** through an analog to digital converter **430d** (see, for example, **FIG. 96**). The signal from the analog to digital converter **430d** may then be used to rotate the image to its righted orientation. In some embodiments, a user may be able to toggle image righting, distortion correction, and/or various other image manipulations which may be performed on or off. Image righting will be further described later in the specification in relation to **FIG. 98****.**

The processed image from the image processing unit **430c** may then be displayed on a graphical user interface or display **454.** In some embodiments, the processed image from the image processing unit **430c** may be stored in memory. In such embodiments, a user may capture images to be stored in memory for later recall by triggering a button **90,** for example. Some embodiments may include a video processing unit **456** which may encode the frames from the image sensor **380** into a recordable video format. In such embodiments, encoded video may then be stored in memory. A user may command the endoscope to initiate and stop video capture via interaction with a button such as button **90** as described above.

In some embodiments, the image processing unit **430c** may also subject a captured image to exposure feedback analysis. In specific embodiments, an image histogram may be created from all the pixels of the image. The image histogram may then be used to tune the image or tune the exposure of subsequent images received by the image chip or sensor **380.** Such further processing by the image processing unit **430c** may help to reduce blown-out white areas of the image or underexposed dark areas of the image. Other means of tuning an image or images, such as, for example, tone mapping, etc. may also be used.

**FIG. 98** depicts an example diagram illustrating how an image may be righted using input from a rotation sensing assembly **150** (see, for example, **FIG. 98**). As shown, a first block **2100** and a second block **2102** are depicted. Within each block **2100, 2102** is an endoscope **10** having a field of view **2104** is depicted. The field of view **2104** of the endoscope **10** in the first block **2100** is oriented approximately 180 degrees from the endoscope **10** in the second block **2102.** This may be accomplished by rotating the distal end of the endoscope **10** relative to the proximal end of the endoscope **10.** In conventional endoscopes **10,** during rotation of the distal section relative to the proximal section, the image sensor does not rotate because the image sensor is housed in the proximal section. Thus, the endoscopes **10** shown in the first block **2100** and second block would both capture image **2106.**

This would not be the case In some embodiments described herein in which the image sensor **380** rotates with the distal end of the endoscope **2106..** The endoscope **10** shown in the first block **2100** would capture image **2106,** while the same endoscope **10** rotated to the position shown in the second block **2102** would capture image **2108.** As the image sensor rotates with the distal end of the endoscope **10,** the image sensor will invert the image. In this position, for example, the top of the image sensor will pick up what one accustomed to a conventional endoscope **10** would expect to be the bottom of the image.

Optionally, the image may be rotated in proportion to the degree of rotation of the distal end of the endoscope **10.** Thus the image can always be displayed in a way which would be expected by a user accustomed to conventional endoscopes **10.** This may help to alleviate problems associated with a rotating image sensor.

Various embodiments shown in the drawings are presented only to demonstrate certain examples of features of the disclosure. Not all features shown in any given drawing necessarily have to be included in a claimed device or feature. The drawings are to be interpreted only for illustrative purposes; as such, the size of some of the elements may be exaggerated and not drawn to a particular scale. Additionally, elements shown within the drawings that have the same reference numbers may be identical elements or may represent similar or analogous elements, depending on the context.

Any terms such as "first", "second", "third" and the like, whether used in the description or in the claims, are intended to distinguish between similar elements and not necessarily to describe a sequential or chronological order. It is to be understood that the terms so used are interchangeable under appropriate circumstances (unless clearly stated otherwise) and that the embodiments of the disclosure described herein are capable of operation in other sequences and/or arrangements than are described or illustrated herein.
The application also includes the following embodiments:
[1]. An endoscope comprising:
   a proximal handle and a shaft having a distal insertion end;
   a camera assembly mounted in a rotatable housing on the insertion end;
   the rotatable housing configured to rotate about an axis generally perpendicular to a long axis of the insertion end;
   wherein the rotatable housing is a distal-most element of the insertion end of the shaft.
[2]. The endoscope of [1], wherein the camera assembly includes a lens adjacent to an image sensor.
[3]. The endoscope of [2], wherein the image sensor is a CMOS or a CCD device.
[4]. The endoscope of [1], wherein the endoscope comprises a pull wire extending from the handle to the insertion end of the shaft, the pull wire wrapping around a portion of the rotatable housing, the pull wire configured to rotate the rotatable housing upon fore or aft movement of the pull wire in the shaft.
[5]. The endoscope of [1], wherein the rotatable housing has a range of motion that provides the camera assembly a field of view that includes a region in line with the long axis of the insertion end and a region at least perpendicular to the long axis of the insertion end.
[6]. The endoscope of [1], wherein the rotatable housing has a range of motion in which an optical axis of the camera assembly is moveable from about 0 degrees to about 120 degrees with respect to the long axis of the insertion end.
[7]. The endoscope of [1], wherein the rotatable housing has a range of motion in which an optical axis of the camera assembly is moveable from about 35 degrees to about 115 degrees with respect to the long axis of the insertion end.
[8]. The endoscope of [1], wherein the rotatable housing of the camera assembly comprises a spheroid shell constructed from an assembly of two half-shells, one or both of the half-shells having an internal cutout to accommodate the camera assembly.
[9]. The endoscope of [1], further comprising a light source located on the rotatable housing, wherein the light source is configured to illuminate a field of view of the camera assembly.
[10]. An endoscope comprising:
   a proximal handle and a shaft having a distal insertion end;
   a camera assembly mounted on the insertion end;
   the camera assembly configured to rotate about an axis generally perpendicular to a long axis of the insertion end;
   a light source located on the insertion end of the shaft, and oriented to project light in a direction generally perpendicular to the long axis of the insertion end.
[11]. The endoscope of [10], wherein the camera assembly is configured to rotate so that it has a field of view including an area directly illuminated by the light source.
[12]. The endoscope of [10], wherein the camera assembly is configured to rotate so that it has a field of view that excludes an area directly illuminated by the light source.
[13]. The endoscope of [12], wherein the field of view of the camera assembly is illuminated indirectly or by reflected or ambient light from the light source.
[14]. The endoscope of any of [10-12], wherein the light source is one or more light emitting diodes.
[15]. A printed circuit board for use in an endoscope comprising:
   a base portion of the printed circuit board configured to reside within a handle of the endoscope; wherein one or more elongate extension portions of the printed circuit board are configured to extend from the base portion of the printed circuit board in the handle through a shaft of the endoscope, and terminating at a distal insertion end of the shaft.
[16]. The printed circuit board of [15], wherein the base portion is a composite of a flexible board mated to a rigid board, and wherein at least one of the one or more extension portions comprises a flexible board extension.
[17]. The printed circuit board of [15], wherein the base portion is a composite of a flexible board mated to a rigid board, and wherein at least one of the one or more extension portions comprises a rigid board extension.
[18]. The printed circuit board of [15], wherein the base portion is a composite of a flexible board mated to a rigid board, and wherein a first one of the one or more extension portions comprises a flexible board extension, and a second one of the one or more extension portions comprises a rigid board extension.
[19]. The printed circuit board of [18], wherein the flexible board extension and the rigid board extension emerge from the base portion adjacent to each other, wherein a proximal leg of the flexible board extension extends away from the rigid board extension at an angle of approximately 90 degrees from the rigid board extension, and wherein a distal leg of the flexible board extension extends away from the base portion parallel to the rigid board extension.
[21]. The printed circuit board of [19], wherein the proximal leg of the flexible board extension is folded to permit the distal leg of the flexible board extension to be aligned parallel to and adjacent to the rigid board extension through the shaft of the endoscope.
[21]. The printed circuit board of [21], wherein the rigid board extension and the flexible board extension extend through a lumen of the endoscope shaft.
[22]. The printed circuit board of [21], wherein the base portion of the printed circuit board, the rigid board extension, and the flexible board extension are coated with a water resistant coating or membrane.
[23]. The printed circuit board of [22], wherein the rigid board extension and the flexible board extension extend through a fluid-carrying lumen of the endoscope shaft.
[24]. The printed circuit board of [18], wherein the rigid board extension carries electrical lines connected to one or more light sources at the insertion end of the endoscope shaft.
[25]. The printed circuit board of [18], wherein the flexible board extension carries communication lines connected to an image sensor at the insertion end of the endoscope shaft.
[26]. The printed circuit board of [25], wherein the image sensor is a CCD or CMOS sensor.
[27]. The printed circuit board of [26], wherein the flexible board extension is configured to permit the image sensor to rotate about an axis generally perpendicular to an axis of the endoscope shaft.
[28]. An endoscope comprising:
   a proximal handle housing configured to house an electronic processing board for processing signals from an image sensor located at a distal end of a shaft of the endoscope; a distal handle housing configured to hold the electronic processing board in a position fixed with respect to the distal handle section;
   one or more magnets attached to an internal wall of the proximal handle housing, said magnets being located next to a Hall effect sensor on the electronic processing board, wherein
   the proximal handle housing is rotatable with respect to the distal handle housing, and the Hall effect sensor is configured to provide a signal to an electronic processor representing the relative rotation of the proximal handle housing with respect to the distal handle housing.
[29]. The endoscope of [28], wherein the electronic processor is connected to a user interface displaying an image generated by the image sensor, and wherein a rotational orientation of the image is altered by a change in the relative rotation of the proximal handle housing with respect to the distal handle housing.
[30]. An endoscope comprising:
   a handle enclosing an electronic processing board for processing signals from an image sensor located at a distal end of a shaft of the endoscope;
   a button on the handle having a member that encloses a magnet;
   the magnet positioned above or adjacent to a portion of the electronic processing board on which a Hall effect sensor is located;
   wherein depression, release or movement of the button alters a magnetic field near the Hall effect sensor sufficiently to alter a signal produced by the Hall effect sensor.
[31]. The endoscope of [30], wherein the button is configured to cause an electronic controller connected to the electronic processing board to start a recording of an image generated by the image sensor, to stop a recording of an image generated by the image sensor, or to take a photograph of an image generated by the image sensor, based on a movement or release of the button by a user.
[32]. The endoscope of [30], wherein the button is configured to cause an electronic controller connected to the electronic processing board to turn on, turn off, or adjust a light source located at a distal insertion end of a shaft of the endoscope, based on a movement or release of the button by a user.
[33]. The endoscope of [31] or [32], wherein the movement or release of the button comprises a short or longer duration depression of the button, a pre-determined series of two or more depressions and releases of the button, or a release of the button between two depressions having two or more variable durations.

## Claims

1. An endoscope comprising:
a proximal handle housing configured to house an electronic processing board for processing signals from an image sensor located at a distal end of a shaft of the endoscope;
a distal handle housing configured to hold the electronic processing board in a position fixed with respect to the distal handle section;
one or more magnets attached to an internal wall of the proximal handle housing, said magnets being located next to a Hall effect sensor on the electronic processing board, wherein the proximal handle housing is rotatable with respect to the distal handle housing, and the Hall effect sensor is configured to provide a signal to an electronic processor representing the relative rotation of the proximal handle housing with respect to the distal handle housing.

2. The endoscope of claim 1, wherein the electronic processor is connected to a user interface displaying an image generated by the image sensor, and wherein a rotational orientation of the image is altered by a change in the relative rotation of the proximal handle housing with respect to the distal handle housing.

3. The endoscope of claim 1 or claim 2 wherein the endoscope comprises two opposing magnets.

4. The endoscope of any preceding claim wherein the magnets are coupled to a retaining structure for holding the magnets in place in the proximal handle housing.

5. The endoscope of any preceding claim further comprising an orientation marker for indicating rotational orientation of the proximal handle housing with respect to the distal handle housing, wherein the orientation marker comprises a protuberance.

6. The endoscope of claim 5 wherein the protuberance has a gripping texture that differs from the texture on the rest of the proximal handle housing.

7. The endoscope of any preceding claim wherein the proximal handle comprises a first half-shell and a second half-shell which form a shell structure, and each of the first and second handle half-shells includes a magnet inserted in an interior wall of each half-shell.

8. An endoscope comprising:
a handle enclosing an electronic processing board for processing signals from an image sensor located at a distal end of a shaft of the endoscope;
a button on the handle having a member that encloses a magnet;
the magnet positioned above or adjacent to a portion of the electronic processing board on which a Hall effect sensor is located;
wherein depression, release or movement of the button alters a magnetic field near the Hall effect sensor sufficiently to alter a signal produced by the Hall effect sensor.

9. The endoscope of claim 8, wherein the button is configured to cause an electronic controller connected to the electronic processing board to start a recording of an image generated by the image sensor, to stop a recording of an image generated by the image sensor, or to take a photograph of an image generated by the image sensor, based on a movement or release of the button by a user.

10. The endoscope of claim 8 or claim 9, wherein the button is configured to cause an electronic controller connected to the electronic processing board to tum on, tum off, or adjust a light source located at a distal insertion end of a shaft of the endoscope, based on a movement or release of the button by a user.

11. The endoscope of claims 9 or claim 10, wherein the movement or release of the button comprises a short or longer duration depression of the button, a pre-determined series of two or more depressions and releases of the button, or a release of the button between two depressions having two or more variable durations.
